(19) Europäisches Patentamt
European Patent Office
Office européen des brevets

(11) Veröffentlichungsnummer : **0 023 964**
**B1**

(12) # EUROPÄISCHE PATENTSCHRIFT

(45) Veröffentlichungstag der Patentschrift : **16.02.83**

(51) Int. Cl.³ : **C 07 D277/42,** A 61 K 31/425

(21) Anmeldenummer : **80103688.0**

(22) Anmeldetag : **28.06.80**

(54) **Phenyliminothiazolinderivate, ihre Herstellung und Zwischenprodukte dafür, sie enthaltende pharmazeutische Präparate und deren Herstellung.**

(30) Priorität : **03.07.79 DE 2926771**

(43) Veröffentlichungstag der Anmeldung : **18.02.81 Patentblatt 81/07**

(45) Bekanntmachung des Hinweises auf die Patenterteilung : **16.02.83 Patentblatt 83/07**

(84) Benannte Vertragsstaaten : **AT BE CH DE FR GB IT LI NL SE**

(56) Entgegenhaltungen :
**DE A 2 533 821**
**DE A 2 546 165**

(73) Patentinhaber : **HOECHST AKTIENGESELLSCHAFT**
**Postfach 80 03 20**
**D-6230 Frankfurt/Main 80 (DE)**

(72) Erfinder : **Lang, Hans-Jochen**
**Rüdesheimer Strasse 7**
**D-6238 Hofheim am Taunus (DE)**
Erfinder : **Seuring, Bernhard, Dr.**
**Johannesallee 20**
**D-6230 Frankfurt am Main 80 (DE)**
Erfinder : **Granzer, Ernold, Dr. Dr.**
**Falkensteiner Strasse 24**
**D-6233 Kelkheim (Taunus) (DE)**

**EP 0 023 964 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Phenyliminothiazolinderivate, ihre Herstellung und Zwischenprodukte dafür, sie enthaltende pharmazeutische Präparate und deren Herstellung

Die Erfindung betrifft Verbindungen der allgemeinen Formel I

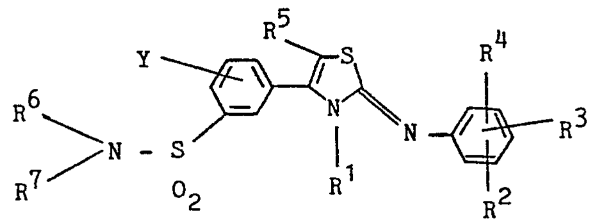

(I)

die als solche oder in Form ihrer pharmakologisch verträglichen Säureadditionssalze wertvolle pharmakologische Eigenschaften besitzen und daher als Arzneimittel geeignet sind. In der Formel bedeuten :

$R^1$ $C_1$-$C_8$-Alkyl, Cycloalkyl mit 3 bis 8 C-Atomen oder Alkenyl mit 3 bis 4 C-Atomen,

$R^2$, $R^3$ und $R^4$ Wasserstoff, Halogen, Alkyl oder Alkoxy mit jeweils 1 bis 4 C-Atomen, Methylendioxy, Äthylendioxy, Dimethyl- oder Diäthylamino, Trifluormethyl,

$R^5$ Wasserstoff oder Alkyl mit 1 bis 3 C-Atomen,

$R^6$ Wasserstoff oder Alkyl mit 1 bis 6 C-Atomen,

$R^7$ Wasserstoff, Alkyl mit 1 bis 12 C-Atomen, Cycloalkyl mit 3 bis 12 C-Atomen, Allyl, Phenyläthyl oder einen Benzylrest

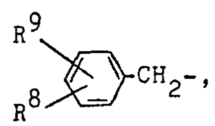

worin $R^8$ und $R^9$ gleich oder verschieden sind, und Wasserstoff, Methyl, Chlor oder Methoxy bedeuten, oder $R^6$ und $R^7$ sind über eine gegebenenfalls verzweigte Alkylenkette mit insgesamt 8 C-Atomen verbunden, worin eine Methylengruppe durch ein O-Atom oder eine $N$-$CH_3$-Gruppe ersetzt sein kann, und Y Wasserstoff, Halogen oder Alkyl mit 1 bis 3 C-Atomen.

Die Erfindung betrifft weiterhin ein Verfahren zur Herstellung der Verbindungen der allgemeinen Formel I, das dadurch gekennzeichnet ist, dass man

a) Verbindungen der allgemeinen Formel II

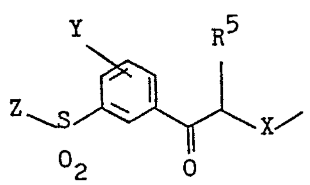

(II)

worin

$R^5$ und Y die angegebene Bedeutung besitzen,

Z für Halogen oder $R^6R^7N$- mit der für $R^6$ und $R^7$ angegebenen Bedeutung steht und

X eine leaving group wie Halogen, $CH_3SO_2$—O— oder

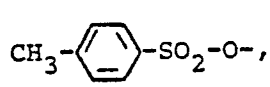

ist, u. ter kondensierenden Reaktionsbedingungen mit einem Thioharnstoff der allgemeinen Formel III

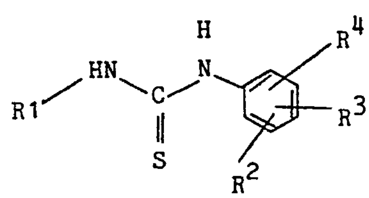

(III)

worin $R^1$ bis $R^4$ die angegebene Bedeutung besitzen, umsetzt, und im Falle, dass Z für Halogen steht, eine erhaltene Verbindung der Formel XI

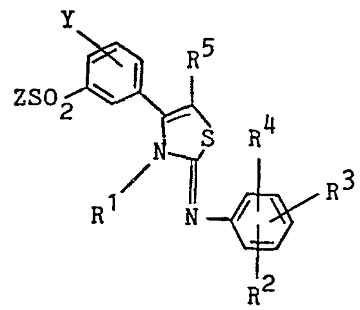

(XI)

worin $R^1$, $R^2$, $R^3$, $R^4$, $R^5$ und Y die zu Formel I angegebenen Bedeutungen besitzen und Z Halogen darstellt, nachfolgend mit einem Amin der allgemeinen Formel $HNR^6R^7$ mit der für $R^6$ und $R^7$ angegebenen Bedeutung zur Umsetzung bringt oder

b) aus Verbindungen der allgemeinen Formel IV

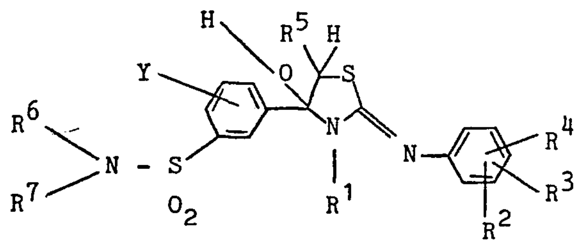

(IV)

worin $R^1$ bis $R^7$ und Y die angegebene Bedeutung besitzen, Wasser abspaltet oder

c) Verbindungen der allgemeinen Formel V

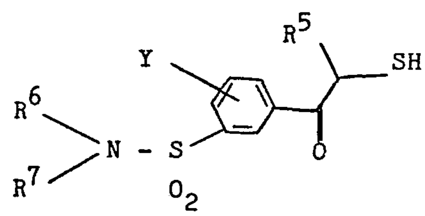

(V)

mit Verbindungen der allgemeinen Formel VI

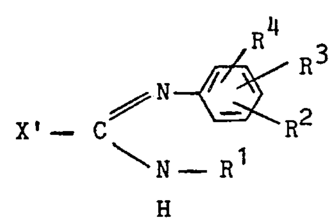

(VI)

zur Reaktion bringt, wobei $R^1$ bis $R^7$ die angegebene Bedeutung haben und X' eine leaving group, wie beispielsweise Halogen, Methoxy- oder Methylthio, ist, oder

d) Verbindungen der Formel V mit Carbodiimiden VII

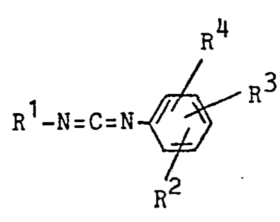

(VII)

umsetzt, wobei $R^1$ bis $R^4$ die angegebene Bedeutung haben, oder

e) Verbindungen der allgemeinen Formel VIII

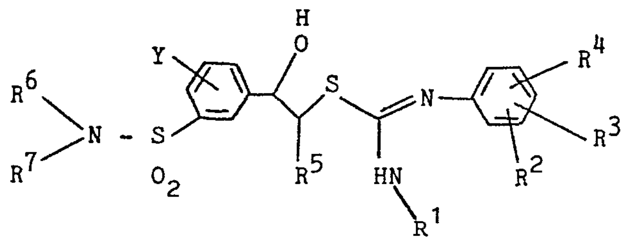

× HHal (VIII)

worin $R^1$ bis $R^7$ und Y die angegebene Bedeutung besitzen und Hal für Chlor oder Brom steht, mit einem Oxidationsmittel behandelt, oder

f) Verbindungen der allgemeinen Formel IX

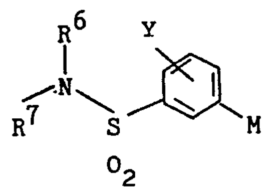

(IX)

worin $R^6$ und $R^7$ nicht für Wasserstoff und Y nicht für Brom und Jod steht, ansonsten aber die obige Bedeutung haben und M für Lithium oder eine MgBr-Gruppe steht, mit Verbindungen der allgemeinen Formel X

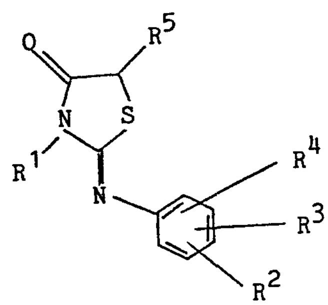

(X)

worin $R^1$ bis $R^5$ die angegebene Bedeutung besitzen, umsetzt und das erhaltene Reaktionsprodukt der Hydrolyse und der Dehydratation unterwirft, und gegebenenfalls die nach Weg a) bis f) erhaltenen Verbindungen der allgemeinen Formel I, in denen $R^6$ und/oder $R^7$ Wasserstoff bedeutet, durch übliche Alkylierung in Verbindungen überführt, in denen $R^6$ und/oder $R^7$ eine der weiteren oben angegebenen Bedeutungen hat, und gegebenenfalls eine erhaltene Verbindung der Formel I mit organischen oder anorganischen Säuren der allgemeinen Formel H—A in ihre Säureadditionssalze oder erhaltene Salze der Verbindungen der allgemeinen Formel I mit Basen in die freien basischen Verbindungen der Formel I überführt.

Als anorganische Säuren H—A kommen beispielsweise in Betracht : Halogenwasserstoffsäuren wie Chlorwasserstoffsäure und Bromwasserstoffsäure, sowie Schwefelsäure, Phosphorsäure und Amidosulfonsäure.

Als organische Säuren H—A seien beispielsweise Methansulfonsäure, Äthansulfonsäure, Benzolsulfonsäure und p-Toluolsulfonsäure genannt.

Die Verbindungen der Formeln IV und XI sind neu. Die Erfindung betrifft daher weiterhin Verbindungen der Formel IV

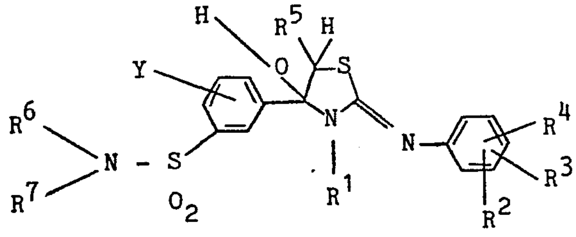

(IV)

worin $R^1$ bis $R^5$ und Y die zu Formel I angegebenen Bedeutungen haben, bzw. deren Säureadditionssalze. Sie sind als Vorprodukte bei der Herstellung von Verbindungen der allgemeinen Formel I geeignet.

Die Erfindung betrifft außerdem Verbindungen der allgemeinen Formel XI

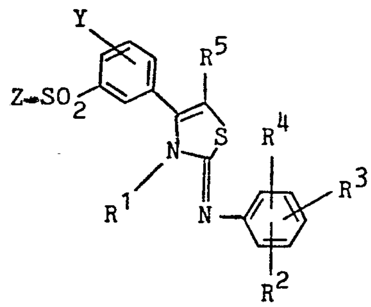

(XI)

worin $R^1$ bis $R^5$ und Y die zu Formel I gegebene Bedeutung haben und Z für Halogen steht sowie deren Säureadditionssalze, die als Zwischenprodukte bei der Herstellung von Verbindungen der allgemeinen Formel I gemäss Verfahrensweise a) verwendet werden können.

Die erfindungsgemässen Verbindungen der Formel I können ausserdem in ihren möglichen isomeren Strukturen vorliegen, wobei zum Zwecke der Vereinfachung nur eine der möglichen isomeren Formen einer jeweiligen Substanz angegeben wird.

Die unter a) bezeichnete Verfahrensweise wird vorteilhaft so ausgeführt, dass man die Verbindungen II mit den Thioharnstoffen III im molaren Verhältnis 1 : 1 bis 1 : 1,5 umsetzt. Mit grösseren molaren Überschüssen an Thioharnstoff werden im allgemeinen keine nennenswerten Vorteile erzielt.

Die Reaktion wird vorteilhaft in inerten polaren organischen Lösungsmitteln wie Dimethylformamid, Dimethylacetamid, Äthylenglykolmono- oder Äthylenglykoldimethyläther, besonders vorteilhaft in stark polaren protischen Lösungsmitteln wie Methanol, Äthanol, Isopropanol, n-Butanol, Essigsäure, Propionsäure, Ameisensäure sowie in Gemischen der genannten Solventien mit Wasser durchgeführt, wie sich auch wasserfreie Gemische der genannten Solventien eignen. Ebenso kann man die Reaktion auch ohne Verwendung eines Lösungsmittels durch Erwärmen des Reaktionsgemisches auf einen Temperaturbereich zwischen 80 und 220 °C, bevorzugt zwischen 100 und 180 °C durchführen. Bei Verwendung eines Lösungsmittels arbeitet man in einem bevorzugten Temperaturbereich von 60 bis 150 °C.

Die Reaktionsdauer ist weitgehend vom Lösungsmittel und der angewendeten Reaktionstemperatur abhängig und liegt im allgemeinen zwischen 15 Minuten und 24 Stunden. Der quantitative Reaktionsablauf zu den erfindungsgemässen Verbindungen I wird vorteilhaft dünnschichtchromatographisch an Kieselgelplatten verfolgt.

Vielfach scheiden sich die erfindungsgemässen Verbindungen I filtrierbar in Form ihrer Säureadditionssalze im Verlauf der Reaktion schwerlöslich ab, andernfalls wird das Solvens verdampft, wobei gegebenenfalls durch nachträglichen Zusatz eines geeigneten Fällungsmittels, wie beispielsweise Essigester, Diäthyläther, Diisopropyläther, Aceton, Acetonitril die Ausbeute erhöht werden kann.

Für den Fall, dass Z in der allgemeinen Formel II Halogen, vorzugsweise Chlor, bedeutet, werden die erhaltenen Verbindungen der Formel XI mit Ammoniak oder einem Amin $HNR^6R^7$ zu Verbindungen I umgesetzt. Dabei können sowohl wässrige Lösungen von Ammoniak und der Amine wie auch flüssiges Ammoniak bzw. reine Amine im Überschuss verwendet werden, wobei das überschüssige Ammoniak bzw. Amin gleichzeitig als Lösungsmittel fungiert. Die Reaktion kann ebenfalls in organischen Lösungsmitteln, wie beispielsweise Dimethylformamid, Dimethylacetamid, Dimethylsulfoxid, Dioxan, Tetrahydrofuran, Diäthylenglykol-dimethyläther, durchgeführt werden, wobei sich allerdings niedere Alkohole mit 1 bis 4 C-Atomen, wie z. B. Methanol, Äthanol oder Isopropanol in besonderer Weise eignen. Theoretisch sind für die Umsetzung der Sulfochloride XI zu den Sulfonamiden I ein Mol Ammoniak bzw. Amin in Gegenwart von zwei Molen einer Hilfsbase erforderlich. Demzufolge kann man bei der Reaktion so verfahren, dass man pro Mol Sulfochlorid XI mindestens 3 Mole Ammoniak oder Amin anwendet. Vorteilhaft ist bei dieser Reaktion die Anwendung von 3-7 Mol Ammoniak bzw. Amin auf ein Mol Sulfochlorid, jedoch können auch grössere Aminüberschüsse verwendet werden. Man kann auch mit einem oder zwei Molen Ammoniak oder Amin arbeiten, wenn in Anwesenheit einer Hilfsbase gearbeitet wird, wobei etwa 1-6 Moläquivalente Hilfsbase verwendet werden. Als Hilfsbasen eignen sich anorganische und organische Hydroxide, Carbonate und Hydrogencarbonate, sowie Salzlösungen schwacher anorganischer und organischer Säuren, wobei in allen Fällen tertiäre Amine, wie beispielsweise Triäthylamin, Tri-n-butylamin, Methyl-dicyclohexylamin, Äthyl-dicyclohexylamin, besonders vorteilhaft sind. Das tertiäre Amin kann ebenfalls, im Überschuss angewandt, ohne Zugabe eines weiteren Lösungsmittels als Reaktionsmedium dienen. Die Reaktion verläuft exotherm, so dass man vorteilhaft kühlt und bei Temperaturen zwischen – 35° und + 100 °C arbeitet, bevorzugt zwischen + 10° und + 60 °C. Die Reaktionsdauer soll mindestens 30 Minuten betragen und die Umsetzung kann spätestens nach zwei Tagen abgebrochen werden, wobei mit längeren Reaktionszeiten keine nennenswerten Vorteile erzielt

werden. Bevorzugt wird eine Reaktionsdauer zwischen 6 und 20 Stunden. Bei Aufarbeitung verfährt man vorteilhaft so, dass gegebenenfalls nach Abdestillieren des Amins und Konzentrierung des Reaktionsgemisches mit Wasser verdünnt wird, wobei die Verbindungen I schwerlöslich zur Abscheidung kommen.

Wenn $R^6$ oder $R^7$ in der so dargestellten Verbindung I ein Wasserstoffatom bedeutet, sollte möglichst ein pH 7,5 bis 8,5 eingestellt werden.

Die Verbindungen der Formel XI können durch Wasserabspaltung aus Verbindungen der Formel XII bzw. deren Salzen

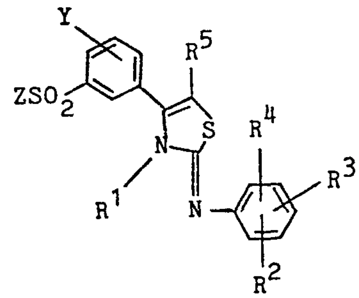

(XII)

erhalten werden, worin $R^1$ bis $R^5$, X, Y und Z die in Formel XI angegebene Bedeutung besitzen.

Dabei verfährt man nach den unter Verfahrensweise b) angegebenen Bedingungen, wobei man bevorzugt in Eisessig oder in azeotrop mit Wasser destillierenden Lösungsmitteln wie Methylenchlorid, Chloroform, Dichloräthan, Chlorbenzol, Nitrobenzol, Nitromethan, Toluol oder Xylol arbeitet, und zweckmässig das bei der Reaktion entwickelte Wasser analytisch bestimmt. Vorteilhaft führt man die Reaktion in den siedenden Lösungsmitteln durch. Besonders vorteilhaft gewinnt man die Verbindungen XI durch trockenes Erhitzen der Verbindungen XII auf Temperaturen von 100° bis 250 °C, bevorzugt von 150 bis 220 °C. Zweckmässig entfernt man das störende Kondensationswasser durch rasches Abdestillieren, bevorzugt im Luftstrom oder durch Anlegen eines wirksamen Vakuums und Verwendung eines Trockenmittels.

Die Verbindungen der Formel XI bzw. deren Salze können ebenfalls aus Anilinderivaten XIII

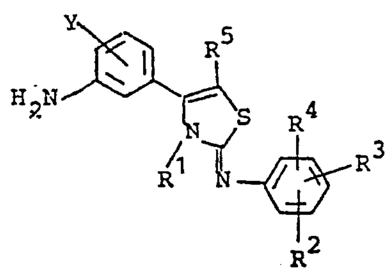

(XIII)

in an sich bekannter Weise durch Diazotierung und nachfolgende Durchführung einer Meerwein-Reaktion gewonnen werden. Die Verbindungen XIII sind aus Aminoketonen XIV bzw. deren Säureadditionssalzen

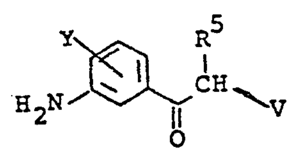

(XIV)

worin Y und $R^5$ die angegebene Bedeutung haben, und V für H steht, durch Halogenierung bevorzugt mit elementarem Brom oder Chlor, und nachfolgende Umsetzung der Halogenketone XIV mit V in der Bedeutung von Cl oder Br mit einem Thioharnstoff der Formel III unter den Durchführungsbedingungen der Verfahrensweise a) darstellbar.

Bei den verwendeten Thioharnstoffen III handelt es sich grösstenteils um Substanzen, die in der Literatur beschrieben sind. Sie werden in bekannter Weise durch Umsetzung von Aminen mit Isothiocyanaten, Schwefelkohlenstoff oder Thiophosgen dargestellt (vergl. Houben-Weyl, « Methoden der organischen Chemie », Bd. *9*, S. 384, 4. Auflage, Georg-Thieme-Verlag, Stuttgart, 1955).

Die Verbindungen der allgemeinen Formel II können nach mehreren in der Literatur beschriebenen Methoden gewonnen werden (vergl. z. B. DE-OS 24 36 263).

Nach der unter b) aufgeführten Verfahrensweise werden 2-Arylimino-4-hydroxy-4-(3-sulfamoylphenyl)-thiazolidine (IV) thermisch, bevorzugt durch Protonenkatalyse, zu den erfindungsgemässen Verbindungen der allgemeinen Formel I dehydratisiert. Man arbeitet dabei vorteilhaft in polaren organischen Lösungsmitteln, wobei sich protische Lösungsmittel wie niedere Alkohole mit 1 bis 6 C-Atomen, beispielsweise Methanol, Äthanol, Propanol, iso-Propanol, 1-bzw. 2-Butanol, Äthylenglykolmonomethyl-

äther, Diäthylenglykolmonomethyläther oder niedere aliphatische Carbonsäuren wie Essigsäure, Propionsäure, Ameisensäure oder auch Gemische der genannten Solventien eignen. Vorteilhaft ist auch die Verwendung von Wasser, insbesondere im Gemisch mit den genannten Lösungsmitteln.

Als Katalysatoren können anorganische oder organische Protonensäuren, wie Salzsäure, Schwefelsäure, Phosphorsäure, Methansulfonsäure, Toluolsulfonsäure, eine der als Solvens genannten aliphatischen Carbonsäuren, eine aromatische Carbonsäure wie Salicylsäure oder Benzoesäure verwendet werden. Die Dehydratisierung der Verbindungen IV kann grundsätzlich auch ohne Anwendung eines Katalysators wie auch ohne Anwendung eines Lösungsmittels durchgeführt werden.

Man arbeitet in einem Temperaturbereich zwischen 0 und 200 °C, wobei tiefere Temperaturen zu langen Reaktionszeiten führen und bei höheren Temperaturen zunehmend die Gefahr des Auftretens von Nebenprodukten besteht. Bevorzugt arbeitet man zwischen 50 und 150 °C, wobei besonders vorteilhaft die Reaktion in siedendem Methanol, Äthanol, Propanol oder Eisessig durchgeführt wird. Der quantitative Ablauf der Reaktion wird zweckmässig im Dünnschichtchromatogramm an Kieselgelplatten verfolgt.

Das Reaktionsgemisch arbeitet man vorteilhaft analog der in Verfahrensweise a) angegebenen Weise auf.

Die Verbindungen der allgemeinen Formel IV erhält man nach an sich bekannten Methoden, beispielsweise analog der in der DE-OS 24 36 263 angegebenen Verfahrensweisen. Dabei sollten möglichst milde Reaktionsbedingungen und Reaktionstemperaturen sowie die Aufarbeitungsbedingungen unterhalb 40 °C gewählt werden, wenn man die Herstellung möglichst reiner Verbindungen der Formel IV anstrebt. Nach Verfahrensweise c) bringt man Verbindungen der allgemeinen Formel V mit Verbindungen der Formel VI vorteilhaft in einem polaren organischen Lösungsmittel, wie beispielsweise in niederen Alkoholen mit 1 bis 4 C-Atomen, Äthylenglykolmono- und Äthylenglykoldimethyläther, Diäthylenglykolmono- oder Diäthylenglykoldimethyläther, Aceton, Essigester, Dimethylformamid, zur Reaktion.

Man führt die Umsetzung vorteilhaft zwischen 0 und 80 °C, vorzugsweise zwischen 15 und 40 °C durch und erwärmt nach Abklingen der exothermen Reaktion bis zur vollständigen Bildung der Verbindungen der Formel I auf Temperaturen zwischen 60° und 140 °C. Der Reaktionsablauf wird zweckmässigerweise dünnschichtchromatographisch an Kieselgelplatten verfolgt. Die Reaktionsdauer liegt zwischen 5 und 60 Stunden. Als besonders geeignet für diese Reaktion erweisen sich insbesondere Verbindungen V, die an der Sulfamoylgruppe neben $R^6$ = Wasserstoff einen voluminösen organischen Rest $R^7$, wie beispielsweise tert.-Butyl, tragen oder solche Verbindungen V, in denen $R^6$ und $R^7$ einen organischen Rest als Substituenten tragen.

Zur Durchführung der Verfahrensweise d) bringt man die Mercaptoketone der Formel V in einem wasserfreien, polaren inerten Lösungsmittel, wie beispielsweise in Essigsäuremethylester, Essigsäureäthylester, Dioxan oder Tetrahydrofuran mit der Carbodiimiden der allgemeinen Formel VII im Molverhältnis 1 : 1 zur Umsetzung. Man führt die Reaktion in einem Temperaturbereich zwischen 0 und 40 °C, bevorzugt zwischen 10 und 30 °C durch. Nach Abklingen der schwach exothermen Reaktion rührt man etwa 10-20 Stunden bei 20 bis 40 °C. Nach dem Zufügen der gleichen Volumenmenge eines protischen Lösungsmittels, bevorzugt von Methanol, Äthanol, Propanol, Isopropanol, n-Butanol, Eisessig oder auch Wasser sowie von Gemischen der genannten Lösungsmittel erwärmt man weitere 2 bis 70 Stunden auf Temperaturen zwischen 60 und 140 °C. Der Reaktionsablauf wird zweckmässigerweise dünnschichtchromatographisch an Kieselgelplatten verfolgt.

Die in Verfahrensweise c) und d) verwendeten Verbindungen der Formel V können nach literaturbekannten Methoden hergestellt werden (z. B. DE-OS 24 36 263). Ebenso ist die Darstellung von Verbindungen der allgemeinen Formeln VI und VII in der Literatur beschrieben (z. B. Chem. Ber. *97,* 1232 (1964), Bull. Chem. Soc. Jap. *46,* 1765 (1973), Angew. Chem. *74,* 214 (1962), Bull. Soc. Chim. Jap. *38,* 1806 (1965)).

Gemäss Verfahrensweise e) werden die Verbindungen der allgemeinen Formel VIII mit einem geeigneten Oxidationsmittel, vorzugsweise mit aktiven Mangan-IV-oxid, in die Verbindungen der Formel I übergeführt. Als Lösungsmittel verwendet man vorzugsweise halogenierte Kohlenwasserstoffe, wie z. B. Methylenchlorid, Chloroform, Tetrachloräthan, besonders bevorzugt aber Acetonitril bzw. Gemische der genannten Lösungsmittel mit Acetonitril. Man arbeitet in einem Temperaturbereich zwischen 0-40 °C, vorzugsweise zwischen 20-30 °C über eine Dauer von 10 bis 60 Stunden, filtriert anschliessend das Oxidationsmittel ab und erhitzt zur Vervollständigung der Reaktion nach Zugabe des gleichen Volumens eines protischen Lösungsmittels wie Methanol, Äthanol, Propanol, Isopropanol, Butanol oder Eisessig über 1 bis 30 Stunden auf Temperaturen zwischen 60-140 °C.

Zu Verbindungen der allgemeinen Formel VIII gelangt man analog den Angaben in der DE-OS 24 36 263 durch Umsetzung von Verbindungen der allgemeinen Formel XV

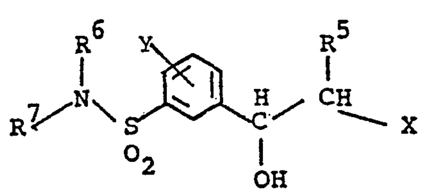

(XV)

worin die Substituenten X, Y und $R^5$ bis $R^7$ die angegebene Bedeutung besitzen, mit Thioharnstoffen der allgemeinen Formel III.

Gemäss Verfahrensweise f) bringt man Verbindungen der allgemeinen Formel IX, worin Y nicht für Brom oder Jod steht und worin $R^6$ und $R^7$ verschieden von Wasserstoff sind, mit den Verbindungen der Formel X zur Reaktion. Die Verbindungen IX und X werden vorteilhaft im Molverhältnis 1 : 1 bis 1 : 1,5 in einem für metallorganische Reaktionen üblichen inerten und wasserfreien Lösungsmittel, vorzugsweise in Tetrahydrofuran, in einem bevorzugten Temperaturbereich zwischen − 30° bis + 60 °C umgesetzt. Nach Beendigung der Umsetzung werden die Reaktionsprodukte in einer für metallorganische Umsetzungen üblichen Weise hydrolysiert, wobei man beispielsweise das Reaktionsgemisch bei Temperaturen zwischen − 5° und + 20 °C unter Aufrechterhaltung eines pH-Bereiches von 6 bis 8 in eine wässrige gesättigte Ammoniumchlorid-Lösung einträgt. Zur Vervollständigung der Überführung in die erfindungsgemässen Verbindungen I kann man unter dünnschichtchromatographischer Kontrolle des Reaktionsfortgangs das Hydrolysegemisch auf Temperaturen von 40-100 °C, bevorzugt von 60-80 °C erwärmen. Vorteilhaft arbeitet man das noch nicht vollständig in die Verbindungen I umgewandelte Produktgemisch durch Filtration oder Extraktion mit einem geeigneten Lösungsmittel, wie Essigsäuremethylester, Essigsäureäthylester, Nitromethan auf und behandelt es anschliessend nach Verfahrensweise b).

Herstellungsweisen der Verbindungen IX sowie deren Vorprodukte sind in der Literatur beschrieben (z. B. DE-OS 24 36 263). Die Verbindungen der Formel X werden z. B. in bekannter Weise durch Umsetzung von α-Halogencarbonsäureester XVI mit B = $OR^8$

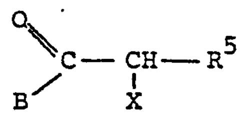

(XVI)

worin $R^5$ und X die angegebene Bedeutung besitzen und $R^8$ vorzugsweise Phenyl oder niederes Alkyl, wie Methyl oder Äthyl, bedeuten, mit Thioharnstoffen der allgemeinen Formel III erhalten. Entsprechend eignen sich auch α-Halogencarbonsäuren (B = OH) und α-Halogencarbonsäurechloride (B = Cl).

Die Verbindungen der Formel I können in einem geeigneten Lösungsmittel mit einer Säure der Formel H—A reversibel umgesetzt werden. Man kann dabei die Verbindungen I in die reinen Säuren, bevorzugt bei Temperaturen zwischen 0° und 60 °C eintragen, sofern diese flüssig sind bzw. einen nicht wesentlich höheren Schmelzpunkt als 60 °C besitzen und sofern sie keine Nebenreaktionen veranlassen. Vorteilhaft arbeitet man aber in einem Lösungsmittel, wie beispielsweise in Wasser oder einem organischen Lösungsmittel, wie beispielsweise in Dioxan, Tetrahydrofuran, Äther, einem Essigsäureniederalkylester mit 1 bis 4 C-Atomen im Alkylteil, Acetonitril, Nitromethan, Aceton, Methyl-äthyl-keton usw., wobei sich niedere Alkohole mit 1 bis 4 und Carbonsäuren mit 2 bis 4 C-Atomen als besonders geeignet erwiesen. Dabei werden pro Mol der Verbindungen I 1-1,5 Mol der Säuren H—A angewendet, man kann aber auch grössere Mengen an Säure verwenden. Zweckmässigerweise arbeitet man bei Temperaturen zwischen 0° und 120 °C, bevorzugt zwischen 10° und 60 °C. Die Reaktion ist mässig exotherm.

Beim Arbeiten in wässriger Lösung kommt es nach Zugabe von Säuren H—A im allgemeinen zur sofortigen Auflösung der Verbindungen I und nur in seltenen Fällen zur Abscheidung der entsprechenden Säureadditionsverbindungen. Zweckmässigerweise isoliert man die erfindungsgemässen Salze beim Erhalten einer Lösung durch schonendes Verdampfen des Wassers, vorzugsweise durch Gefriertrocknung. Beim Arbeiten in organischen Lösungsmitteln scheiden sich die Säureadditionssalze vielfach nach Zugabe der jeweiligen Säure H—A schwerlöslich ab. Wird eine Lösung erhalten, so bringt man die Säureadditions-Verbindungen gegebenenfalls nach vorangehender Konzentrierung mit einem geeigneten Fällungsmittel zur Abscheidung. Als Fällungsmittel eignen sich die zum gleichen Zweck in Verfahren a) beschriebenen Solvenzien.

Die Säureadditionsprodukte fallen auch bei sehr hohem Reinigungsgrad sehr oft in Form zäher Öle oder amorpher glasartiger Produkte an. Diese amorphen Produkte lassen sich vielfach gegebenenfalls durch Erwärmen auf 40° bis 80 °C unter Behandlung mit einem organischen Lösungsmittel zur Kristallisation bringen. Als kristallisationsfördernde Solvenzien eignen sich insbesondere Essigsäureniederalkylester mit 1 bis 4 C-Atomen im Alkylteil, wie Essigsäuremethylester, Essigsäureäthylester, Essigsäure-n-butylester, sowie niedere Dialkylketone, wie Aceton oder Methyl-äthyl-keton, niedere Dialkyläther wie Diäthyläther, Diisopropyläther oder Di-n-butyläther, sowie Acetonitril, Nitromethan und auch in einigen Fällen auch niedere Alkohole, wie Methanol, Äthanol, Isopropanol oder n-Butanol.

Die Säureadditionsprodukte können in einem geeigneten Lösungsmittel durch Behandlung mit Basen zu den Verbindungen der allgemeinen Formel I deprotoniert werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natriumcarbonat, Kaliumcarbonat, Natrium- oder Kaliumhydrogencarbonat, Ammoniak und Amine, wie Triäthylamin, Dicyclohexylamin, Piperidin, Methyl-dicyclohexylamin in Frage.

Beim Arbeiten im wässrigen Medium scheiden sich die freien basischen Verbindungen I schwerlöslich ab und können durch Filtration oder Extraktion mit einem organischen Lösungsmittel, vorzugsweise mit Essigsäureäthylester, abgetrennt und isoliert werden. Als organische Reaktionsmedien eignen sich in besonderer Weise niedere Alkohole mit 1 bis 4 C-Atomen, vorzugsweise Methanol und Äthanol, es können jedoch auch Essigester, Diäthyläther, Tetrahydrofuran, Dioxan, Diäthylenglykol-dimethyläther, Dimethylformamid u. a. m. verwendet werden. Die Reaktion zu den Verbindungen I findet spontan statt. Die Reaktion wird zwischen – 35° und 100 °C, bevorzugt zwischen 0° und 60 °C durchgeführt. Wird ein mit Wasser mischbares organisches Lösungsmittel verwendet, so fällt man gegebenenfalls nach vorangehender Konzentrierung des Reaktionsgemisches die freien Basen der Formel I durch Zugabe von Wasser aus. Bei Verwendung eines mit Wasser nicht mischbaren Lösungsmittels arbeitet man vorteilhafterweise so, dass man nach der Umsetzung das Reaktionsgemisch mit Wasser wäscht und das organische Lösungsmittel gegebenenfalls nach vorangehender Trocknung verdampft.

Lässt man auf Verbindungen der Formel I, worin $R^6$ und/oder $R^7$ Wasserstoff bedeuten, mindestens 1 Mol einer hinreichend starken Base einwirken, so erhält man unter Deprotonierung der Sulfonamidgruppe Salze der allgemeinen Formel XVII

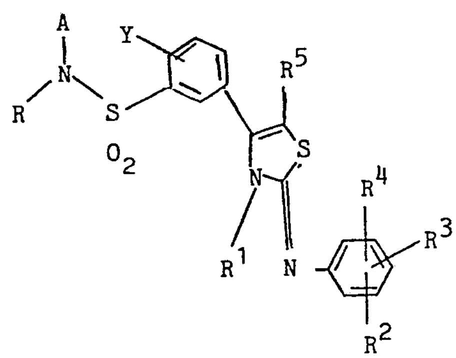

(XVII)

worin A das Kation eines Alkali- oder Erdalkalimetalls ist und $R^1$ bis $R^5$ sowie Y die angegebene Bedeutung haben und R die Bedeutung von $R^6$ oder $R^7$ besitzt.

Als Basen können Hydroxide der Alkali- und Erdalkalimetalle, vorzugsweise NaOH und KOH, Alkali- und Erdalkalialkoholate, $NaOCH_3$ und $NaOC_2H_5$, NaH Natrium-methylsulfinylmethid usw. verwendet werden.

Als Lösungsmittel verwendet man Wasser oder polare organische Lösungsmittel wie Methanol, Äthanol, Isopropanol, n-Butanol, Dimethylformamid, Dimethylsulfoxid, Diäthylenglykol-dimethyläther, Acetonitril.

Durch Zugabe eines Mols einer geeigneten Säure H—A erhält man die erfindungsgemässen Verbindungen I zurück, wobei als Säuren auch Ammoniumsalze verwendet werden können.

Diese reversible Säure-Base-Reaktion kann man zur Reinigung der Verbindungen I heranziehen. Ausserdem kann man die Salze XVII verwenden, um über Alkylierungsreaktionen an der Sulfonamidgruppe entsprechend umgewandelte Verbindungen der Formel I herzustellen.

Bei Alkylierungsreaktionen kann Wasser als Solvens verwendet werden. Vorzugsweise arbeitet man jedoch in den aufgeführten polaren organischen Lösungsmitteln, besonders vorteilhaft in einem Zweiphasengemisch aus Wasser und einer mit Wasser nicht mischbaren organischen Phase wie z. B. Toluol, Benzol, Xylol, Methylenchlorid, Chloroform, Tetrachlorkohlenstoff, Essigester bzw. einem Gemisch der genannten Lösungsmittel. Vorteilhaft kann auch die Anwendung eines Phasentransferkatalysators, wie beispielsweise Tetra-n-butylammoniumchlorid, Benzyl-triäthylammoniumchlorid, Benzyldimethyl-tetradecyl-ammoniumchlorid, Tetra-n-butylphosphoniumchlorid, Dicyclohexyl-[18] krone-6, sein. Man arbeitet in einem Temperaturbereich von – 20° bis + 100 °C, vorzugsweise zwischen + 10 und 40 °C, wobei man den Reaktionsverlauf dünnschichtchromatographisch verfolgt. Es werden übliche Alkylierungsmittel der allgemeinen Formel R-X verwendet, worin R die Bedeutung von $R^6$ oder $R^7$ besitzt und X beispielsweise für Brom, Chlor, Jod, $—O—SO_2—OR$, $—O—SO_2CH_3$,

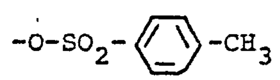

steht.

Die Salze XVII erzeugt man vorteilhaft ohne nachfolgende Isolierung in der angegebenen Weise im Reaktionsgemisch durch Einwirkung einer der aufgeführten Basen auf die Verbindungen I und nachfolgende oder parallele Zugabe eines der bezeichneten Alkylierungsmittel R-X.

Die Verbindungen der Formel XVII

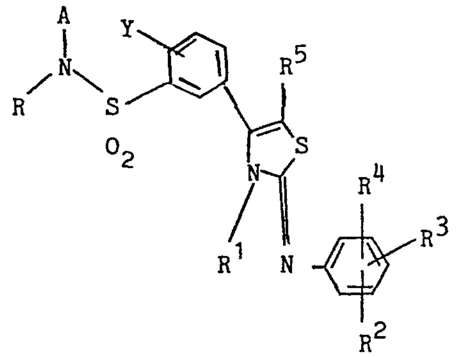

(XVII)

worin $R^1$ bis $R^5$ und Y die zu Formel I genannten Bedeutungen hat, R die Bedeutung von $R^6$ oder $R^7$ besitzt und A das Kation eines Alkali- oder Erdalkalimetalls bedeutet, sind neu. Die Erfindung betrifft daher auch diese Verbindungen. Sie eignen sich insbesondere als Zwischenprodukte bei der Alkylierung von Verbindungen der Formel I, worin $R^6$ und/oder $R^7$ Wasserstoff bedeuten.

Bevorzugt von den erfindungsgemässen Verbindungen sind diejenigen der allgemeinen Formel I, in denen die Substituenten die nachfolgenden, in Tabelle 1 beschriebene Bedeutungen besitzen :

Tabelle 1

$R^1$ = Methyl, Äthyl, Cyclopropyl
$R^2$ = Wasserstoff, Methyl, Äthyl, Brom, Chlor, Fluor, Trifluormethyl, Methoxy, Äthoxy, $—N(CH_3)_2$, $—N(C_2H_5)_2$
$R^3$ = Wasserstoff, Methyl, Äthyl, Chlor
$R^4$ = Wasserstoff, Methyl
$R^5$ = Wasserstoff
$R^6$, $R^7$ = Wasserstoff, Methyl, Äthyl, wobei $R^6$ und $R^7$ gleich oder Verschieden sind
Y = Brom, Chlor, Methyl in 2-, 3- oder 4-Stellung zum Thiazolring

wobei als besonders bevorzugte Verbindungen solche Verbindungen der Formel I in Betracht kommen, worin die Substituenten die nachfolgenden, in Tabelle 2 angegebenen Bedeutungen besitzen :

Tabelle 2

$R^1$ = Methyl, Äthyl
$R^2$ = Wasserstoff, Methyl, Chlor, Methoxy, Fluor, Trifluormethyl
$R^3$ = Wasserstoff, Methyl
$R^4$ = Wasserstoff
$R^5$ = Wasserstoff
$R^6$, $R^7$ = Methyl, Äthyl
Y = Chlor in 2-, 3- oder 4-Stellung zum Thiazolring

Erfindungsgemäss können ausser den in den Ausführungsbeispielen beschriebenen Thiazolinderivaten auch die in der folgenden Tabelle 3 zusammengestellten Verbindungen der allgemeinen Formeln I und IV bzw. deren Säureadditionsprodukte erhalten werden :

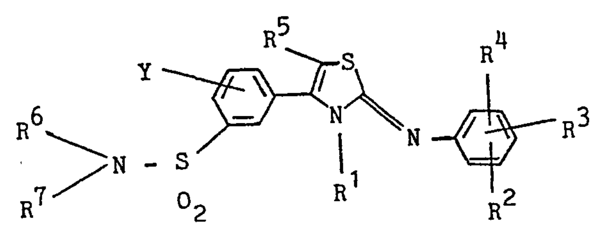

(I)

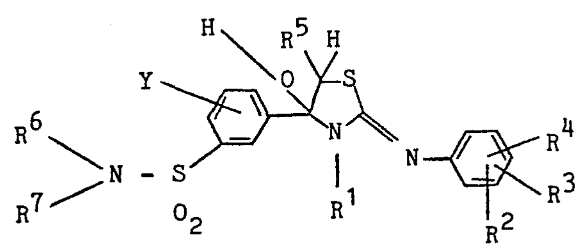

(IV)

Tabelle 3

(Zeichenerklärung : Me = Methyl, Et = Ethyl, Prop = Propyl, But = Butyl, Pent = Pentyl, Hex = Hexyl, i = Iso, sek. = sekundär, c = cyclo, die vor dem Substituenten angegebenen Nummern bezeichnen die Stellung von Y am Phenylrest, wobei der Thiazolring in 1- und der Sulfamoylrest in 3-Position festgelegt sind)

| Lauf-.Nr. | $R^1$ | $R^2$ | $R^3$ | $R^4$ | $R^5$ | $R^6$ | $R^7$ | Y |
|---|---|---|---|---|---|---|---|---|
| 1 | c-Prop | H | H | H | H | H | H | 4-Cl |
| 2 | i-Prop | H | H | H | H | H | H | 4-Cl |
| 3 | Me | 2-Me | 6-Me | H | H | H | H | 4-Cl |
| 4 | Me | 3-Me | 5-Me | H | H | H | H | 4-Cl |
| 5 | Me | 2-Me | 4-Me | 6-Me | H | H | H | 4-Cl |
| 6 | Et | 2-Me | 4-Me | H | H | H | H | 4-Cl |
| 7 | Me | 2-Et | 4-Et | H | H | H | H | 4-Cl |
| 8 | Me | 2-Me | 6-Cl | H | H | H | H | 4-Cl |
| 9 | Me | H | H | H | Et | H | H | 4-Cl |
| 10 | Me | 2-Br | H | H | H | H | H | 4-Cl |
| 11 | Me | 2-Br | H | H | Et | H | H | 4-Cl |
| 12 | Me | 4-Br | H | H | H | H | H | 4-Cl |
| 13 | Me | 2-Me | H | H | Me | H | H | 4-Cl |
| 14 | Me | 2-Me | H | H | Et | H | H | 4-Cl |
| 15 | Me | 2-Me | 4-Me | H | Me | H | H | 4-Cl |
| 16 | Et | 2-Me | 4-Me | H | Me | H | H | 4-Cl |
| 17 | c-Prop | 2-Me | 4-Me | H | H | H | H | 4-Cl |
| 18 | Et | 2-Cl | H | H | H | H | H | 4-Cl |
| 19 | Me | $2-CF_3$ | H | H | H | H | H | 4-Cl |
| 20 | Et | $2-CF_3$ | H | H | H | H | H | 4-Cl |
| 21 | Et | 2-Me | H | H | H | H | Me | 4-Cl |
| 22 | Me | 2-Me | 4-Me | H | H | H | Me | 4-Cl |
| 23 | Me | 2-Et | 4-Et | H | H | H | Et | 4-Cl |
| 24 | Me | $3-CF_3$ | H | H | H | H | Me | 4-Cl |
| 25 | Me | 4-F | H | H | H | H | Et | 4-Cl |
| 26 | Me | 4-Br | H | H | H | H | Me | 4-Cl |
| 27 | c-Prop | H | H | H | H | H | Me | 4-Cl |
| 28 | c-Prop | 2-Me | H | H | H | H | Me | 4-Cl |
| 29 | Me | 2-Br | H | H | H | H | Me | 4-Cl |
| 30 | Me | 2-Me | 4-Me | 6-Me | H | H | Me | 4-Cl |
| 31 | Me | 2-Me | H | H | H | Me | Me | 4-Cl |
| 32 | Me | 2-Cl | H | H | H | H | But | 4-Cl |
| 33 | Me | 2-Me | 4-Me | H | H | H | c-Prop | 4-Cl |
| 34 | Me | 2-Me | 3-Me | H | H | H | c-Prop | 4-Cl |
| 35 | Me | 4-F | H | H | H | H | c-Prop | 4-Cl |
| 36 | Et | 2-Me | 4-Me | H | H | H | c-Hex | 4-Cl |
| 37 | Et | 2-Cl | H | H | H | H | c-Hex | 4-Cl |
| 38 | c-Prop | H | H | H | H | H | c-Hex | 4-Cl |

Tabelle 3 (Fortsetzung)

| Lauf-.Nr. | $R^1$ | $R^2$ | $R^3$ | $R^4$ | $R^5$ | $R^6$ | $R^7$ | Y |
|---|---|---|---|---|---|---|---|---|
| 39 | Me | 2-Cl | H | H | H | H | i-Prop | 4-Cl |
| 40 | Me | 2-Me | 5-Me | H | H | H | i-Prop | 4-Cl |
| 41 | Et | 2-Me | H | H | H | H | sek.But | 4-Cl |
| 42 | Me | 2-Cl | H | H | H | H | sek.But | 4-Cl |
| 43 | Me | 2-Me | 4-Me | H | H | H | i-But | 4-Cl |
| 44 | Me | 2-Me | 4-Me | 6-Me | H | H | Et | 4-Cl |
| 45 | Me | 4-F | H | H | H | H | Hex | 4-Cl |
| 46 | Me | H | H | H | Me | Me | Me | 4-Cl |
| 47 | Et | H | H | H | Et | Me | Me | 4-Cl |
| 48 | Me | 2-Me | H | H | Et | Me | Me | 4-Cl |
| 49 | Me | 2-Me | 4-Me | H | Me | Me | Me | 4-Cl |
| 50 | Me | 2-Cl | H | H | Et | Me | Me | 4-Cl |
| 51 | Me | 3-$CF_3$ | H | H | Me | Me | Me | 4-Cl |
| 52 | Me | 2-Cl | 4-Me | H | Me | Me | Me | 4-Cl |
| 53 | Me | 4-Prop | H | H | H | Me | Me | 4-Cl |
| 54 | Me | 4-But | H | H | H | Me | Me | 4-Cl |
| 55 | Me | 3-Et | H | H | H | Me | Me | 4-Cl |
| 56 | Me | 3-Prop | H | H | H | Me | Me | 4-Cl |
| 57 | Me | 3-But | H | H | H | Me | Me | 4-Cl |
| 58 | Me | 2-Et | 4-Et | H | H | Me | Me | 4-Cl |
| 59 | Me | 2-Me | 5-Me | H | H | Me | Me | 4-Cl |
| 60 | Me | 2-Me | 6-Me | H | H | Me | Me | 4-Cl |
| 61 | Me | 2-Me | 5-Et | H | H | Me | Me | 4-Cl |
| 62 | Me | 2-Me | 5-Prop | H | H | Me | Me | 4-Cl |
| 63 | Me | 2-Me | 5-i-But | H | H | Me | Me | 4-Cl |
| 64 | Me | 2-Et | 5-Et | H | H | Me | Me | 4-Cl |
| 65 | Me | 2-Me | 5-But | H | H | Me | Me | 4-Cl |
| 66 | Me | 3-Et | 4-Me | H | H | Me | Me | 4-Cl |
| 67 | Me | 3-Cl | 5-Et | H | H | Me | Me | 4-Cl |
| 68 | Me | 3-Br | 5-Et | H | H | Me | Me | 4-Cl |
| 69 | Me | 3-Et | 4-Cl | H | H | Me | Me | 4-Cl |
| 70 | Me | 3-Et | 4-Br | H | H | Me | Me | 4-Cl |
| 71 | Me | 2-Cl | 5-Me | H | H | Me | Me | 4-Cl |
| 72 | Me | 2-Cl | 5-Et | H | H | Me | Me | 4-Cl |
| 73 | Me | 2-Cl | 5-Prop | H | H | Me | Me | 4-Cl |
| 74 | Me | 2-Cl | 5-But | H | H | Me | Me | 4-Cl |
| 75 | Me | 2-Cl | 5-i-But | H | H | Me | Me | 4-Cl |
| 76 | Me | 2-Br | 5-Et | H | H | Me | Me | 4-Cl |
| 77 | Me | 2-Br | 5-Prop | H | H | Me | Me | 4-Cl |
| 78 | Me | 2-MeO | 5-Me | H | H | Me | Me | 4-Cl |
| 79 | Me | 2-MeO | 5-Et | H | H | Me | Me | 4-Cl |
| 80 | Me | 2-MeO | 5-i-But | H | H | Me | Me | 4-Cl |

Tabelle 3 (Fortsetzung)

| Lauf-Nr. | $R^1$ | $R^2$ | $R^3$ | $R^4$ | $R^5$ | $R^6$ | $R^7$ | Y |
|---|---|---|---|---|---|---|---|---|
| 81 | Me | 2-EtO | 5-Me | H | H | Me | Me | 4-Cl |
| 82 | Me | 2-EtO | 5-Et | H | H | Me | Me | 4-Cl |
| 83 | Me | 2-PropO | 4-Et | H | H | Me | Me | 4-Cl |
| 84 | Me | 2-PropO | 5-Et | H | H | Me | Me | 4-Cl |
| 85 | Me | 3-Me | 4-OMe | H | H | Me | Me | 4-Cl |
| 86 | Me | 3-Me | 4-OEt | H | H | Me | Me | 4-Cl |
| 87 | Me | 3-Et | 4-OMe | H | H | Me | Me | 4-Cl |
| 88 | Me | 2-Me | 4-Me | 6-Me | H | Me | Me | 4-Cl |
| 89 | Me | 2-Me | 4-Me | 5-Et | H | Me | Me | 4-Cl |
| 90 | Me | 2-Cl | 4-Cl | 5-Et | H | Me | Me | 4-Cl |
| 91 | Me | 2-Me | 3-Me | 5-Et | H | Me | Me | 4-Cl |
| 92 | Me | 2-MeO | 3-Me | 5-Me | H | Me | Me | 4-Cl |
| 93 | Me | 2-MeO | 3-Me | 5-Et | H | Me | Me | 4-Cl |
| 94 | Me | 2-MeO | 3-Cl | 5-Et | H | Me | Me | 4-Cl |
| 95 | Me | 2-MeO | 3-MeO | 5-Me | H | Me | Me | 4-Cl |
| 96 | Me | 2-MeO | 3-MeO | 5-Et | H | Me | Me | 4-Cl |
| 97 | Me | 2-MeO | 4-MeO | 5-Et | H | Me | Me | 4-Cl |
| 98 | Me | 2-Me | 4-Me | 5-Me | H | Me | Me | 4-Cl |
| 99 | Et | 2-Me | 4-Me | H | H | Me | Me | 4-Cl |
| 100 | Et | 2-Me | 4-Cl | H | H | Me | Me | 4-Cl |
| 101 | Et | $3-CF_3$ | H | H | H | Me | Me | 4-Cl |
| 102 | Et | 4-F | H | H | H | Me | Me | 4-Cl |
| 103 | Et | 2-Et | 4-Et | H | H | Me | Me | 4-Cl |
| 104 | Et | $2-CF_3$ | H | H | H | Me | Me | 4-Cl |
| 105 | Et | $4-NEt_2$ | H | H | H | Me | Me | 4-Cl |
| 106 | Me | $2-CF_3$ | H | H | H | Me | Me | 4-Cl |
| 107 | Et | 4-OMe | H | H | H | Me | Me | 4-Cl |
| 108 | c-Prop | 2-Me | H | H | H | Me | Me | 4-Cl |
| 109 | c-Prop | 2-Me | 4-Me | H | H | Me | Me | 4-Cl |
| 110 | c-Prop | 2-Cl | H | H | H | Me | Me | 4-Cl |
| 111 | c-Prop | 4-OMe | H | H | H | Me | Me | 4-Cl |
| 112 | Me | 2-Me | 4-Me | H | H | Et | Et | 4-Cl |
| 113 | Me | 2-Cl | H | H | H | Et | Et | 4-Cl |
| 114 | Me | 2-Br | H | H | H | Et | Et | 4-Cl |
| 115 | Me | 2-Me | 6-Me | H | H | Et | Et | 4-Cl |
| 116 | Me | $3-CF_3$ | H | H | H | Et | Et | 4-Cl |
| 117 | Me | $4-CF_3$ | H | H | H | Et | Et | 4-Cl |
| 118 | Me | 4-MeO | H | H | H | Et | Et | 4-Cl |
| 119 | Me | 4-F | H | H | H | Et | Et | 4-Cl |
| 120 | Me | 2-Me | 4-Me | H | Me | Et | Et | 4-Cl |
| 121 | Me | H | H | H | H | H | H | 4-Br |
| 122 | Me | 2-Me | H | H | H | H | H | 4-Br |

Tabelle 3 (Fortsetzung)

| Lauf-Nr. | $R^1$ | $R^2$ | $R^3$ | $R^4$ | $R^5$ | $R^6$ | $R^7$ | Y |
|---|---|---|---|---|---|---|---|---|
| 123 | Me | 2-Cl | H | H | H | H | H | 4-Br |
| 124 | Me | 2-Me | 4-Me | H | H | H | H | 4-Br |
| 125 | Me | 2-Me | 6-Me | H | H | H | H | 4-Br |
| 126 | Me | 4-MeO | H | H | H | H | H | 4-Br |
| 127 | Me | 4-F | H | H | H | H | H | 4-Br |
| 128 | Me | $3-CF_3$ | H | H | H | H | H | 4-Br |
| 129 | Me | 2-Cl | 6-Cl | H | H | Me | Me | 4-Cl |
| 130 | Me | 2-Cl | 4-Cl | H | H | Me | Me | 4-Cl |
| 131 | Me | 2-Me | H | H | H | Me | Me | 4-Br |
| 132 | Me | 2-Cl | H | H | H | Me | Me | 4-Br |
| 133 | Me | 2-Me | 4-Me | H | H | Me | Me | 4-Br |
| 134 | Me | 2-Me | 3-Me | H | H | Me | Me | 4-Br |
| 135 | Me | 2-Me | 6-Me | H | H | Et | Et | 4-Br |
| 136 | Me | 2-Cl | 6-Cl | H | H | Et | Et | 4-Br |
| 137 | Me | 2-Cl | 6-Cl | H | H | Me | Me | 4-Br |
| 138 | Me | 2-Me | 6-Me | H | H | Me | Me | 4-Br |
| 139 | Me | 4-MeO | H | H | H | Me | Me | 4-Br |
| 140 | Me | 4-F | H | H | H | Me | Me | 4-Br |
| 141 | Me | H | H | H | H | H | H | 4-F |
| 142 | Me | 2-Cl | H | H | H | H | H | 4-F |
| 143 | Et | 2-Cl | H | H | H | H | H | 4-F |
| 144 | Me | 2-Me | 4-Me | H | H | H | H | 4-F |
| 145 | Me | 2-Me | H | H | H | Me | Me | 4-F |
| 146 | Me | 2-Me | 4-Me | H | H | Me | Me | 4-F |
| 147 | Me | 2-Cl | H | H | H | Me | Me | 4-F |
| 148 | Me | 2-Br | H | H | H | Me | Me | 4-F |
| 149 | Me | 4-Cl | H | H | H | Me | Me | 4-F |
| 150 | Me | 2-Cl | 4-Cl | H | H | Me | Me | 4-F |
| 151 | Me | 4-F | H | H | H | Me | Me | 4-F |
| 152 | Me | 4-MeO | H | H | H | Me | Me | 4-F |
| 153 | Me | 2-Me | 3-Me | H | H | Me | Me | 4-F |
| 154 | Et | $3-CF_3$ | H | H | H | Me | Me | 4-F |
| 155 | Et | 2-Cl | H | H | H | Me | Me | 4-F |
| 156 | Et | 2-Me | 4-Me | H | H | Me | Me | 4-F |
| 157 | Me | 2-Me | H | H | H | Et | Et | 4-F |
| 158 | Me | 2-Me | 4-Me | H | H | Et | Et | 4-F |
| 159 | Me | 2-Cl | H | H | H | Et | Et | 4-F |
| 160 | Me | 4-MeO | H | H | H | Et | Et | 4-F |
| 161 | Me | H | H | H | H | H | H | 4-H |
| 162 | Me | Cl | H | H | H | H | H | 4-H |
| 163 | Me | Br | H | H | H | H | H | 4-H |
| 164 | Me | 2-Me | 4-Me | H | H | H | H | 4-H |

Tabelle 3 (Fortsetzung)

| Lauf-Nr. | $R^1$ | $R^2$ | $R^3$ | $R^4$ | $R^5$ | $R^6$ | $R^7$ | Y |
|---|---|---|---|---|---|---|---|---|
| 165 | Et | Cl | H | H | H | H | H | 4-H |
| 166 | Et | H | H | H | H | H | H | 4-H |
| 167 | c-Prop | H | H | H | H | H | H | 4-H |
| 168 | Me | H | H | H | H | Me | Me | 4-H |
| 169 | Me | 2-Cl | H | H | H | Me | Me | 4-H |
| 170 | Me | 2-Br | H | H | H | Me | Me | 4-H |
| 171 | Me | 2-Me | 3-Me | H | H | Me | Me | 4-H |
| 172 | Me | 2-Me | 4-Me | H | H | Me | Me | 4-H |
| 173 | Me | 2-Me | 6-Me | H | H | Me | Me | 4-H |
| 174 | Et | 2-Me | 4-Me | H | H | Me | Me | 4-H |
| 175 | Me | 4-OMe | H | H | H | Me | Me | 4-H |
| 176 | Me | $3-CF_3$ | H | H | H | Me | Me | 4-H |
| 177 | Et | 2-Me | H | H | H | Me | Me | 4-H |
| 178 | c-Prop | 2-Me | H | H | H | Me | Me | 4-H |
| 179 | Et | 2-Cl | H | H | H | Me | Me | 4-H |
| 180 | Me | 4-Cl | H | H | H | Me | Me | 4-H |
| 181 | Me | H | H | H | H | H | H | 4-Me |
| 182 | Et | H | H | H | H | H | H | 4-Me |
| 183 | Me | 2-Me | H | H | H | H | H | 4-Me |
| 184 | Me | 2-Et | H | H | H | H | H | 4-Me |
| 185 | Et | 2-Me | H | H | H | H | H | 4-Me |
| 186 | Me | 2-Cl | H | H | H | H | H | 4-Me |
| 187 | Me | 2-Br | H | H | H | H | H | 4-Me |
| 188 | Me | 2-Cl | 4-Me | H | H | H | H | 4-Me |
| 189 | Et | 2-Cl | H | H | H | H | H | 4-Me |
| 190 | Me | 4-MeO | H | H | H | H | H | 4-Me |
| 191 | Me | 4-Cl | H | H | H | H | H | 4-Me |
| 192 | Me | 4-F | H | H | H | H | H | 4-Me |
| 193 | Et | 4-F | H | H | H | H | H | 4-Me |
| 194 | Me | $3-CF_3$ | H | H | H | H | H | 4-Me |
| 195 | Me | $4-CF_3$ | H | H | H | H | H | 4-Me |
| 196 | Me | 2-Me | 4-Me | H | H | H | H | 4-Me |
| 197 | Et | 2-Me | 4-Me | H | H | H | H | 4-Me |
| 198 | Me | 2-Me | 3-Me | H | H | H | H | 4-Me |
| 199 | Me | 2-Me | 6-Me | H | H | H | H | 4-Me |
| 200 | Me | 2-Cl | 6-Cl | H | H | H | H | 4-Me |
| 201 | Me | H | H | H | H | H | Me | 4-Me |
| 202 | Me | 2-Me | H | H | H | H | Me | 4-Me |
| 203 | Me | 2-Me | 4-Me | H | H | H | Me | 4-Me |
| 204 | Me | 2-Cl | H | H | H | H | Me | 4-Me |
| 205 | Me | H | H | H | H | Me | Me | 4-Me |
| 206 | Et | H | H | H | H | Me | Me | 4-Me |

Tabelle 3 (Fortsetzung)

| Lauf-Nr. | $R^1$ | $R^2$ | $R^3$ | $R^4$ | $R^5$ | $R^6$ | $R^7$ | Y |
|---|---|---|---|---|---|---|---|---|
| 207 | c-Prop | H | H | H | H | Me | Me | 4-Me |
| 208 | Me | 2-Me | H | H | H | Me | Me | 4-Me |
| 209 | Me | 2-Et | H | H | H | Me | Me | 4-Me |
| 210 | Et | 2-Me | H | H | H | Me | Me | 4-Me |
| 211 | Me | 2-Cl | H | H | H | Me | Me | 4-Me |
| 212 | Me | 2-Br | H | H | H | Me | Me | 4-Me |
| 213 | Et | 2-Cl | H | H | H | Me | Me | 4-Me |
| 214 | c-Prop | 2-Cl | H | H | H | Me | Me | 4-Me |
| 215 | Me | 4-MeO | H | H | H | Me | Me | 4-Me |
| 216 | Me | 4-Cl | H | H | H | Me | Me | 4-Me |
| 217 | Me | 4-F | H | H | H | Me | Me | 4-Me |
| 218 | Et | 4-F | H | H | H | Me | Me | 4-Me |
| 219 | Me | 3-$CF_3$ | H | H | H | Me | Me | 4-Me |
| 220 | Me | 4-$CF_3$ | H | H | H | Me | Me | 4-Me |
| 221 | Me | 2-Me | 4-Me | H | H | Me | Me | 4-Me |
| 222 | Me | 2-Me | 4-Me | 6-Me | H | Me | Me | 4-Me |
| 223 | Et | 2-Me | 4-Me | H | H | Me | Me | 4-Me |
| 224 | Me | 2-Me | 6-Me | H | H | Me | Me | 4-Me |
| 225 | Me | 2-Me | 3-Me | H | H | Me | Me | 4-Me |
| 226 | Me | 2-Cl | 6-Cl | H | H | Me | Me | 4-Me |
| 227 | c-Prop | 2-Me | 4-Me | H | H | Me | Me | 4-Me |
| 228 | Et | 2-Br | H | H | H | Me | Me | 4-Me |
| 229 | Me | 2-Me | H | H | H | Et | Et | 4-Me |
| 230 | Me | 2-Me | 4-Me | H | H | Et | Et | 4-Me |
| 231 | Me | H | H | H | H | Et | Et | 4-Me |
| 232 | Me | 2-Cl | H | H | H | Et | Et | 4-Me |
| 233 | Me | 4-F | H | H | H | Et | Et | 4-Me |
| 234 | Me | 4-$CF_3$ | H | H | H | Et | Et | 4-Me |
| 235 | Me | H | H | H | H | H | H | 4-i-Prop |
| 236 | Me | H | H | H | H | Me | Me | 4-i-Prop |
| 237 | Me | 2-Me | 4-Me | H | H | Me | Me | 4-i-Prop |
| 238 | Me | 2-Cl | H | H | H | Me | Me | 4-i-Prop |
| 239 | Et | 2-Me | H | H | H | Me | Me | 4-i-Prop |
| 240 | Me | 2-Me | 6-Me | H | H | Me | Me | 4-i-Prop |
| 241 | Me | 2-Br | H | H | H | Me | Me | 2-Cl |
| 242 | Me | 3-Cl | H | H | H | Me | Me | 2-Cl |
| 243 | Me | 4-Cl | H | H | H | Me | Me | 2-Cl |
| 244 | Me | 3-$CF_3$ | H | H | H | Me | Me | 2-Cl |
| 245 | Me | 2-F | H | H | H | Me | Me | 2-Cl |
| 246 | Me | 2-Me | 4-Me | H | H | Me | Me | 2-Cl |
| 247 | Me | 2-Cl | 4-Me | H | H | Me | Me | 2-Cl |
| 248 | Me | 4-MeO | H | H | H | Me | Me | 2-Cl |

Tabelle 3 (Fortsetzung)

| Lauf-.Nr. | $R^1$ | $R^2$ | $R^3$ | $R^4$ | $R^5$ | $R^6$ | $R^7$ | Y |
|---|---|---|---|---|---|---|---|---|
| 249 | Me | 2-Me | 4-MeO | H | H | Me | Me | 2-Cl |
| 250 | Me | 2-Cl | 4-MeO | H | H | Me | Me | 2-Cl |
| 251 | Me | 3-F | H | H | H | Me | Me | 2-Cl |
| 252 | Me | 4-F | H | H | H | Me | Me | 2-Cl |
| 253 | Me | 4-Br | H | H | H | Me | Me | 2-Cl |
| 254 | Me | H | H | H | H | Et | Et | 2-Cl |
| 255 | Et | H | H | H | H | Et | Et | 2-Cl |
| 256 | c Prop | H | H | H | H | Et | Et | 2-Cl |
| 257 | Prop | H | H | H | H | Et | Et | 2-Cl |
| 258 | Me | 2-Cl | H | H | H | Et | Et | 2-Cl |
| 259 | Me | 4-Cl | H | H | H | Et | Et | 2-Cl |
| 260 | Me | 2-Me | H | H | H | Et | Et | 2-Cl |
| 261 | Me | 2-Me | 4-Me | H | H | Et | Et | 2-Cl |
| 262 | Me | $3-CF_3$ | H | H | H | Et | Et | 2-Cl |
| 263 | Me | 4-OMe | H | H | H | Et | Et | 2-Cl |
| 264 | Me | 2-Br | H | H | H | Et | Et | 2-Cl |
| 265 | Me | H | H | H | H | Me | Prop | 2-Cl |
| 266 | Me | 2-Cl | H | H | H | Me | Prop | 2-Cl |
| 267 | Me | 4-MeO | H | H | H | Me | Prop | 2-Cl |
| 268 | Me | 4-Cl | H | H | H | Me | Prop | 2-Cl |
| 269 | Me | 2-Me | 4-Me | H | H | Me | Prop | 2-Cl |
| 270 | Me | 2-Cl | H | H | H | Me | Me | 3-Cl |
| 271 | Me | 4-MeO | H | H | H | Me | Me | 3-Cl |
| 272 | Me | 4-Cl | H | H | H | Me | Me | 3-Cl |
| 273 | Me | 2-Me | 4-Me | H | H | H | Me | 3-Cl |
| 274 | Me | H | H | H | H | H | Me | 3-Cl |
| 275 | Me | H | H | H | H | Me | Prop | 3-Cl |
| 276 | Me | 2-Cl | H | H | H | Me | Prop | 3-Cl |
| 277 | Me | H | H | H | H | Et | Et | 3-Cl |
| 278 | Et | H | H | H | H | Et | Et | 3-Cl |
| 279 | Me | 2-Cl | | H | H | Et | Et | 3-Cl |
| 280 | Me | 2-Me | 4-Me | H | H | Et | Et | 3-Cl |
| 281 | Me | 4-MeO | H | H | H | Et | Et | 3-Cl |

Die erfindungsgemässen Verbindungen der Formel I sind wertvolle Arzneimittel und zeichnen sich durch eine sehr günstige Wirkung auf die Serumlipoproteine aus. Sie können daher als Arzneimittel insbesonders zur Beeinflussung der Serumlipoproteine verwendet werden. Die Erfindung betrifft daher auch pharmazeutische Präparate auf Basis der Verbindungen der Formel I und ihrer pharmakologisch verträglichen Salze sowie die Verwendung als Arzneimittel.

In der Literatur wird über eine anorektische, ZNS-stimulierende und diuretische Wirkung von 4-Phenyl-2,3-dihydrothiazolin-Derivaten berichtet, wobei es sich um Verbindungen ohne Sulfonamidsubstitution im Phenylteil handelt und die 2-Iminofunktion nicht durch Aryl substituiert ist (vergl. US-PS 3.671.533, DE-OS 19 38 674). Beschrieben sind auch 3-Alkyl-4-phenyl-2-phenylimino-4-thiazoline (vergl. Univ. Kansas Sci. Bull. *24*, 45-49 (1936)), bei denen der in Position 4 befindliche Phenylrest keine Sulfonamidgruppe trägt. Unterschiedlich substituierte 4-(3-Sulfamoyl-phenyl)-3-alkyl-2-imino-4-thiazoline bzw. -thiazolidine werdenebenfalls in der Literatur erwähnt, und zwar insbesondere als Diuretika (vergl. « Diuretic Agents », E.J. Cragoe, Jr., Editor ; ACS-Symposium Series *83*, Seite 24, Washington D.C., 1978).

Es war nun überraschend, dass die erfindungsgemässen Verbindungen der Formel I eine sehr starke und günstige Beeinflussung der Serumlipoproteine zeigen, während die in der oben genannten Literatur beschriebenen Thiazolinderivate keine oder nur geringe in qualitativer und quantitativer Hinsicht deutlich unterlegene Effekte verursachen.

Es ist allgemein anerkannt, dass für die Entstehung arteriosklerotischer Gefässveränderungen, insbesondere der coronaren Herzkrankheit, Hyperlipoproteinämien einen wesentlichen Risikofaktor darstellen. Für die Prophylaxe und die Regression von atherosklerotischen Veränderungen kommt daher der Senkung erhöhter Serum-Lipoproteine eine ausserordentliche Bedeutung zu. Hierbei kommt es aber auf ganz bestimmte Klassen von Serum-Lipoproteinen an, da die low density (LDL) und very low density-Lipoproteine (VLDL) einen atherogenen Risikofaktor darstellen, während die high density-Lipoproteine (HDL) eine Schutzfunktion gegenüber der coronaren Herzkrankheit darstellen. Hypolipidämika sollen demnach VLDL-Cholesterin und LDL-Cholesterin im Serum erniedrigen, dabei aber die HDL-Cholesterin-Konzentration nach Möglichkeit unbeeinflusst lassen oder sogar erhöhen. Die hier angeführten erfindungsgemässen/Verbindungen haben wertvolle therapeutische Eigenschaften. So erniedrigen sie vor allem die Konzentration von LDL und VLDL, während die HDL-Fraktion entweder in wesentlich geringerem Masse erniedrigt, oder sogar erhöht wird. Sie stellen daher einen wesentlichen Fortschritt gegenüber der Vergleichsverbindung Chlofibrat dar, wie aus dem nachfolgend beschriebenen Versuch ersichtlich ist. Sie können daher zur Prophylaxe und Regression von atherosklerotischen Veränderungen herangezogen werden, indem sie einen kausalen Risikofaktor ausschalten. Hierzu zählen nicht nur die primären Hyperlipoproteinämien, sondern auch gewisse sekundäre Hyperlipidamien wie sie z. B. beim Diabetes vorkommen. Das relative Lebergewicht wird durch die Verbindungen I nicht verändert, während das als hypolipidämischer Standard verwendete Clofibrat zu einer starken Erhöhung des relativen Lebergewichts führt.

Die Wirkung der in der nachfolgenden Tabelle angeführten Verbindungen auf die Serum-Lipoproteine wurde an männlichen Wistar-Ratten untersucht, die 7 Tage per Schlundsonde mit den angeführten in Polyäthylenglykol 400 suspendierten Verbindungen behandelt wurden. Ausserdem wurde eine Kontrollgruppe, die nur das Lösungsmittel Polyäthylenglykol 400 erhielt, mitgeführt, sowie bei den meisten Versuchen eine Ratten-Gruppe mit dem Standardhypolipidämikum Clofibrat. Pro Gruppe wurden in der Regel 10 Tiere eingesetzt, denen am Ende der Behandlung das Blut nach leichter Äthernarkose aus dem Orbitalplexus entnommen wurde und das daraus gewonnene Serum zur Trennung der Lipoproteinklassen in der präparativen Ultrazentrifuge nach gängigen Methoden gepoolt wurde. Die Serum-Lipoproteine wurden in der Ultrazentrifuge in folgende Dichteklassen getrennt:

VLDL 1,006 ; LDL 1,006 bis 1,04 ; HDL 1,04 bis 1,21.

Aus den in der Ultrazentrifuge isolierten Lipoprotein-Fraktionen wurde das darin enthaltene Cholesterin vollenzymatisch nach der CHOD-PAP-Methode mittels der Testkombination von Boehringer-Mannheim bestimmt und die Werte in µg/ml Serum umgerechnet. In der angeführten Tabelle ist die Veränderung des Lipoprotein-Cholesterins in der behandelten Gruppe gegenüber einer unter gleichen Bedingungen mitgeführten Kontrollgruppe angegeben. Wie aus der Tabelle ersichtlich, bewirkt Clofibrat eine etwa gleichstarke Senkung der LDL-Fraktion und eine starke Senkung der HDL-Fraktion, während die neuen Verbindungen eine starke selektiv senkende Wirkung auf die atherogenen Lipoproteinfraktionen (VLDL und LDL) ausüben und die schützende HDL-Fraktion im wesentlichen unbeeinflusst lassen oder sogar vermehren.

Tabelle
Änderung der Serumtipoprotein-Spiegel bei Ratten
nach 7-tägiger p.o. Behandlung mit den Verbindungen

| Verbindung gemäß | Dosis mg/kg Tag | % Veränderung des Cholesterins (im Vergleich zur Kontrollgruppe) im Serum | in den Serumlipoprotein-Fractionen VLDL | LDL | HDL |
|---|---|---|---|---|---|
| Beispiel 2 | 100 | − 17 | − 77 | − 56 | − 3 |
| | 30 | − 8 | − 57 | − 43 | − 4 |
| | 10 | − 5 | − 26 | − 38 | − 2 |
| | 3 | + 2 | + 23 | − 39 | + 9 |
| | 1 | 0 | | − 39 | + 13 |
| Beispiel 13 | 30 | − 12 | − 18 | − 32 | 0 |
| | 10 | − 12 | 0 | − 41 | − 13 |
| | 3 | − 14 | − 15 | − 31 | − 3 |
| | 1 | + 7 | − 36 | − 14 | + 13 |

| Verbindung gemäß | Dosis mg/kg Tag | % Veränderung des Cholesterins (im Vergleich zur Kontrollgruppe) im Serum | in den Serumlipoprotein-Fractionen VLDL | LDL | HDL |
|---|---|---|---|---|---|
| Beispiel 19 | 10 | − 8 | − 41 | − 53 | + 3 |
| | 3 | − 11 | − 85 | − 41 | − 2 |
| | 1 | − 8 | − 57 | − 12 | − 2 |
| Beispiel 353 | 3 | − 6 | − 33 | − 61 | + 4 |
| | 1 | + 8 | − 10 | − 30 | + 4 |
| | 0,3 | − 9 | − 14 | − 25 | − 5 |
| | 0,1 | + 7 | − 17 | − 23 | + 2 |
| Beispiel 354 | 30 | − 15 | − 50 | − 60 | − 9 |
| | 3 | − 18 | − 23 | − 66 | − 4 |
| | 1 | + 3 | | − 43 | + 9 |
| Clofibrat | 100 | − 47 | − 30 | − 33 | − 38 |

Als therapeutische Zubereitung der Verbindungen der Formel I kommen vor allem Tabletten, Dragees, Kapseln, Suppositorien und Säfte in Frage. Die neuen Verbindungen können dabei entweder allein oder mit pharmakologisch annehmbaren Trägern vermischt, angewandt werden. Eine orale Anwendungsform wird bevorzugt. Zu diesem Zweck werden die aktiven Verbindungen vorzugsweise mit an sich bekannten Substanzen vermischt und durch an sich bekannte Methoden in geeignete Darreichungsformen gebracht, wie Tabletten, Steckkapseln, wässrige oder ölige Suspensionen oder wässrige oder ölige Lösungen. Als inerte Träger können z. B. Magnesiumkarbonat, Milchzucher oder Maisstärke unter Zusatz anderer Stoffe wie z. B. Magnesiumstearat verwendet werden. Dabei kann die Zubereitung sowohl als Trocken- oder Feuchtgranulat erfolgen. Als ölige Trägerstoffe oder Lösungsmittel kommen besonders pflanzliche und tierische Öle in Betracht wie z. B. Sonnenblumenöl oder Lebertran. Als tägliche Dosis kommen etwa 50 mg bis 5 g in Betracht. Eine Dosierungseinheit enthält vorzugsweise 250 bis 500 mg.

Die Zubereitungen können bei der Behandlung von Lipidstoffwechselstörungen ausser den üblichen Füll- und Trägerstoffen noch ein Antihypertensivum, wie beispielsweise ein Saluretikum, Reserpin, Hydralazin, Guanethidin, α-Methyldopa, Clonidin oder ein β-Sympathikolytikum, oder ein antihyperurikämisch wirksames Mittel, ein orales Antidiabetikum, ein Geriatrikum oder ein Mittel mit durchblutungssteigernder Wirkung enthalten.

Die reinen erfindungsgemässen Vorprodukte der allgemeinen Formel (IV) zeigen im Vergleich zu den erfindungsgemässen Verbindungen der Formel (I) — wenn überhaupt — deutlich schwächere Effekte auf die Serumlipoproteine besitzen aber wie auch strukturverwandte Thiazolidinderivate (vergl. DE-OS 24 36 263) eine zum Teil sehr gute salidiuretische Wirksamkeit.

Die in den nachfolgenden Beispielen aufgeführten Schmelz- und Zersetzungspunkte sind nicht korrigiert.

Beispiel 1

4-(4-Chlor-3-dimethylsulfamoylphenyl)-3-methyl-2-phenyl-imino-4-thiazolin-hydrobromid

a) 6,8 g (0,02 Mol) 2-Brom-4′-chlor-3′-dimethylsulfamoyl-acetophenon und 3,3 g (0,02 Mol) 1-Methyl-3-phenyl-thioharnstoff werden in 100 ml Äthanol im Verlauf von 1 Stunde bis zum Sieden erhitzt. Man versetzt mit 50 ml Eisessig und erhitzt 2 bis 3 weitere Stunden zum Sieden. Nach dem Abdestillieren des Lösungsmittels im Wasserstrahlvakuum versetzt man den Rückstand mit Diisopropyläther, Essigester oder Diäthyläther und filtriert ab. Farblose Kristalle, Schmp. 258-260 °C (Zers.).

b) 10,1 g (0,02 Mol) 4-(4-Chlor-3-dimethylsulfamoyl-phenyl)-3-methyl-2-phenyliminothiazolidin-4-ol-hydrobromid werden in 80 ml Eisessig über eine Dauer von 20 Min. zum Sieden erhitzt. Nach dem Abkühlen vervollständigt man die Kristallisation durch Zugabe von ca. 150 ml Diisopropyläther, rührt eine weitere Stunde bei Raumtemperatur und filtriert ab. Farblose Kristalle, Schmp. 258-260 °C (Zers.).

Beispiel 2

4-(4-Chlor-3-dimethylsulfamoylphenyl)-3-methyl-2-phenyl-imino-4-thiazolin

a) Zu einer Suspension aus 9,8 g (0,02 Mol) 4-(4-Chlor-3-dimethylsulfamoylphenyl)-3-methyl-2-phenylimino-4-thiazolin-hydrobromid in 200 ml Methanol gibt man 10 ml Triäthylamin. Man rührt 3 Stunden bei etwa 20 bis 30 °C und entfernt das Lösungsmittel unter vermindertem Druck. Der Rückstand wird 2 Stunden in 100 ml Wasser gerührt und die Kristalle abfiltriert. Schmp. 179-181 °C.

b) 8,52 g (0,02 Mol) 4-(4-Chlor-3-dimethylsulfamoyl-phenyl)-3-methyl-2-phenyliminothiazolidin-4-ol werden in 100 ml Eisessig 3 Stunden zum Sieden erhitzt, das Lösungsmittel abdestilliert und der Rückstand unter Wasser zur Kristallisation gebracht. Schmp. 180 °C.

Beispiel 3

4-(4-Chlor-3-dimethylsulfamoylphenyl)-3-methyl-2-phenyl-imino-4-thiazolin-hydrochlorid

a) erhält man analog Beispiel 1a) aus 2,4'-Dichlor-3'-dimethylsulfamoyl-acetophenon und 1-Methyl-3-phenylthioharnstoff. Farblose Kristalle, Schmp. 228 °C (Zers.).

b) 8,52 g (0,02 Mol) 4-(4-Chlor-3-dimethylsulfomoyl-phenyl)-3-methyl-2-phenyliminothiazolidin-4-ol werden in 125 ml Methanol mit ätherischer Salzsäurelösung stark sauer gestellt und das Solvens abdestilliert. Der Rückstand wird 1 Stunde in 100 ml Eisessig zum Sieden erhitzt, das Lösungsmittel abdestilliert und der Rückstand unter Essigester zur Kristallisation gebracht. Farblose Kristalle. Schmp. 228-231 °C (Zers.) (aus Äthanol).

c) 8,9 g (0,02 Mol) 4-(4-Chlor-3-dimethylsulfamoylphenyl)-3-methyl-2-phenylimino-4-thiazolin werden in 150 ml Methanol mit gesättigter ätherischer Chlorwasserstofflösung sauer gestellt, das Lösungsmittel abdestilliert und der Rückstand aus Äthanol umkristallisiert. Schmp. 229-233 °C (Zers.).

Beispiel 4

4-(4-Chlor-3-dimethylsulfamoylphenyl)-3-methyl-2-phenyl-imino-4-thiazolin-methansulfonat

erhält man analog der in Beispiel 3c) angegebenen Vorschrift aus 4-(4-Chlor-3-dimethylsulfamoylphenyl)-3-methyl-2-phenylimino-4-thiazolin und 0,02 Mol Methansulfonsäure. Farblose Kristalle, Schmp. 198-199 °C.

Beispiel 5

4-(4-Chlor-3-dimethylsulfamoylphenyl)-3-methyl-2-phenyl-imino-4-thiazolin-p-toluolsulfonat

erhält man analog der in Beispiel 3c) angegebenen Vorschrift aus 4-(4-Chlor-3-dimethylsulfamoylphenyl)-3-methyl-2-phenylimino-4-thiazolin und 0,02 Mol p-Toluol-sulfonsäure. Farblose Kristalle, Schmp. 196 °C.

Beispiel 6

4-(4-Chlor-3-dimethylsulfamoylphenyl)-3-methyl-2-(2-methylphenyl-imino)-4-thiazolin-hydrobromid

erhält man analog der in Beispiel 1b) angegebenen Vorschrift aus 4-(4-Chlor-3-dimethylsulfamoylphenyl)-3-methyl-2-(2-methylphenyl-imino)-thiazolidin-4-ol-hydrobromid und filtriert die sich aus Eisessig schwerlöslich abscheidenden Kristalle bei Raumtemperatur ab. Farblose Kristalle, Schmp. 256 °C.

Beispiel 7

4-(4-Chlor-3-dimethylsulfamoylphenyl)-3-methyl-2-(2-methylphenyl-imino)-4-thiazolin

erhält man analog der in Beispiel 2a) angegebenen Vorschrift aus 4-(4-Chlor-3-dimethylsulfamoylphenyl)-3-methyl-2-(2-methylphenyl-imino)-4-thiazolin-hydrobromid mit Triäthylamin in Methanol. Farblose Kristalle aus Methanol-Essigester, Schmp. 158-162 °C.

Beispiel 8

4-(4-Chlor-3-dimethylsulfamoylphenyl)-2-(4-fluorphenyl-imino)-3-methyl-4-thiazolin-hydrobromid

a) erhält man analog der in Beispiel 1a) angegebenen Vorschrift aus 2-Brom-4'-chlor-3'-dimethylsulfamoylacetophenon und 1-(4-Fluorphenyl)-3-methylthioharnstoff. Farblose Kristalle,

Schmp. 251-253 °C (Zers.) oder

b) erhält man analog der in Beispiel 1b) angegebenen Vorschrift aus 4-(4-Chlor-3-dimethylsulfamoylphenyl)-2-(4-fluorphenyl-imino)-thiazolidin-4-ol-hydrobromid. Farblose Kristalle, Schmp. 252 °C.

Beispiel 9

4-(4-Chlor-3-dimethylsulfamoylphenyl)-2-(4-fluorphenyl-imino)-3-methyl-4-thiazolin

erhält man analog der in Beispiel 2a) angegebenen Vorschrift aus 4-(4-Chlor-3-dimethylsulfamoylphenyl)-2-(4-fluorphenyl-imino)-3-methyl-4-thiazolin-hydrobromid. Farblose bis blaßgelbe Kristalle, Schmp. 144-145 °C.

Beispiel 10

2-(4-Diäthylaminophenyl-imino)-4-(4-chlor-3-dimethylsulfamoylphenyl)-3-methyl-4-thiazolin-hydrobromid

erhält man analog der in Beispiel 1b) angegebenen Vorschrift aus 2-(4-Diäthylaminophenyl-imino)-4-(4-chlor-3-dimethylsulfamoylphenyl)-3-methyl-thiazolidin-4-ol-hydrobromid. Nach dem Erhitzen in Eisessig destilliert man das Lösungsmittel bis auf ein Volumen von 30 ml ab und fällt das gewünschte Produkt mit 150 ml Diisopropyläther. Farblose Kristalle, Schmp. 257 °C (Zers.).

Beispiel 11

2-(4-Diäthylaminophenyl-imino)-4-(4-chlor-3-dimethylsulfamoylphenyl)-3-methyl-4-thiazolin

erhält man analog der in Beispiel 2a) angegebenen Vorschrift aus 2-(4-Diäthylaminophenyl-imino)-4-(4-chlor-3-dimethylsulfamoylphenyl)-3-methyl-4-thiazolin-hydrobromid und Triäthylamin in Methanol bei Raumtemperatur. Schmp. 184-185 °C.

Beispiel 12

4-(4-Chlor-3-dimethylsulfamoylphenyl)-2-(2-chlorphenyl-imino)-3-methyl-4-thiazolin-hydrobromid

erhält man

a) analog der in Beispiel 1a) angegebenen Vorschrift aus 2-Brom-4'-chlor-3'-dimethylsulfamoylacetophenon und 1-(2-Chlorphenyl)-3-methylthioharnstoff. Nach dem Erhitzen unter Rückfluß kühlt man ab, versetzt mit dem dreifachen Volumen Diisopropyläther, rührt 2 Stunden bei Raumtemperatur und filtriert die Kristalle ab. Schmp. 246-248 °C.

b) analog der in Beispiel 1b) angegebenen Vorschrift aus 4-(4-Chlor-3-dimethylsulfamoylphenyl)-2-(2-chlorphenyl-imino)-3-methylthiazolidin-4-ol-hydrobromid. Farblose Kristalle, Schmp. 248 °C.

Beispiel 13

4-(4-Chlor-3-dimethylsulfamoylphenyl)-2-(2-chlorphenyl-imino)-3-methyl-4-thiazolin

a) erhält man analog der in Beispiel 2a) angegebenen Vorschrift aus dem Hydrobromid der Titelverbindung mit Triäthylamin. Farblose Kristalle, Schmp. 152-154 °C (aus Äthanol).

b) erhält man analog Beispiel 2b) durch Kochen von 4-(4-Chlor-3-dimethylsulfamoylphenyl)-2-(2-chlorphenyl-imino)-3-methylthiazolidin-4-ol in Eisessig über 20 Minuten und nachfolgender analoger Aufarbeitung. Schmp. 155-157 °C (aus Äthanol).

c) 10,8 g 4-(4-Chlor-3-dimethylsulfamoylphenyl)-2-(2-chlorphenyl-imino)-3-methylthiazolidin-4-ol-hydrobromid werden in einem Gemisch aus 100 ml Methanol und 10 ml Triäthylamin 30 Min. zum Sieden erhitzt und sodann in das gleiche Volumen Wasser gegossen. Man rührt etwa 2 Stunden bei Raumtemperatur, filtriert die Kristalle ab und kristallisiert aus Äthanol um. Schmp. 153-156 °C.

Beispiel 14

4-(4-Chlor-3-dimethylsulfamoylphenyl)-2-(4-methoxyphenyl-imino)-3-methyl-4-thiazolin-hydrobromid

erhält man analog der in Beispiel 1b) angegebenen Vorschrift aus 4-(4-Chlor-3-dimethylsulfamoylphenyl)-2-(4-methoxyphenyl-imino)-3-methylthiazolidin-4-ol-hydrobromid. Nach dem Abdestillieren des Eisessigs kocht man den Rückstand mehrmals mit Aceton aus und filtriert die Kristalle ab. Schmp. 240-241 °C (Zers.).

Beispiel 15

4-(4-Chlor-3-dimethylsulfamoylphenyl)-2-(4-methoxyphenyl-imino)-3-methyl-4-thiazolin

erhält man analog der in Beispiel 2a) angegebenen Vorschrift aus dem Hydrobromid der Titelverbindung (Beispiel 14) mit Triäthylamin in Äthanol. Farblose Kristalle, Schmp. 198-199 °C.

Beispiel 16

4-(4-Chlor-3-dimethylsulfamoylphenyl)-3-methyl-2-(4-trifluormethylphenyl-imino)-4-thiazolin-hydrobromid

erhält man analog der in Beispiel 1b) angegebenen Vorschrift aus 4-(4-Chlor-3-dimethylsulfamoylphenyl)-3-methyl-2-(4-trifluormethylphenyl-imino)-thiazolidin-4-ol-hydrobromid. Farblose Kristalle, Schmp. 228 °C.

Beispiel 17

4-(4-Chlor-3-dimethylsulfamoylphenyl)-3-methyl-2-(4-trifluormethylphenyl-imino)-4-thiazolin

erhält man analog der in Beispiel 2a) angegebenen Vorschrift aus dem Hydrobromid der Titelverbindung von Beispiel 16 mit Triäthylamin. Schmp. 147-151 °C.

Beispiel 18

4-(4-Chlor-3-dimethylsulfamoylphenyl)-3-methyl-2-(2,4-dimethylphenyl-imino)-4-thiazolin-hydrobromid

a) erhält man analog der in Beispiel 1a) angegebenen Vorschrift aus 2-Brom-4′-chlor-3′-dimethylsulfamoylacetophenon und 1-(2,4-Dimethylphenyl)-3-methylthioharnstoff. Nach dem Vertreiben des Lösungsmittels wird der Rückstand in 100 ml Aceton zum Sieden erhitzt, das Gemisch auf Raumtemperatur gekühlt und die Kristalle abfiltriert. Schmp. 262-264 °C (Zers.).

b) erhält man analog der in Beispiel 1b) angegebenen Vorschrift aus 4-(4-Chlor-3-dimethylsulfamoylphenyl)-3-methyl-2-(2,4-dimethylphenyl-imino)-thiazolidin-4-ol-hydrobromid. Farblose Kristalle, Schmp. 264 °C (aus Eisessig).

Beispiel 19

4-(4-Chlor-3-dimethylsulfamoylphenyl)-3-methyl-2-(2,4-dimethylphenyl-imino)-4-thiazolin

erhält man analog der in Beispiel 2a) angegebenen Vorschrift aus 4-(4-Chlor-3-dimethylsulfamoylphenyl)-3-methyl-2-(2,4-dimethylphenyl-imino)-4-thiazolin-hydrobromid. Farblose Kristalle, Schmp. 152-154 °C.

Beispiel 20

2-(4-Chlor-2-methylphenyl-imino)-4-(4-chlor-3-dimethylsulfamoylphenyl)-3-methyl-4-thiazolin-hydrobromid

erhält man analog der in Beispiel 1b) angegebenen Vorschrift aus 2-(4-Chlor-3-methylphenyl-imino)-4-(4-chlor-3-dimethylsulfamoylphenyl)-3-methylthiazolidin-4-ol-hydrobromid in siedendem Eisessig und anschließender Fällung mit Diäthyläther. Farblose Kristalle aus Eisessig, Schmp. 231 °C (Zers.).

Beispiel 21

2-(4-Chlor-2-methylphenyl-imino)-4-(4-chlor-3-dimethylsulfamoylphenyl)-3-methyl-4-thiazolin

erhält man analog der in Beispiel 2a) angegebenen Vorschrift aus 2-(4-Chlor-3-methylphenyl-imino)-4-(4-chlor-3-dimethylsulfamoylphenyl)-3-methyl-4-thiazolin-hydrobromid und Triäthylamin. Schmp. 137-141 °C.

Beispiel 22

2-(4-Chlor-3-dimethylsulfamoylphenyl)-2-(4-chlorphenyl-imino)-3-methyl-4-thiazolin-hydrobromid

a) erhält man analog der in Beispiel 1a) angegebenen Vorschrift aus 2-Brom-4′-chlor-3′-dimethylsulfamoylacetophenon und (4-chlorphenyl)-3-methylthioharnstoff. Farblose Kristalle, Schmp. 244-246 °C (Zers.).

b) erhält man analog Beispiel 1b) durch 2-stündiges Kochen von 2-(4-Chlor-3-dimethylsulfamoylphe-

nyl)-2-(4-chlorphenyl-imino)-3-methylthiazolidin-4-ol-hydrobromid in Eisessig. Schmp. 246 °C (Zers.).

Beispiel 23

4-(4-Chlor-3-dimethylsulfamoylphenyl)-2-(4-chlorphenyl-imino)-3-methyl-4-thiazolin

erhält man analog der in Beispiel 2a) angegebenen Vorschrift aus dem Hydrobromid der Titelverbindung (Beispiel 22) und Triäthylamin. Farblose Kristalle, Schmp. 184 °C.

Beispiel 24

4-(4-Chlor-3-dimethylsulfamoylphenyl)-3-methyl-2-(2,3-dimethylphenyl-imino)-4-thiazolin-hydrobromid

erhält man analog der in Beispiel 1b) angegebenen Vorschrift aus 4-(4-Chlor-3-dimethylsulfamoylphenyl)-3-methyl-2-(2,3-dimethylphenyl-imino)-thiazolidin-4-ol-hydrobromid durch Kochen über 2 Stunden in Eisessig und Abfiltrieren der Kristalle bei Raumtemperatur. Farblose Kristalle aus Eisessig, Schmp. 256 °C (Zers.).

Beispiel 25

4-(4-Chlor-3-dimethylsulfamoylphenyl)-3-methyl-2-(2,3-dimethylphenyl-imino)-4-thiazolin

erhält man analog der in Beispiel 2a) angegebenen Vorschrift aus 4-(4-Chlor-3-dimethylsulfamoylphenyl)-3-methyl-2-(2,3-dimethylphenyl-imino)-4-thiazolinhydrobromid und Triäthylamin in Methanol. Schmp. 226 °C.

Beispiel 26

2-(3-Chlor-2-methylphenyl-imino)-4-(4-chlor-3-dimethylsulfamoylphenyl)-3-methyl-4-thiazolin-hydrobromid

erhält man analog der in Beispiel 1b) angegebenen Vorschrift aus 2-(3-Chlor-2-methylphenyl-imino)-4-(4-chlor-3-dimethylsulfamoylphenyl)-3-methylthiazolidin-4-ol-hydrobromid durch Erhitzen unter Rückfluß über 20 Min. in Eisessig oder durch Erhitzen über 45 Min. auf 110 °C in Propionsäure. Farblose Kristalle, Schmp. 226-228 °C (Zers.).

Beispiel 27

2-(3-Chlor-2-methylphenyl-imino)-4-(4-chlor-3-dimethylsulfamoylphenyl)-3-methyl-4-thiazolin

erhält man analog der in Beispiel 2a) angegebenen Vorschrift aus 2-(3-Chlor-2-methylphenyl-imino)-4-(4-chlor-3-dimethylsulfamoylphenyl)-3-methyl-4-thiazolinhydrobromid, wobei man anstelle von Triäthylamin mit einer 20 %igen methanolischen Ammoniaklösung alkalisch stellt und entsprechend Beispiel 2a) aufarbeitet. Farblose Kristalle, Schmp. 144-146 °C.

Beispiel 28

2-(4-Chlor-2-methoxyphenyl-imino)-4-(4-chlor-3-dimethylsulfamoylphenyl)-3-methyl-4-thiazolin-hydrobromid

erhält man analog der in Beispiel 1b) angegebenen Vorschrift aus 2-(4-Chlor-2-methoxyphenyl-imino)-4-(4-chlor-3-dimethylsulfamoylphenyl)-3-methylthiazolidin-4-ol-hydrobromid durch Kochen über 30 Min. in Ameisensäure, nachfolgendes Abdestillieren des Solvens, Behandlung des Rückstandes mit Essigester oder Diisopropyläther und Filtration des Feststoffes. Schmp. 244 °C (Zers.).

Beispiel 29

2-(4-Chlor-2-methoxyphenyl-imino)-4-(4-chlor-3-dimethylsulfamoylphenyl)-3-methyl-4-thiazolin

erhält man analog der in Beispiel 2a) angegebenen Vorschrift aus 2-(4-Chlor-2-methoxyphenyl-imino)-4-(4-chlor-3-dimethylsulfamoylphenyl)-3-methyl-4-thiazolin-hydrobromid. Farblose Kristalle, Schmp. 148-150 °C.

Beispiel 30

4-(4-Chlor-3-dimethylsulfamoylphenyl)-3-methyl-2-(3,4-methylendioxyphenyl-imino)-4-thiazolin-hydrobromid

erhält man analog der in Beispiel 1b) angegebenen Vorschrift aus 4-(4-Chlor-3-dimethylsulfamoyl-

phenyl)-3-methyl-2-(3,4-methylendioxyphenyl-imino)-thiazolidin-4-ol-hydrobromid. Farblose Kristalle, Schmp. 230-232 °C (Zers.).

Beispiel 31

4-(4-Chlor-3-dimethylsulfamoylphenyl)-3-methyl-2-(3,4-methylendioxyphenyl-imino)-4-thiazolin

erhält man analog der in Beispiel 27 angegebenen Vorschrift aus 4-(4-Chlor-3-dimethylsulfamoylphenyl)-3-methyl-2-(3,4-methylendioxyphenyl-imino)-4-thiazolin-hydrobromid. Kristalle vom Schmp. 171-173 °C.

Beispiel 32

2-(3,4-Äthylendioxyphenyl-imino)-4-(4-chlor-3-dimethylsulfamoylphenyl)-3-methyl-4-thiazolinhydrobromid

a) erhält man analog der in Beispiel 1a) angegebenen Vorschrift aus 2-Brom-4′-chlor-3′-dimethylsulfamoylacetophenon und 1-(3,4-Äthylendioxyphenyl)-3-methylthioharnstoff. Farblose Kristalle, Schmp. 265-267 °C (Zers.). oder

b) erhält man entsprechend der in Beispiel 1b) angegebenen Vorschrift aus 2-(3,4-Äthylendioxyphenyl-imino)-4-(4-chlor-3-dimethylsulfamoylphenyl)-3-methylthiazolidin-4-ol-hydrobromid. Schmp. 268 °C (Zers.).

Beispiel 33

2-(3,4-Äthylendioxyphenyl-imino)-4-(4-chlor-3-dimethylsulfamoylphenyl)-3-methyl-4-thiazolin

erhält man analog der in Beispiel 2a) angegenbenen Vorschrift aus 2-(3,4-Äthylendioxyphenyl-imino)-4-(chlor-3-dimethylsulfamoylphenyl)-3-methyl-4-thiazolin-hydrobromid. Farblose Kristalle, Schmp. 200-203 °C.

Beispiel 34

4-(4-Chlor-3-dimethylsulfamoylphenyl)-3-methyl-2-(3,4,5-trimethoxyphenyl-imino)-4-thiazolin-hydrobromid

erhält man analog der in Beispiel 1b) angegebenen Vorschrift aus 4-(4-Chlor-3-dimethylsulfamoylphenyl)-3-methyl-2-(3,4,5-trimethoxyphenyl-imino)-thiazolidin-4-ol-hydrobromid durch Kochen über 30 Min. in Eisessig und Fällung mit Diisopropyläther. Schmp. 246 °C.

Beispiel 35

4-(4-Chlor-3-dimethylsulfamoylphenyl)-3-methyl-2-(3,4,5-trimethoxyphenyl-imino)-4-thiazolin

a) erhält man analog der in Beispiel 2a) angegebenen Vorschrift aus 4-(4-Chlor-3-dimethylsulfamoylphenyl)-3-methyl-2-(3,4,5-trimethoxyphenyl-imino)-4-thiazolin-hydrobromid mit Triäthylamin in Methanol oder

b) durch Rühren in einem Gemisch aus 100 ml Essigester/50 ml Toluol und 100 ml wäßriger Natriumbicarbonatlösung bei pH 8 bis 8,5. Man trennt die organische Phase nach 4 Stunden ab, destilliert das Solvens im Wasserstrahlvakuum ab und behandelt den Rückstand für die folgende Filtration der Kristalle mit Diisopropyläther oder Wasser. Schmp. 119-122 °C.

Beispiel 36

4-(4-Chlor-3-dimethylsulfamoylphenyl)-2-(2,4-dichlor-5-methylphenyl-imino)-3-methyl-4-thiazolin-hydrobromid

erhält man durch Umsetzung von 0,02 Mol. 2-Brom-4′-chlor-3′-dimethylsulfamoylacetophenon mit 0,02 Mol. 1-(2,4-Dichlor-5-methyl)-3-methylthioharnstoff in 140 ml Aceton. Man rührt über 16 Stunden bei Raumtemperatur, erhitzt 6 Stunden am aufgesetzten Rückflußkühler zum Sieden und filtriert die abgeschiedenen Kristalle nach Stehenlassen über Nacht bei Raumtemperatur ab. Schmp. 242 °C (Zers.).

Beispiel 37

3-Äthyl-4-(4-chlor-3-dimethylsulfamoylphenyl)-2-(2-methylphenyl-imino)-4-thiazolin-hydrobromid

a) erhält man analog der in Beispiel 1a) angegebenen Vorschrift aus 2-Brom-4′-chlor-3′-di-

methylsulfamoylacetophenon und 3-Äthyl-1-(2-methylphenyl)-thioharnstoff. Farblose Kristalle, Schmp. 280-282 °C (Zers.).

b) erhält man analog der in Beispiel 1b) angegebenen Vorschrift aus 3-Äthyl-4-(4-chlor-3-dimethylsulfamoylphenyl)-2-(2-methylphenyl-imino)-thiazolidin-4-ol-hydrobromid. Schmp. 281-282 °C (Zers.).

Beispiel 38

3-Äthyl-4-(4-chlor-3-dimethylsulfamoylphenyl)-2-(2-methylphenyl-imino)-4-thiazolin

erhält man analog der in Beispiel 2a) angegebenen Vorschrift aus 3-Äthyl-4-(4-chlor-3-dimethylsulfamoylphenyl)-2-(2-methylphenyl-imino)-4-thiazolin-hydrobromid. Farblose Kristalle. Schmp. 164-166 °C.

Beispiel 39

4-(4-Chlor-3-dimethylsulfamoylphenyl)-2-phenylimino-3-propyl-4-thiazolin-hydrobromid

erhält man analog der in Beispiel 1b) angegebenen Vorschrift aus 4-(4-Chlor-3-dimethylsulfamoylphenyl)-2-phenylimino-3-propylthiazolidin-4-ol-hydrobromid. Farblose Kristalle, Schmp. 225 °C (Zers.).

Beispiel 40

4-(4-Chlor-3-dimethylsulfamoylphenyl)-3-cyclopropyl-2-phenylimino-4-thiazolin-hydrobromid

a) erhält man analog der in Beispiel 1a) angegebenen Vorschrift aus 1-Cyclopropyl-3-phenylthioharnstoff und 2-Brom-4'-chlor-3'-dimethylsulfamoylacetophenon durch Rühren über 48 Stunden bei Raumtemperatur in 200 ml Äthanol und nachfolgendem Kochen am aufgesetztem Rückflußkühler für 2 Stunden, Abdestillieren des Solvens und Filtration der Kristalle nach Aufschlämmen in Diisopropyläther oder Essigester. Farblose Kristalle, Schmp. 260-262 °C (Zers.).

b) erhält man analog Beispiel 1b) aus 4-(4-Chlor-3-dimethylsulfamoylphenyl)-3-cyclopropyl-2-phenyliminothiazolidin-4-ol-hydrobromid. Farblose Kristalle, Schmp. 259-264 °C (Zers.).

Beispiel 41

4-(4-Chlor-3-dimethylsulfamoylphenyl)-3-cyclopropyl-2-phenylimino-4-thiazolin

erhält man analog der in Beispiel 2a) angegebenen Vorschrift aus 4-(4-Chlor-3-dimethylsulfamoylphenyl)-3-cyclopropyl-2-phenylimino-4-thiazolin-hydrobromid. Schmp. 156-159 °C.

Beispiel 42

3-sek. Butyl-4-(4-chlor-3-dimethylsulfamoylphenyl)-2-phenylimino-4-thiazolin-hydrobromid

erhält man analog der in Beispiel 1a) angegebenen Vorschrift aus 2-Brom-4'-chlor-3'-dimethylsulfamoylacetophenon und 3-sek. Butyl-1-phenylthioharnstoff oder analog Beispiel 1b) aus 3-sek. Butyl-4-(4-chlor-3-dimethylsulfamoylphenyl)-2-phenyliminothiazolidin-4-ol-hydrobromid. Farblose Kristalle, Schmp. 250 °C (Zers.).

Beispiel 43

3-sek. Butyl-4-(4-chlor-3-dimethylsulfamoylphenyl)-2-phenylimino-4-thiazolin

erhält man analog der in Beispiel 2a) oder 35b) angegebenen Vorschrift aus 3-sek. Butyl-4-(4-chlor-3-dimethylsulfamoyl)-2-phenylimino-4-thiazolin-hydrobromid. Farblose Kristalle, Schmp. 138 °C.

Beispiel 44

4-(4-Chlor-3-dimethylsulfamoylphenyl)-3-n-hexyl-2-phenylimino-4-thiazolin-hydrobromid

erhält man a) analog der in Beispiel 1a) angegebenen Vorschrift aus 2-Brom-4'-chlor-3'-dimethylsulfamoylacetonphenon und 3-n-Hexyl-1-phenylthioharnstoff oder b) analog der in Beispiel 1b) angegebenen Vorschrift aus 4-(4-Chlor-3-dimethylsulfamoylphenyl)-3-n-hexyl-2-phenyliminothiazolidin-4-ol-hydrobromid. Farblose Kristalle, Schmp. 234 °C (Zers.).

Beispiel 45

4-(4-Chlor-3-dimethylsulfamoylphenyl)-3-n-hexyl-2-phenylimino-4-thiazolin

erhält man analog der in Beispiel 2a) angegebenen Vorschrift aus 4-(4-Chlor-3-dimethylsulfamoylphenyl)-3-n-hexyl-2-phenylimino-4-thiazolin-hydrobromid. Schmp. 86 °C.

Beispiel 46

4-(4-Chlor-3-dimethylsulfamoylphenyl-3-cyclohexyl-2-phenylimino-4-thiazolin-hydrobromid

erhält man analog der in Beispiel 1b) angegebenen Vorschrift aus 4-(4-Chlor-3-dimethylsulfamoylphenyl)-3-cyclohexyl-2-phenyliminothiazolidin-4-ol-hydrobromid. Farblose Kristalle, Schmp. 236 °C.

Beispiel 47

4-(4-Chlor-3-dimethylsulfamoylphenyl)-3-cyclohexyl-2-phenylimino-4-thiazolin.

erhält man analog der in Beispiel 2a) oder 35b) angegebenen Vorschrift aus 4-(4-Chlor-3-dimethylsulfamoylphenyl)-3-cyclohexyl-2-phenylimino-4-thiazolinhydrobromid. Farblose Kristalle, Schmp. 148 °C.

Beispiel 48

3-n-Butyl-4-(4-chlor-3-dimethylsulfamoylphenyl)-2-(2-methylphenyl-imino)-4-thiazolin-hydrobromid

erhält man a) analog der in Beispiel 1a) angegebenen Vorschrift aus 3-n-Butyl-1-phenylthioharnstoff und 2-Brom-4'-chlor-3'-dimethylsulfamoylacetophenon oder b) aus 3-n-Butyl-4-(4-chlor-3-dimethylsulfamoylphenyl)-2-(2-merthylphenyl-imino)-thiazolidin-4-ol-hydrobromid- Farblose Kristalle, Schmp. 238 °C.

Beispiel 49

3-n-Butyl-4-(4-chlor-3-dimethylsulfamoylphenyl)-2-(2-methylphenyl-imino)-4-thiazolin

erhält man analog der in Beispiel 2a) angegebenen Vorschrift aus 3-n-Butyl-4-(4-chlor-3-dimethylsulfamoylphenyl)-2-(2-methylphenyl-imino)-4-thiazolin-hydrobromid.

Beispiel 50

4-(3-Diäthylsulfamoyl-4-chlorphenyl)-3-methyl-2-phenyl-imino-4-thiazolin-hydrochlorid

erhält man durch Kochen einer Suspension von 0,02 Mol 4-(3-Diäthylsulfamoyl-4-chlorphenyl)-3-methyl-2-phenyl-iminothiazolidin-4-ol für 2 Stunden in 120 ml Äthanol nach dem Sauerstellen mit ätherischer HCl-Lösung. Das Lösungsmittel wird abdestilliert und der Rückstand unter Diisopropyläther kristallisiert. Farblose Kristalle, Schmp. 222 °C (aus Äthanol/Äther).

Beispiel 51

4-(3-Diäthylsulfamoyl-4-chlorphenyl)-2-(4-chlorphenyl-imino)-3-methyl-4-thiazolin-hydrobromid

erhält man a) analog der in Beispiel 1a) angegebenen Vorschrift aus 3'-Diäthylsulfamoyl-2-brom-4'-chloracetophenon und 1-(4-Chlorphenyl)-3-methylthioharnstoff oder b) analog Beispiel 1b) durch Kochen von 4-(3-Diäthylsulfamoyl-4-chlorphenyl)-2-(4-chlorphenyl-imino)-3-methylthiazolidin-4-ol-hydrobromid über 2 Stunden in Eisessig und Filtration der Kristalle nach dem Abkühlen. Schmp. 207 °C (Zers.).

Beispiel 52

4-(3-Diäthylsulfamoyl-4-chlorphenyl)-2-(4-chlorphenyl-imino)-3-methyl-4-thiazolin

erhält man analog der in Beispiel 2a) angegebenen Vorschrift aus dem entsprechenden Hydrobromid (Beispiel 51). Farblose Kristalle, Schmp. 198 °C.

Beispiel 53

4-(3-Diäthylsulfamoyl-4-chlorphenyl)-3-methyl-2-(2-methylphenyl-imino)-4-thiazolin-hydrobromid

erhält man analog der in Beispiel 51b) angegebenen Vorschrift aus 4-(3-Diäthylsulfamoyl-4-chlorphenyl)-3-methyl-2-(2-methylphenyl-imino)-thiazolidin-4-ol-hydrobromid. Farblose Kristalle, Schmp. 258 °C (Zers.).

Beispiel 54

4-(3-Diäthylsulfamoyl-4-chlorphenyl)-3-methyl-2-(2-methylphenyl-imino)-4-thiazolin

erhält man analog der in Beispiel 2a) angegebenen Vorschrift aus dem entsprechenden Hydrobromid (Beispiel 53). Farblose Kristalle, Schmp. 166 °C.

Beispiel 55

4-(3-N-Butyl-N-methylsulfamoyl-4-chlorphenyl)-3-methyl-2-phenylimino-4-thiazolin

erhält man analog der in Beispiel 2b) angegebenen Vorschrift aus 4-(3-N-Butyl-N-methylsulfamoyl-4-chlorphenyl)-3-methyl-2-phenyliminothiazolidin-4-ol. Farblose Kristalle, Schmp. 98-100 °C.

Beispiel 56

4-(3-N-Butyl-N-methylsulfamoyl-4-chlorphenyl)-3-methyl-2-phenylimino-4-thiazolin-hydrochlorid

erhält man analog der in Beispiel 3c) angegebenen Vorschrift aus dem Thiazolin von Beispiel 55. Farbloser Feststoff, Schmp. 84-87 °C (Zers.).

Beispiel 57

4-(4-Chlor-3-dipropylsulfamoylphenyl)-3-methyl-2-(2-methylphenyl-imino)-4-thiazolin-hydrobromid

erhält man analog der in Beispiel 1b) angegebenen Vorschrift aus 4-(4-Chlor-3-dipropylsulfamoylphenyl)-3-methyl-2-(2-methylphenyl)-thiazolidin-4-ol-hydrobromid durch 2 stündiges Kochen in Eisessig. Man engt ein, versetzt den Rückstand mit Wasser und filtriert die Kristalle ab. Schmp. 218-220 °C.

Beispiel 58

4-(4-Chlor-3-dipropylsulfamoylphenyl)-3-methyl-2-(2-methylphenyl-imino)-4-thiazolin

erhält man analog der in Beispiel 2a) angegebenen Vorschrift aus dem entsprechendem Hydrobromid (Beispiel 57) und Triäthylamin. Farblose Kristalle, Schmp. 114-116 °C.

Beispiel 59

4-(4-Chlor-3-dipropylsulfamoylphenyl)-3-methyl-2-(2,4-dimethylphenyl-imino)-4-thiazolin-hydrobromid

erhält man analog der in Beispiel 51b) angegebenen Vorschrift aus 4-(4-Chlor-3-dipropylsulfamoylphenyl)-3-methyl-2-(2,4-dimethylphenyl)-thiazolidin-4-ol-hydrobromid. Farblose Kristalle, Schmp. 239-241 °C (Zers.).

Beispiel 60

4-(4-Chlor-3-dipropylsulfamoylphenyl)-3-methyl-2-(2,4-dimethylphenyl-imino)-4-thiazolin

erhält man analog der in Beispiel 2a) angegebenen Vorschrift aus dem entsprechenden Hydrobromid (Beispiel 59). Farblose Kristalle, Schmp. 139-141 °C.

Beispiel 61

4-(4-Chlor-3-dipropylsulfamoylphenyl)-3-methyl-2(2,3-dimethylphenyl-imino)-4-thiazolin-hydrobromid

erhält man analog der in Beispiel 1b) angegebenen Vorschrift aus 4-(4-Chlor-3-dipropylsulfamoylphenyl)-3-methyl-2-(2,3-dimethylphenyl-imino)-thiazolidin-4-ol-hydrobromid durch Rückfluß in Eisessig, nachfolgendem Eindampfen und Kristallisation des viskosen Rückstandes unter Wasser. Farblose Kristalle aus Methanol-Äther, Schmp. 210-212 °C.

Beispiel 62

4-(4-Chlor-3-dipropylsulfamoylphenyl)-3-methyl-2-(2,3-dimethylphenyl-imino)-4-thiazolin

erhält man analog der in Beispiel 2a) angegebenen Vorschrift aus dem Hydrobromid der Titelverbindung (Beispiel 61). Farblose Kristalle, Schmp. 184-187 °C.

Beispiel 63

2-(5-Chlor-2,4-dimethoxyphenyl-imino)-4-(4-chlor-3-dipropylsulfamoylphenyl)-3-methyl-4-thiazolin-hydrobromid

erhält man analog der in Beispiel 1b) angegebenen Vorschrift aus 2-(5-Chlor-2,4-dimethoxyphenylimino)-4-(4-chlor-3-dipropylsulfamoylphenyl)-3-methylthiazolidin-4-ol-hydrobromid durch Kochen in Eisessig und Rührung des Rückstandes unter Wasser nach Destillation des Solvens. Amorpher glasiger Feststoff, Schmp. 130-150 °C.

Beispiel 64

2-(5-Chlor-2,4-dimethoxyphenyl-imino)-4-(4-chlor-3-dipropylsulfamoylphenyl)-3-methyl-4-thiazolin

erhält man analog der in Beispiel 2a) angegebenen Vorschrift aus dem entsprechenden Hydrobromid. Farblose Kristalle, Schmp. 173-175 °C.

Beispiel 65

4-(4-Chlor-3-N-morpholinosulfamoylphenyl)-3-methyl-2-phenylimino-4-thiazolin

erhält man analog der in Beispiel 2b) angegebenen Vorschrift aus 4-(4-Chlor-3-N-morpholinosulfonylphenyl)-3-methyl-2-phenylimino-thiazolidin-4-ol durch Kochen in Eisessig und Ausfällung mit Diisopropyläther. Schmp. 212-214 °C.

Beispiel 66

4-[4-Chlor-3-(1-methyl-4-piperazinylsulfonyl)-phenyl]-3-methyl-2-phenylimino-4-thiazolin

erhält man analog der in Beispiel 2b) angegebenen Vorschrift aus 4-[4-Chlor-3-(1-methyl-4-piperazinylsulfonyl)-phenyl]-3-methyl-2-phenyliminothiazolidin-4-ol durch Kochen mit Eisessig und nachfolgendem Abdestillieren des Solvens. Der Rückstand wird mit Wasser versetzt und mit 2 N NaOH auf pH 13 gestellt. Man filtriert die Kristalle ab und kristallisiert aus Isopropanol um. Schmp. 156-158 °C.

Beispiel 67

4-[4-Chlor-3-(3,5-dimethylmorpholino-N-sulfonyl)-phenyl]-3-methyl-2-phenylimino-4-thiazolin

erhält man analog der in Beispiel 2b) angegebenen Vorschrift aus 4-[4-Chlor-3-(3,5-dimethylmorpholino-N-sulfonyl)-phenyl]-3-methyl-2-phenylimino-4-thiazolin. Farblose Kristalle, Schmp. 190-192 °C (aus Äthanol).

Analog der in Beispiel 2b) beschriebenen Vorschrift erhält man aus den entsprechend substituierten Thiazolidin-4-ol-Derivaten IV die in den folgenden Beispielen aufgeführten Thiazoline der Formel (I) :

Beispiel 68

4-(4-Chlor-3-cyclopropylsulfamoylphenyl)-3-methyl-2-phenylimino-4-thiazolin, Schmp. 176 °C.

Beispiel 69

4-(4-Chlor-3-cyclohexylsulfamoylphenyl)-3-methyl-2-phenylimino-4-thiazolin, Zers. p. 143 °C.

Beispiel 70

4-[4-Chlor-3-(4-methylbenzylsulfamoyl)-phenyl]-3-methyl-2-phenylimino-4-thiazolin, Schmp. 164-169 °C.

Beispiel 71

4-(4-Chlor-3-n-propylsulfamoylphenyl)-3-methyl-2-phenylimino-4-thiazolin, Schmp. 157-160 °C.

Beispiel 72

4-[4-Chlor-3-(4-chlorbenzylsulfamoyl)-phenyl]-3-methyl-2-phenylimino-4-thiazolin, Schmp. 246-247 °C.

Beispiel 73

4-(4-Chlor-3-sulfamoylphenyl)-3-methyl-2-phenylimino-4-thiazolin, Schmp. 170-173 °C.

Analog der in Beispiel 1b) beschriebenen Vorschrift erhält man aus den entsprechend substituierten Thiazolidin-4-ol-Derivaten (IV) die in den folgenden Beispielen aufgeführten Thiazolin-Derivate der Formel (I) :

Beispiel 74

4-(4-Chlor-3-sulfamoylphenyl)-3,5-dimethyl-2-phenylimino-4-thiazolin-hydrobromid, Zers. p. 219 °C.

Beispiel 75

4-(4-Brom-3-sulfamoylphenyl)-2-(4-methoxyphenyl-imino)-3-methyl-4-thiazolin-hydrobromid, Schmp. 273 °C.

Beispiel 76

4-(4-Chlor-3-sulfamoylphenyl)-2-(3,4,5-trimethoxyphenyl-imino)-3-methyl-4-thiazolin-hydrobromid, Schmp. 294 °C (unter Zers.).

Beispiel 77

2-(3,4-Äthylendioxyphenyl-imino)-4-(4-chlor-3-sulfamoyl-phenyl)-3-methyl-4-thiazolin-hydrobromid, Schmp. 283 °C (unter Zers.).

Beispiel 78

4-(4-Chlor-3-sulfamoylphenyl)-2-(3,4-methylendioxyphenyl-imino)-3-methyl-4-thiazolin-hydrobromid, Schmp. 275 °C (unter Zers.).

Beispiel 79

4-(4-Chlor-3-sulfamoylphenyl)-2-(4-methoxyphenyl-imino)-3-methyl-4-thiazolin-hydrobromid, Schmp. 277-280 °C (unter Zers.).

Beispiel 80

3-Äthyl-4-(4-chlor-3-sulfamoylphenyl)-2-phenylimino-4-thiazolin-hydrochlorid, Schmp. 241 °C (Zers.).

Beispiel 81

4-(4-Chlor-3-sulfamoylphenyl)-2-(4-isopropylphenyl-imino)-3-methyl-4-thiazolin-hydrochlorid, Schmp. 276-278 °C (Zers.).

Beispiel 82

4-(4-Chlor-3-sulfamoylphenyl)-3-methyl-2-(2,3-dimethylphenyl-imino)-4-thiazolin-hydrochlorid, Schmp. 240 °C (Zers.).

Beispiel 83

2-(2-Chlorphenyl-imino)-4-(4-chlor-3-sulfamoylphenyl)-3-methyl-4-thiazolin-hydrochlorid, Schmp. 245-247 °C.

Beispiel 84

2-(4-Chlor-2-methoxyphenyl-imino)-4-(4-chlor-3-sulfamoylphenyl)-3-methyl-4-thiazolin-hydrochlorid, Schmp. 234-237 °C (Zers.).

Beispiel 85

2-(5-Chlor-2,4-dimethoxyphenyl-imino)-4-(4-chlor-3-sulfamoylphenyl)-3-methyl-4-thiazolin-hydrobromid, Schmp. 278-279 °C (Zers.).

Beispiel 86

4-(4-Chlor-3-sulfamoylphenyl)-3-methyl-2-(3-dimethylaminophenyl-imino)-4-thiazolin-hydrobromid, Schmp. 258-260 (Zers.).

Beispiel 87

4-(4-Chlor-3-sulfamoylphenyl)-3-methyl-2-(2,4-dimethylphenyl-imino)-4-thiazolin-hydrobromid, Schmp. 255-258 °C (Zers.).

Beispiel 88

2-(2-Äthoxy-5-methylphenylimino)-4-(4-chor-3-sulfamoylphenyl)-3-methyl-4-thiazolin-hydrobromid, Schmp. 227-230 °C.

Beispiel 89

4-(4-Chlor-3-sulfamoylphenyl)-2-(2-methoxy-4,5-dimethylphenyl-imino)-3-methyl-4-thiazolin-hydrobromid, Schmp. 257-260 °C.

Beispiel 90

4-(4-Chlor-3-sulfamoylphenyl)-3-methyl-2-(3-trifluormethylphenyl-imino)-4-thiazolin-hydrobromid, Schmp. 217 °C (Zers.).

Beispiel 91

4-(4-Chlor-3-sulfamoylphenyl)-2-(4-fluorphenyl-imino)-3-methyl-4-thiazolin-hydrobromid.

Beispiel 92

3-Äthyl-4-(4-chlor-3-sulfamoylphenyl)-2-(4-methylphenyl-imino)-4-thiazolin-hydrobromid, Schmp. 268 °C (Zers.).

Beispiel 93

2-(4-Äthoxyphenyl-imino)-4-(4-chlor-3-sulfamoylphenyl)-3-methyl-4-thiazolin-hydrobromid, Schmp. 263 °C (Zers.).

Analog der in Beispiel 3 b) und 50 angegebenen Vorschrift erhält man aus den entsprechend substituierten Thiazolidin-4-ol-Derivaten der allgemeinen Formel IV die in den folgenden Beispielen aufgeführten Thiazolin-Derivate der Formel I:

Beispiel 94

4-(3-n-Butylsulfamoyl-4-chlorphenyl)-3-methyl-2-phenyl-imino-4-thiazolin-hydrochlorid, Schmp. 237 °C.

Beispiel 95

4-(3-Benzylsulfamoyl-4-chlorphenyl)-3-methyl-2-phenyl-imino-4-thiazolin-hydrochlorid, Zers. p. 152 °C.

Beispiel 96

4-(3-N-Benzyl-N-methylsulfamoyl-4-chlorphenyl)-3-methyl-2-phenylimino-4-thiazolin-hydrochlorid, Zers. p. 165 °C (aus Äthanol).

Beispiel 97

4-[4-Chlor-3-(2,4-dimethoxybenzylsulfamoyl)-phenyl]-3-methyl-2-phenylimino-4-thiazolin-hydrochlorid, Zers. p. 158 °C.

Beispiel 98

4-[3-(2-Chlorbenzylsulfamoyl)-4-chlorphenyl]-3-methyl-2-phenylimino-4-thiazolin-hydrochlorid, Schmp. 264 °C.

Beispiel 99

4-(4-Chlor-3-cyclopentylsulfamoyl-phenyl)-3-methyl-2-phenyl-imino-4-thiazolin-hydrochlorid, Schmp. 257 °C (Zers.).

Beispiel 100

4-(3-Äthylsulfamoyl-4-chlorphenyl)-3-methyl-2-phenyl-imino-4-thiazolin-hydrochlorid, Schmp. 240-241 °C (Zers.).

Beispiel 101

4-[4-Chlor-3-(4-methoxybenzylsulfamoyl)-phenyl]-3-methyl-2-phenylimino-4-thiazolin-hydrochlorid, Zers. p. 133-137 °C.

Beispiel 102

4-[4-Chlor-3-(3,5-dimethyl-1-piperidylsulfonyl)-phenyl]-3-methyl-2-phenylimino-4-thiazolin-hydrochlorid, Schmp. 198 °C.

Beispiel 103

4-(4-Chlor-3-sulfamoylphenyl)-3-methyl-2-(2-methylphenyl-imino)-4-thiazolin-hydrochlorid, Schmp. 278 °C (Zers.).

Beispiel 104

4-(4-Chlor-3-sulfamoylphenyl)-3-methyl-2-(4-methylphenyl-imino)-4-thiazolin-hydrobromid

0,02 Mol (9,8 g) 4-(4-Chlor-3-sulfamoylphenyl)-3-methyl-2-(4-methylphenyl-imino)-thiazolidin-4-ol-hydrobromid werden in 120 ml Äthanol für 2 Stunden unter Rückfluß gekocht. Nach Abkühlen auf Raumtemperatur versetzt man mit 200 ml Diisopropyläther und filtriert die Kristalle ab. Schmp. 265 °C (Zers.)

Analog der in Beispiel 104 angegebenen Vorschrift erhält man aus den entsprechend substituierten Thiazolidin-4-ol-Derivaten der allgemeinen Formel IV die in den folgenden Beispielen aufgeführten Thiazolin-Derivate der Formel I :

Beispiel 105

2-(2-Äthylphenyl-imino)-4-(4-chlor-3-sulfamoylphenyl)-3-methyl-4-thiazolin-hydrochlorid, Zers. p. 176 °C.

Beispiel 106

2-(2-Äthylphenyl-imino)-4-(4-chlor-3-sulfamoylphenyl)-3-methyl-4-thiazolin-hydrobromid, Zers. p. 178 °C.

Beispiel 107

4-(4-Chlor-3-methylsulfamoylphenyl)-3-methyl-2-(2,3-dimethylphenyl-imino)-4-thiazolin-hydrobromid, Schmp. 270 (unter Zers.).

Beispiel 108

4-[4-Chlor-3-(1-piperidinylsulfonyl)-phenyl]-3-methyl-2-phenylimino-4-thiazolin-hydrochlorid, Schmp. 187-191 °C.

Beispiel 109

4-[4-Chlor-3-(1-pyrrolidinylsulfonyl)-phenyl]-3-methyl-2-phenylimino-4-thiazolin-hydrochlorid, Schmp. 162 °C (Zers.).

Beispiel 110

4-[4-Chlor-3-(1-n-dodecylsulfamoyl)-phenyl]-3-methyl-2-phenylimino-4-thiazolin-hydrochlorid, Schmp. 128 °C (Zers.).

Beispiel 111

4-(4-Chlor-3-sulfamoylphenyl)-2-phenylimino-3-propyl-4-thiazolin-hydrobromid, Schmp. 198 °C (Zers.).

Beispiel 112

3-Allyl-4-(4-chlor-3-sulfamoylphenyl)-2-phenylimino-4-thiazolin-hydrobromid, Schmp. 248-252 °C (Zers.).

Beispiel 113

3-sek. Butyl-4-(4-chlor-3-sulfamoylphenyl)-2-phenylimino-4-thiazolin-hydrobromid, Schmp. 265-268 °C (Zers.).

Beispiel 114

4-[4-Chlor-3-(1-n-hexylsulfamoyl)-phenyl]-3-methyl-2-phenylimino-4-thiazolin-hydrochlorid, Schmp. 117-182 °C (Zers.).

Beispiel 115

2-(4-Diäthylaminophenyl-imino)-4-(4-chlor-3-sulfamoylphenyl)-3-methyl-4-thiazolin-hydrochlorid, Zers. ab 180 °C.

Aus den Säureadditionssalzen der Verbindungen der Formel I lassen sich durch Einwirkung einer Base analog der in den Beispielen 2 a, 27 und 35 b) angegebenen Vorschriften die in den folgenden Beispielen aufgeführten basischen Verbindungen der Formel I erhalten :

Beispiel 116

4-(4-Chlor-3-sulfamoylphenyl)-3,5-dimethyl-2-phenyl-imino-4-thiazolin, Zers. ab 117 °C.

Beispiel 117

4-(4-Brom-3-sulfamoylphenyl)-2-(4-methoxyphenyl-imino)-3-methyl-4-thiazolin, Schmp. 197 °C (aus Alkohol).

Beispiel 118

2-(2-Äthylphenyl-imino)-4-(4-chlor-3-sulfamoylphenyl)-3-methyl-4-thiazolin, Schmp. 161-163 °C.

Beispiel 119

4-(4-Chlor-3-sulfamoylphenyl)-3-methyl-2-(4-methylphenyl-imino)-4-thiazolin, Schmp. 267 °C.

Beispiel 120

4-[4-Chlor-3-(1-piperidylsulfonyl)-phenyl]-3-methyl-2-phenylimino-4-thiazolin, Schmp. 189-195 °C.

Beispiel 121

4-[4-Chlor-3-(1-pyrrolidinylsulfonyl)-phenyl]-3-methyl-2-phenylimino-4-thiazolin, Schmp. 191-194 °C.

Beispiel 122

4-(4-Chlor-3-sulfamoylphenyl)-2-phenylimino-3-propyl-4-thiazolin, Schmp. 165-170 °C.

Beispiel 123

3-sek. Butyl-4-(4-chlor-3-sulfamoylphenyl)-2-phenylimino-4-thiazolin, Schmp. 80 °C.

Beispiel 124

4-[3-(1-Butylsulfamoyl)-4-chlorphenyl]-3-methyl-2-phenylimino-4-thiazolin, Schmp. 130-135 °C.

Beispiel 125

4-(3-Diäthylsulfamoyl-4-chlorphenyl)-3-methyl-2-phenyl-imino-4-thiazolin, Schmp. 173-175 °C.

Beispiel 126

4-(4-Chlor-3-sulfamoylphenyl)-3-methyl-2-(3,4,5-trimethoxy-phenyl-imino)-4-thiazolin, Schmp. 187-189 °C.

Beispiel 127

2-(3,4-Äthylendioxyphenyl-imino)-4-(4-chlor-3-sulfamoylphenyl)-3-methyl-4-thiazolin, Schmp. 247-249 °C.

Beispiel 128

4-(4-Chlor-3-sulfamoylphenyl)-2-(3,4-methylendioxyphenyl-imino)-3-methyl-4-thiazolin, Schmp. 187-189 °C.

Beispiel 129

4-(4-Chlor-3-sulfamoylphenyl)-2-(4-methoxyphenyl-imino)-3-methyl-4-thiazolin, Schmp. 210-214 °C.

Beispiel 130

4-(4-Chlor-3-sulfamoylphenyl)-2-(4-fluorphenylimino)-3-methyl-4-thiazolin, Schmp. 234-236 °C.

Beispiel 131

2-(4-Äthoxyphenyl-imino)-3-methyl-4-(4-chlor-3-sulfamoylphenyl)-4-thiazolin, Schmp. 233 °C.

Beispiel 132

4-(4-Chlor-3-sulfamoylphenyl)-3-methyl-2-(3-methylphenyl-imino)-4-thiazolin, Schmp. 193-194° (aus Methanol).

Beispiel 133

2-(5-Chlor-2,4-dimethoxyphenyl-imino)-4-(4-chlor-3-sulfamoylphenyl)-3-methyl-4-thiazolin, Schmp. 204-206 °C.

Beispiel 134

4-(4-Chlor-3-sulfamoylphenyl)-3-methyl-2-(3-dimethylaminophenyl-imino)-4-thiazolin, Schmp. 134-140 °C.

Beispiel 135

4-(4-Chlor-3-sulfamoylphenyl)-3-methyl-2-(2,4-dimethylphenyl-imino)-4-thiazolin, Schmp. 270-275 °C.

Beispiel 136

2-(2-Äthoxy-5-methylphenyl-imino)-4-(4-chlor-3-sulfamoylphenyl)-3-methyl-4-thiazolin, Schmp. 194-197 °C.

Beispiel 137

4-(4-Chlor-3-chlorsulfonylphenyl)-3-methyl-2-phenyl-iminothiazolidin-4-ol-hydrobromid.

Zu einer Lösung aus 6,64 g (0,02 Mol) 2-Brom-4′-chlor-3′-chlorsulfonylacetophenon in 40 ml Aceton gibt man unter Rührung eine Lösung aus 3-Methyl-1-phenylthioharnstoff, wobei es nach mäßiger Eigenerwärmung zur Abscheidung der kristallinen Titelverbindung kommt. Man rührt 4 weitere Stunden bei Raumtemperatur, kühlt sodann das Reaktionsgemisch auf 0 °C und filtriert die farblosen Kristalle ab.

1. Zers. p. 220 °C unter Wiedererstarren
2. Schmp. 264-265 °C unter Zersetzung

Analog der in Beispiel 137 angegebenen Vorschrift erhält man aus den entsprechend substituierten Verbindungen der Formel II mit Z = Halogen und den Thioharnstoffen der Formel III beispielsweise die folgenden Verbindungen der Formel XII mit Z = Halogen

a) 4-(4-Chlor-3-chlorsulfonylphenyl)-2-(4-methoxyphenyl-imino)-3-methylthiazolidin-4-ol-hydrobromid,

b) 4-(4-Chlor-3-chlorsulfonylphenyl)-3-methyl-2-(2-methylphenyl-imino)-thiazolidin-4-ol-hydrobromid, Schmp. 236-238 °C (Zers.),

c) 4-(4-Chlor-3-chlorsulfonylphenyl)-2-(2-chlorphenyl-imino)-3-methylthiazolidin-4-ol-hydrobromid,

d) 4-(4-Chlor-3-chlorsulfonylphenyl)-3-methyl-2-(2,4-dimethylphenyl-imino)-thiazolidin-4-ol-hydrobromid,

e) 3-Äthyl-4-(4-chlor-3-chlorsulfonylphenyl)-2-(2-methylphenyl-imino)-thiazolidin-4-ol-hydrobromid,

f) 2-(2,4-Diäthylphenyl-imino)-4-(4-chlor-3-chlorsulfonylphenyl)-3-methylthiazolidin-4-ol-hydrobromid,

g) 4-(4-Chlor-3-chlorsulfonylphenyl)-2-(4-chlorphenyl-imino)-3-methylthiazolidin-4-ol-hydrobromid,

h) 4-(4-Chlor-3-chlorsulfonylphenyl)-2-(4-fluorphenyl-imino)-3-methylthiazolidin-4-ol-hydrobromid,

Beispiel 138

4-(4-Chlor-3-methylsulfamoylphenyl)-2-(4-methoxyphenyl-imino)-3-methyl-4-thiazolin-hydrochlorid

10,6 g (0,02 Mol) 4-(4-Chlor-3-chlorsulfonylphenyl)-2-(4-methoxyphenyl-imino)-3-methylthiazolidin-4-ol-hydrobromid werden in eine Mischung aus 10 ml 40 %igem wäßrigen Methylamin und 150 ml Methanol eingetragen und 20 Stunden bei Raumtemperatur gerührt. Man destilliert das Solvens ab, nimmt den Rückstand in 100 ml Äthanol auf, stellt mit methanolischer oder äthanolischer Chlorwasserstofflösung sauer und erhitzt 2 Stunden zum Sieden. Man destilliert das Lösungsmittel ab, bringt den Rückstand unter Äther, Essigester oder Diisopropyläther zur Kristallisation und filtriert die Kristalle ab. Schmp. 257 °C (Zers.) (aus Isopropanol).

Analog der in Beispiel 138 angegebenen Vorschrift erhält man aus den entsprechend substituierten Verbindungen der Formel XII mit Z = Halogen nach Umsetzung mit einem entsprechend substituierten Amin $HNR^6R^7$ oder Ammoniak beispielsweise die nachfolgend beschriebenen Verbindungen der Formel I :

Beispiel 139

4-(4-Chlor-3-sulfamoylphenyl)-3-methyl-2-(4-methoxyphenyl-imino)-4-thiazolin-hydrochlorid, Schmp. 267 °C (Zers.).

Beispiel 140

4-(4-Chlor-3-sulfamoylphenyl)-3-methyl-2-phenylimino-4-thiazolin-hydrochlorid, Schmp. 242 °C (Zers.) (aus Methanol).

Beispiel 141

4-(4-Chlor-3-dimethylsulfamoylphenyl)-3-methyl-2-phenyl-imino-4-thiazolin-hydrochlorid, Schmp. 228-231 °C (Zers.).

Beispiel 142

4-(3-n-Butylsulfamoyl-4-chlorphenyl)-2-(4-methoxyphenyl-imino)-3-methyl-4-thiazolin-hydrochlorid, ab 102 °C (Zers.).

Beispiel 143

4-(4-Chlor-3-(1-hexylsulfamoyl)-phenyl)-2-(4-methoxyphenyl-imino)-3-methyl-4-thiazolin-hydrochlorid, ab 98 °C (Zers.).

Beispiel 144

4-(3-Benzylsulfamoyl-4-chlorphenyl)-2-(4-methoxyphenyl-imino)-3-methyl-4-thiazolin-hydrochlorid, Zers. ab 135 °C.

Analog der in Beispiel 3 c angegebenen Vorschrift erhält man durch Einwirkung von Protonensäuren der Formel HA auf die basischen Verbindungen der Formel I die weiteren in den folgenden Beispielen beschriebenen Säureadditionssalze der Formel I

Beispiel 146

4-(4-Chlor-3-sulfamoylphenyl)-3-methyl-2-(3-trifluormethylphenyl-imino)-4-thiazolin-hydrochlorid, Schmp. 222 °C.

Beispiel 147

4-(4-Chlor-3-n-propylsulfamoylphenyl)-3-methyl-2-phenylimino-4-thiazolin-hydrochlorid, Schmp. 239 °C.

Beispiel 148

4-[(4-Chlor-3-(4-methylbenzylsulfamoyl)-phenyl]]-3-methyl-2-phenylimino-4-thiazolin, Schmp. 92-100 °C.

Beispiel 149

4-(4-Chlor-3-sulfamoylphenyl)-2-(4-methoxyphenyl-imino)-3-methyl-4-thiazolin-hydrochlorid, Schmp. 276 °C.

Beispiel 150

4-(4-Chlor-3-dimethylsulfamoylphenyl)-3-methyl-2-phenyl-imino-4-thiazolin-amidosulfonat, Schmp. 296-298 °C.

Beispiel 151

4-(4-Chlor-3-dimethylsulfamoylphenyl)-3-methyl-2-(2,4-dimethylphenyl-imino)-4-thiazolin-hydrobromid

3,41 g (0,01 Mol) 2-Brom-4'-chlor-3'-dimethylsulfamoylacetophenon werden in eine Lösung von 1, 83 g (0,01 Mol) 3-Methyl-1-(2,4-dimethylphenyl)-thioharnstoff in 60 ml Eisessig gegeben und 20 Min. bei Raumtemperatur gerührt. Sodann kocht man 20 Min. am Rückflußkühler, läßt abkühlen, versetzt das Reaktionsgemisch mit 60 ml Essigester oder Diisopropyläther und filtriert die Kristalle ab. Schmp. 260-264 °C (Zers.).

Beispiel 152

4-(4-Chlor-3-chlorsulfonylphenyl)-3-methyl-2-phenyl-imino-4-thiazolin-hydrobromid

2,5 g (50 mMol) 4-(4-Chlor-3-chlorsulfonylphenyl)-3-methyl-2-phenyliminothiazolidin-4-ol-hydrobromid werden in einem auf 220 °C vorgeheiztem Mantel im Vakuum (0,1 Torr) über Phosphorpentoxid rasch erhitzt. Die Substanz schmilzt unter Aufschäumen infolge Wasserabspaltung und erstarrt alsbald nach dem Ende der Reaktion unter Rekristallisation. Schwach grün gefärbte Kristalle, Schmp. 264 °C.

Analog der in Beispiel 152 angegebenen Vorschrift erhält man aus den entsprechend substituierten Thiazolidin-4-ol-Derivaten der allgemeinen Formel XII beispielsweise die folgenden Thiazolin-Derivate der Formel XI

a) 4-(4-Chlor-3-chlorsulfonylphenyl)-2-(4-methoxyphenyl-imino)-3-methyl-4-thiazolin-hydrobromid
b) 4-(4-Chlor-3-chlorsulfonylphenyl)-3-methyl-2-(2-methylphenyl-imino)-4-thiazolin-hydrobromid Schmp. 250 °C (Zers.)
c) 4-(4-Chlor-3-chlorsulfonylphenyl)-2-(2-chlorphenyl-imino)-3-methyl-4-thiazolin-hydrobromid
d) 4-(4-Chlor-3-chlorsulfonylphenyl)-3-methyl-2-(2,4-dimethylphenyl-imino)-4-thiazolin-hydrobromid
e) 3-Äthyl-4-(4-chlor-3-chlorsulfonylphenyl)-2-(2-methylphenylimino)-4-thiazolin-hydrobromid
f) 2-(2,4-Diäthylphenyl-imino)-4-(4-chlor-3-chlorsulfonylphenyl)-3-methyl-4-thiazolin-hydrobromid
g) 4-(4-Chlor-3-chlorsulfonylphenyl)-2-(4-chlorphenyl-imino)-3-methyl-4-thiazolin-hydrobromid
h) 4-(4-Chlor-3-chlorsulfonylphenyl)-2-(4-fluorphenyl-imino)-3-methyl-4-thiazolin-hydrobromid

Beispiel 153

4-(4-Chlor-3-dimethylsulfamoylphenyl)-3-methyl-2-phenyl-imino-4-thiazolin

Zu einer Mischung aus 5 ml (ca. 0,05 Mol) 40 %iger wäßriger Dimethylaminlösung und 50 ml Methanol (oder Äthanol) gibt man portionsweise unter Außenkühlung und Rührung 4,8 g (0,01 Mol) 4-(4-Chlor-3-chlorsulfonylphenyl)-3-methyl-2-phenylimino-4-thiazolin-hydrobromid so zu, daß die Temperatur möglichst 35 °C nicht überschreitet. Man rührt 14 Stunden bei Raumtemperatur, destilliert das Lösungsmittel im Wasserstrahlvakuum ab und bringt den Rückstand unter 50 ml Wasser und bei magnetischer Rührung zur Kristallisation. Farblose Kristalle, Schmp. 178-181 °C.

Analog der in Beispiel 153 angegebenen Vorschrift erhält man aus den entsprechend substituierten Verbindungen der Formel XI mit einem entsprechend substituierten Amin der Formel $HNR^6R^7$ bzw. Ammoniak beispielsweise die folgenden Thiazolinderivate der Formel I:

a) 4-(3-Diäthylsulfamoyl-4-chlorphenyl)-3-methyl-2-phenyl-imino-4-thiazolin
b) 4-(4-Chlor-3-sulfamoylphenyl)-3-methyl-2-phenylimino-4-thiazolin
c) 3-Äthyl-4-(4-Chlor-3-dimethylsulfamoylphenyl)-2-phenylimino-4-thiazolin
d) 4-(4-Chlor-3-dimethylsulfamoylphenyl)-3-methyl-2-(2-methylphenylimino)-4-thiazolin
e) 4-(3-Diäthylsulfamoyl-4-chlorphenyl)-3-methyl-2-(2-methylphen ylimino)-4-thiazolin
f) 4-(4-Chlor-3-dimethylsulfamoylphenyl)-3-methyl-2-(2-chlorphenyl-imino)-4-thiazolin
g) 4-(4-Chlor-3-dimethylsulfamoylphenyl)-3-methyl-2-(4-methoxyphenyl-imino)-4-thiazolin
h) 4-(4-Chlor-3-dimethylsulfamoylphenyl)-3-methyl-2-(2,4-dimethylphenyl-imino)-4-thiazolin
i) 4-(4-Chlor-3-dimethylsulfamoylphenyl)-2-(4-chlorphenyl-imino)-3-methyl-4-thiazolin
j) 4-(4-Chlor-3-dimethylsulfamoylphenyl)-2-(4-fluorphenyl-imino)-3-methyl-4-thiazolin

Beispiel 154

4-(4-Chlor-3-dimethylsulfamoylphenyl)-3-methyl-2-phenylimino-4-thiazolin

0,01 Mol 4-(4-Chlor-3-sulfamoylphenyl)-3-methyl-2-phenylimino-4-thiazolin oder dessen Hydrochlorid werden in einem Gemisch von 110 ml Toluol und 50 ml Wasser suspendiert und die wäßrige Phase mit 2 N NaOH auf pH 13-14 gestellt. Nach Zugabe katalytischer Mengen Benzyl-triäthyl-ammoniumchlorid als Phasentransferkatalysator und 0,024 Mol Dimethylsulfat erhitzt man das Reaktionsgemisch unter Rührung und Beibehaltung des pH-Wertes auf 80 bis 90 °C und fügt in Abständen von etwa 2 Stunden solange jeweils etwa 1 g-Portionen Dimethylsulfat zu, bis sich im Dünnschichtchromatogramm an Kieselgel (Toluol-Essigester, 1 : 1) die vollständige Umsetzung zeigt. Die organische Phase wird zur Zerstörung evt. vorhandener Dimethylsulfatmengen mit wässriger Ammoniaklösung 4 Stunden bei 40° gerührt, über Natriumsulfat getrocknet und das Lösungsmittel abdestilliert. Farblose Kristalle, Schmp. 179-180 °C (aus Eisessig).

Beispiel 155

A. 4-(4-Chlor-3-sulfamoylphenyl)-3-methyl-2-phenyl-iminothiazolidin-4-ol-hydrobromid

Zu einer Lösung aus 1,66 g (0,01 Mol) 3-Methyl-1-phenylthioharnstoff in 100 ml Aceton gibt man unter magnetischer Rührung eine Lösung von 3,13 g (0,01 Mol) 2-Brom-4'-chlor-3'-sulfamoylacetophenon in 50 ml Aceto wobei die Reaktionstemperatur 30 °C nicht übersteigen soll. Nach 5 stündigem Rühren bei Raumtemperatur filtriert man die Kristalle ab. Schmp. 164 °C (Zers.)

B. 4-(4-Chlor-3-sulfamoylphenyl)-3-methyl-2-phenyl-iminothiazolidin-4-ol

a) 4,8 g (0,01 Mol) 4-(4-Chlor-3-sulfamoylphenyl)-3-methyl-2-phenyliminothiazolidin-4-ol-hydrobromid werden in 70 ml Methanol auf 0 °C gekühlt und nach Zugabe von 3 ml Triäthylamin 30 Min. bis 1 Stunde bei Raumtemperatur gerührt. Man destilliert das Methanol unter milden Bedingungen im Vakuum ab, wobei die Badtemperatur unter 40 °C gehalten wird, rührt den Rückstand 30 Min. in Wasser und filtriert die Kristalle ab. Schmp. 125-129 °C (Zers.).

b) 5 g (0,01 Mol) 4-(4-Chlor-3-chlorsulfonylphenyl)-3-methyl-2-phenyliminothiazolidin-4-ol-hydrobromid werden in eine Mischung aus 50 ml Methanol und 5 ml 20 %iges wäßriges Ammoniak eingetragen und 3 Stunden bei Raumtemperatur gerührt. Man destilliert das Lösungsmittel unter milden Bedingungen im Vakuum ab, wobei die Badtemperatur unter 40 °C gehalten wird, rührt den Rückstand 30 Min. in Wasser und filtriert die Kristalle ab. Schmp. 126-129 °C (Zers.)

C. 4-(4-Chlor-3-sulfamoylphenyl)-3-methyl-2-phenyliminothiazolidin-4-ol-hydrochlorid

4 g (0,01 Mol) 4-(4-Chlor-3-sulfamoylphenyl)-3-methyl-2-phenyliminothiazolidin-4-ol werden in 150 ml Aceton, Essigester oder Äther mit ätherischer Chlorwasserstofflösung sauer gestellt und die Kristalle nach 1 bis 3 stündigem Rühren bei Raumtemperatur abfiltriert. Schmp. 179 °C (Zers.)

Analog Beispiel 155 werden die in der folgenden Tabelle 4 aufgeführten Verbindungen der Formel IV erhalten.

(Siehe die Tabelle 4, Seiten 37 bis 45)

Tabelle 4

(Zeichenerklärung: Me = Methyl, Et = Äthyl, Prop = Propyl, But = Butyl, Pent = Pentyl, Hex = Hexyl, Bz = Benzyl, i = Iso, sek. = sekundär, c = Cyclo. Der Substituent Y befindet sich in 4-Stellung am Phenylrest, wobei der Thiazolring in 1- und der Sulfamoylrest in 3-Position festgelegt sind)

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 156 | Me | 2-Me | 4-Me | H | H | Me | Me | Cl | HBr | 257 |
| 157 | Me | 2-Me | 4-Cl | H | H | Me | Me | Cl | HBr | 223 |
| 158 | Me | 4-F | H | H | H | Me | Me | Cl | HBr | 247 |
| 159 | Me | 2-Et | H | H | H | Me | Me | Cl | HBr | 276 |
| 160 | But | 2-Me | H | H | H | Me | Me | Cl | HBr | 239 |
| 161 | Me | 2-OMe | 4-Cl | H | H | Me | Me | Cl | HBr | 242 |
| 162 | Me | H | H | H | H | $-(CH_2)_2-O-(CH_2)_2-$ | | Cl | – | 130 |
| 163 | Me | 2-Me | 3-Cl | H | H | Me | Me | Cl | HBr | 223 |
| 164 | Me | $4-NEt_2$ | H | H | H | Me | Me | Cl | HBr | 223 |
| 165 | Me | H | H | H | H | $-(CH_2)_2-N(Me)-(CH_2)_2-$ | | Cl | – | 148 |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 166 | Me | H | H | H | H | H | c-Prop | Cl | – | 107 |
| 167 | Me | H | H | H | H | H | c-Pent | Cl | – | 178 |
| 168 | Me | H | H | H | H | H | c-Pent | Cl | HCl | |
| 169 | Me | H | H | H | H | $-CH_2-CH-Me$<br>O<br>$-CH_2-CH-Me$ | | Cl | – | 125 |
| 170 | Me | H | H | H | H | H | c-Hex | Cl | – | 113 |
| 171 | Me | 3-Me | H | H | H | H | H | Cl | HBr | 178 |
| 172 | Me | 2-Me | 3-Me | H | H | Prop | Prop | Cl | HBr | 211 |
| 173 | Me | H | H | H | H | H | Et | Cl | – | 140 |
| 174 | Me | H | H | H | H | H | 4-Cl-Bz | Cl | – | 77 |
| 175 | Me | H | H | H | H | H | 4-Me-Bz | Cl | – | 85 |
| 176 | Me | 2-MeO | 4-MeO | 5-Cl | H | H | H | Cl | HBr | 262 |
| 177 | Me | 2-Me | 4-MeO | 5-Cl | H | Prop | Prop | Cl | HBr | 208 |
| 178 | Me | H | H | H | H | H | 4-MeO-Bz | Cl | – | 68 |
| 179 | Me | H | H | H | H | Me | But | Cl | – | 119 |
| 180 | Me | H | H | H | H | Prop | Prop | Cl | - | 146 |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 181 | Me | 3-$NMe_2$ | H | H | H | H | H | Cl | HBr | 169 |
| 182 | Me | 2-Me | H | H | H | Me | Me | Cl | HBr | 249 |
| 183 | Me | 4-Cl | H | H | H | Me | Me | Cl | HBr | 233 |
| 184 | Me | 2-Me | 3-Me | H | H | Me | Me | Cl | HBr | 245 |
| 185 | Me | 2-Cl | H | H | H | Et | Et | Cl | HBr | 206 |
| 186 | Me | 2-Me | H | H | H | Et | Et | Cl | HBr | 248 |
| 187 | Me | 2-Me | H | H | H | Me | But | Cl | HBr | — (amorph, |
| 188 | Me | 2-Me | 4-Me | H | H | H | H | Cl | HBr | 220 |
| 189 | Me | 2-Me | 4-Me | H | H | Prop | Prop | Cl | HBr | 236 |
| 190 | Me | 2-EtO | 5-Me | H | H | H | H | Cl | HBr | 169 |
| 191 | Me | 2-MeO | 4-Me | 5-Me | H | H | H | Cl | HBr | 255 |
| 192 | Me | 2-Me | H | H | H | Prop | Prop | Cl | HBr | 216 |
| 193 | Me | H | H | H | H | H | Prop | Cl | — | 90 |
| 194 | Me | H | H | H | H | H | 3-Me-Pent | Cl | — | 152 |
| 195 | Me | H | H | H | H | $-CH_2-CH-Me$<br>$CH_2$<br>$-CH_2-CH-Me$ | | Cl | — | (amorph, |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 196 | Me | 4-$NEt_2$ | H | H | H | H | H | Cl | HCl | 180 |
| 197 | Me | H | H | H | H | Et | Et | Cl | – | 145 |
| 198 | sek.But | H | H | H | H | Me | Me | Cl | HBr | 203 |
| 199 | Hex | H | H | H | H | Me | Me | Cl | HBr | 231 |
| 200 | c-Prop | H | H | H | H | Me | Me | Cl | HBr | 264 |
| 201 | c-Hex | H | H | H | H | Me | Me | Cl | HBr | 233 |
| 202 | Me | 3-MeO | 4-MeO | 5-MeO | H | Me | Me | Cl | HBr | 242 |
| 203 | Prop | H | H | H | H | Me | Me | Cl | HBr | 125 |
| 204 | Me | 4-$CF_3$ | H | H | H | Me | Me | Cl | HBr | 223 |
| 205 | Me | 2-Cl | 4-Cl | 5-Me | H | Me | Me | Cl | HBr | 242 |
| 206 | Me | 3,4-O-$CH_2$-O | | H | H | Me | Me | Cl | HBr | 234 |
| 207 | Me | 3,4-O-$(CH_2)_2$-O- | | H | H | Me | Me | Cl | HBr | 261 |
| 208 | Me | 3-MeO | 4-MeO | 5-MeO | H | H | H | Cl | HBr | 240 |
| 209 | Me | 3,4-O-$CH_2$-O- | | H | H | H | H | Cl | HBr | 283 |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 210 | Me | 3,4-O-$(CH_2)_2$-O- | | H | H | H | H | Cl | HBr | 275 |
| 211 | Me | 3-$CF_3$ | H | H | H | H | H | Cl | HBr | 234 |
| 212 | Me | 3-$CF_3$ | H | H | H | H | H | Cl | – | 174 |
| 213 | Me | 3-$CF_3$ | H | H | H | H | H | Cl | HCl | 208 |
| 214 | Me | 4-Me | H | H | H | H | H | Cl | HCl | 184 |
| 215 | Me | 4-Me | H | H | H | H | H | Cl | HBr | 256 |
| 216 | Me | 4-EtO | H | H | H | H | H | Cl | HBr | 176 |
| 217 | Me | 4-MeO | H | H | H | Me | Me | Cl | HBr | 232 |
| 218 | Me | 2-Me | 3-Me | H | H | H | H | Cl | HCl | 189 |
| 219 | Me | 2-Et | H | H | H | H | H | Cl | HCl | 170 |
| 220 | Me | 2-Et | H | H | H | H | H | Cl | – | 124 |
| 221 | Me | 2-Et | H | H | H | H | H | Cl | HBr | 177 |
| 222 | Me | 4-MeO | H | H | H | H | H | Cl | HBr | 162 |
| 223 | Me | 4-MeO | H | H | H | H | H | Cl | HCl | 179 |
| 224 | Me | 2-Me | 4-MeO | H | H | H | H | Cl | HCl | 191 |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 225 | Et | 4-Me | H | H | H | H | H | Cl | HBr | 212 |
| 226 | Me | H | H | H | H | Me | Me | Cl | HBr | 250 |
| 227 | Me | H | H | H | H | Me | Me | Cl | – | 138 |
| 228 | Me | 2-Cl | H | H | H | H | H | Cl | HBr | 247 |
| 229 | Me | H | H | H | H | Prop | Prop | Cl | HBr | 201 |
| 230 | Me | H | H | H | H | Prop | Prop | Cl | – | 117 |
| 231 | Me | 2-Cl | H | H | H | Prop | Prop | Cl | HBr | 227 |
| 232 | Me | 2-Cl | H | H | H | Prop | Prop | Cl | – | 126 |
| 233 | Me | 4-Cl | H | H | H | H | H | Cl | HBr | 168 |
| 234 | Me | 4-Cl | H | H | H | H | H | Cl | – | 228 |
| 235 | Me | $4-NEt_2$ | H | H | H | H | H | Cl | HBr | 172 |
| 236 | Me | $4-NEt_2$ | H | H | H | H | H | Cl | – | 179 |
| 237 | Me | 2-Me | 3-Me | H | H | H | Me | Cl | – | 100 |
| 238 | Me | H | H | H | H | $-(CH_2)_5-$ | | Cl | – | 110 |
| 239 | Me | H | H | H | H | $-(CH_2)_4-$ | | Cl | – | 110 |
| 240 | Me | H | H | H | H | H | Dodecyl | Cl | – | – (Öl) |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 241 | Me | H | H | H | H | But | H | Cl | – | 120 |
| 242 | Me | H | H | H | H | H | Hex | Cl | – | – (Öl) |
| 243 | Me | 2-Me | 3-Me | H | H | H | H | Cl | HBr | 189 |
| 244 | Me | 2-Me | 3-Me | H | H | H | H | Cl | – | 110 |
| 245 | Me | 2-Me | 3-Me | H | H | H | Me | Cl | HBr | 237 |
| 246 | Me | 2-Me | H | H | H | H | sek.But | Cl | HBr | 252 |
| 247 | Me | 3-Cl | H | H | H | H | H | Cl | HCl | 205 |
| 248 | Me | 4-i-Prop | H | H | H | H | H | Cl | HCl | 270 |
| 249 | Me | 2-Cl | H | H | H | H | H | Cl | HCl | 186 |
| 250 | Me | 2-MeO | H | H | H | H | H | Cl | HCl | 247 |
| 251 | Me | 4-EtO | H | H | H | H | H | Cl | HCl | 174 |
| 252 | Me | 2-MeO | Cl | H | H | H | H | Cl | HCl | 209 |
| 253 | Me | $3-CF_3$ | H | H | H | H | H | Br | HBr | 215 |
| 254 | Me | $3-CF_3$ | H | H | H | H | H | Br | – | 94 |
| 255 | Et | H | H | H | H | H | H | Cl | HBr | 249 |
| 256 | Et | H | H | H | H | H | H | Cl | – | 155 |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 257 | Me | H | H | H | H | H | H | Cl | HCl | 179 |
| 258 | Me | H | H | H | H | H | H | Cl | – | 126 |
| 259 | Me | H | H | H | H | H | H | Br | HBr | 162 |
| 260 | Me | H | H | H | Me | H | H | Cl | HBr | 145 |
| 261 | Me | H | H | H | Et | H | H | Cl | HBr | 144 |
| 262 | Me | H | H | H | H | H | c-Pent | Cl | – | 94 |
| 263 | Me | H | H | H | H | H | c-Hex | Cl | – | 108 |
| 264 | Me | H | H | H | H | H | c-Hex | Cl | HCl | |
| 265 | Me | H | H | H | H | H | Allyl | Cl | – | 87 |
| 266 | Me | H | H | H | H | Me | c-Hex | Cl | – | 143 |
| 267 | Me | H | H | H | H | H | Bz | Cl | – | 73 |
| 268 | Me | H | H | H | H | Me | Bz | Cl | – | 69 |
| 269 | Me | H | H | H | H | H | 2,4-$(MeO)_2$-Bz | Cl | – | 74 |
| 270 | Me | H | H | H | H | H | 2-Cl-Bz | Cl | – | 77 |
| 271 | Me | 4-MeO | H | H | H | H | H | Br | HBr | 177 |

| Beispiel-Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 272 | Me | 4-MeO | H | H | H | Prop | Prop | Cl | HBr | 206 |
| 273 | Prop | H | H | H | H | H | H | Cl | HBr | 149 |
| 274 | Allyl | H | H | H | H | H | H | Cl | HBr | 242 |
| 275 | sek. But | H | H | H | H | H | H | Cl | HBr | 260 |
| 276 | Me | 4-MeO | H | H | H | Me | Me | Cl | HBr | 214 |
| 277 | Me | H | H | H | H | H | c-Pent | Cl | HCl | 164 |
| 278 | Me | 4-EtO | H | H | H | H | H | Cl | HBr | 176 |
| 279 | Me | 4-Br | H | H | H | Me | Me | Cl | HBr | 269 |
| 280 | Me | 2-Me | H | 6-Me | H | Me | Me | Cl | HBr | 244 |
| 281 | Me | 2-Br | H | H | H | Me | Me | Cl | HBr | 242 |

*1) Die meisten der aufgeführten Verbindungen schmelzen unter Zersetzung.

Beispiel 282

4-(4-Chlor-3-dimethylsulfamoylphenyl)-3-methyl-2-phenylimino-4-thiazolin

a) 3-Methyl-4-oxo-2-phenyliminothiazolidin-hydrobromid

10 g Bromessigsäureäthylester und 9,95 g 1-Methyl-3-phenylthioharnstoff werden in 150 ml Aceton 1 Stunde am Rückflußkühler gekocht. Man läßt abkühlen, filtriert die Kristalle ab und wäscht mit Aceton nach. Schmp. 212-215 °C.

b) 3-Methyl-4-oxo-2-phenyliminothiazolidin

4 g 3-Methyl-4-oxo-2-phenyliminothiazolidin-hydrobromid werden in 100 ml Äthanol suspendiert und mit 8,4 g Triäthylamin versetzt. Die erhaltene Lösung rührt man 3 Stunden bei Raumtemperatur, destilliert das Äthanol ab, versetzt den Rückstand mit Wasser, extrahiert mehrmals mit Essigester und destilliert nach Trocknung der organischen Phasen über Natriumsulfat das Lösungsmittel ab. Gelbes viskoses Öl.

c) 4-(4-Chlor-3-dimethylsulfamoylphenyl)-3-methyl-2-phenylimino-4-thiazolin

Zu einer bei − 78 °C unter Argon gerührten Lösung von 3 g 5-Brom-2-chlorbenzol-dimethylsulfonamid in 40 ml absol. Tetrahydrofuran gibt man innerhalb von 10 min 20 mmol tert.-Butyllithium in Pentan. Die Lösung wurde über ca. 60 min bei − 78 °C gehalten, sodann mit 2 g 3-Methyl-4-oxo-2-phenyliminothiazolin versetzt und das Reaktionsgemisch über Nach bei Raumtemperatur gerührt. Man gießt in 15 ml gesättigte Ammoniumchloridlösung, extrahiert mehrfach mit Chloroform, wäscht die vereinigten organischen Phasen mit Wasser und trocknet über Magnesiumsulfat. Nach Filtration des Trockenmittels versetzt man mit 60 ml Eisessig, erhitzt 2 Stunden zum Sieden, destilliert das Lösungsmittel ab und chromatographiert nach Lösen des Rückstandes in 5 ml Chloroform auf der Säule an Kieselgel mit einem 1 : 1 Gemisch aus Essigester/Toluol. Kristalle, Schmp. 177-179 °C.

Beispiel 283

4-(4-Chlor-3-dimethylsulfamoylphenyl)-3-methyl-2-phenyl-imino-4-thiazolin

a) N-Methyl-N'-phenylcarbodiimid

Eine Suspension aus 2,05 g N-Methyl-N'-phenyl-chlorformamidin-hydrochlorid in 8 ml Chloroform wird bei 10-12 °C zu 6 g 20 %iger Natronlauge gegeben. Man rührt das Gemisch 10 Min., trennt die organische Phase ab, extrahiert die wäßrige Phase noch zweimal mit Chloroform und trocknet die vereinigten organischen Phasen über $K_2CO_3$. Nach der Filtration des Trockenmittels setzt man die Lösung ohne Isolierung des Carbodiimides weiter um.

b) 4-(4-Chlor-3-dimethylsulfamoylphenyl)-3-methyl-2-phenylimino-4-thiazolin

Die unter a) dargestellte Lösung gibt man zu einer unter Sauerstoffausschluß gerührten Lösung aus 2,8 g 4'-Chlor-3'-dimethylsulfamoylacetophenon-2-thiol in 55 ml Chloroform, rührt 2 Stunden bei Raumtemperatur und kocht 4 weitere Stunden am Rückflußkühler. Nach Abdestillieren des Chloroforms kocht man den Rückstand 30 Min. in 25 ml Eisessig, destilliert das Solvenz ab und unterwirft den Rückstand — wie in Beispiel 282 c) beschrieben — der Säulenchromatographie an Kieselgel mit Essigester/Toluol = 1 : 1 als Elutionsmittel. Blassgelbe Kristalle, Schmp. 177-180 °C.

Beispiel 284

4-(4-Chlor-3-dimethylsulfamoylphenyl)-3-methyl-2-phenylimino-4-thiazolin

a) N-Methyl-N'-phenyl-chlorformamidin-hydrochlorid

Nachdem 6,3 g Phosgen in 40 ml absol. Tetrahydrofuran bei Raumtemperatur eingeleitet wurden, versetzt man unter Rührung mit 8 g 1-Methyl-3-phenylthioharnstoff, wobei sich die Suspension augenblicklich nach gelb verfärbt. Nach Zugabe von 0,5 ml Dimethylformamid rührt man 20 Stunden bei Raumtemperatur, leitet sodann zur Vertreibung des Phosgens für etwa 30 Min. Stickstoff durch das Reaktionsgemisch, filtriert die Kristalle ab und wäscht mit Tetrahydrofuran nach. Kristalle, Schmp. 169 °C (Zers.).

b) 4-(4-Chlor-3-dimethylsulfamoylphenyl)-3-methyl-2-phenylimino-4-thiazolin

Zu einer Mischung aus 3 g 4'-Chlor-3'-dimethylsulfamoylacetophenon-2-thiol und 2,1 g N-Methyl-N'-phenylchlorformamidin-hydrochlorid in 50 ml Isopropanol tropft man im Verlauf von 30 Min. unter Feuchtigkeitsausschluß und Außenkühlung eine Lösung von 2 g Triäthylamin in 10 ml Isopropanol, wobei die Reaktionstemperatur zwischen 10 und 15 °C gehalten wird.

Nach Zugabe von 50 ml Chloroform rührt man über Nacht bei Raumtemperatur und kocht nach Zugabe von 20 ml Eisessig 1 Stunde am Rückflußkühler. Man destilliert das Lösungsmittel unter vermindertem Druck ab, nimmt den Rückstand in 10 ml Chloroform auf, wäscht die organische Phase mit Wasser und unterwirft diese nach dem Trocknen über $MgSO_4$ der Säulenchromatographie (Kieselgel, Elutionsmittel : Essigester/Toluol, 1 : 1). Farblose Kristalle, Schmp. 178-180 °C (aus Äthanol/Essigester).

Beispiel 285

4-(4-Chlor-3-sulfamoylphenyl)-3-methyl-2-phenylimino-4-thiazolidin

a) 2-Brom-1-(4-chlor-3-sulfamoylphenyl)-äthanol

Zu einer eisgekühlten Lösung aus 3,1 g 2-Brom 4'-chlor-3'-sulfamoylacetophenon und 1 ml wäßriger Methylorangelösung in 20 ml Tetrahydrofuran gibt man unter Rührung 0,94 g Natriumcyanborhydrid und stellt sodann rasch durch Zutropfen einer 1 : 1-Mischung aus Eisessig und 2 N HCl auf den pH 3-4. (Rotfärbung des Indikators), der nachfolgend durch gelegentliches Zutropfen der Säuremischung gehalten wird. Nach ca. 11/2 Stunden ist im Dünnschichtchromatogramm (Kieselgel-Festigplatten Merck, Essigester als Laufmittel) kein Ausgangsmaterial mehr nachweisbar. Man gießt in 300 ml Wasser, sättigt mit Kochsalz und extrahiert mehrfach mit Essigester. Nachdem die vereinigten organischen Phasen mit Wasser gewaschen und über Natriumsulfat getrocknet wurden, engt man am Rota ein. Farblose Kristalle, Schmp. 145 °C (Zers.).

b) S-[2-(4-Chlor-3-sulfamoylphenyl)-2-hydroxyäthyl]-N-methyl-N'-phenylisothioronium-bromid

1,6 g 2-Brom-1-(4-chlor-3-sulfamoylphenyl)-äthanol werden zu einer Lösung von 0,8 g 1-Methyl-3-phenylthioharnstoff in 30 ml Aceton gegeben. Nach Rühren bei Raumtemperatur über 48 Stunden destilliert man das Lösungsmittel unter vermindertem Druck und bringt den Rückstand unter Diisopropyläther zur Kristallisation. Gelber Feststoff, Schmp. 115 °C (Zers.).

c) 4-(4-Chlor-3-sulfamoylphenyl)-3-methyl-2-phenylimino-4-thiazolin

1,5 g s-[2-(4-Chlor-3-sulfamoylphenyl)-2-hydroxyäthyl]-N-methyl-N'-phenylisothioronium-bromid werden in 70 ml Methylenchlorid gelöst und nach Zugabe von 15 g aktiven Mangan-IV-oxid 30 Stunden bei Raumtemperatur gerührt. Nach Filtration des anorganischen Niederschlages rührt man die organische Phase intensiv über 1 Stunde mit wäßriger Natriumbicarbonatlösung, wäscht einmal mit Wasser, versetzt mit 50 m. Eisessig, kocht 1 Stunde am Rückflußkühler und vertreibt unter vermindertem Druck das Lösungsmittel. Der Rückstand zeigt ein mit dem Produkt von Beispiel 73 identisches Dünnschichtchromatogramm (Merck Kieselgel Fertigplatten, Laufmittel Essigester), Schmp. 168-171 °C.

Beispiel 286

4-(4-Chlor-3-N-methyl-N-cyclohexylsulfamoylphenyl)-3-methyl-2-phenylimino-4-thiazolin

erhält man analog der in Beispiel 138 angegebenen Vorschrift unter Verwendung von N-Methyl-N-cyclohexylamin als Aminkomponente. Farblose Kristalle, Schmp. 180-181 °C.

Beispiel 287

4-[4-Chlor-3-(1-methyl-4-piperazinylsulfonyl)-phenyl]-3-methyl-2-phenylimino-4-thiazolin

erhält man analog der in Beispiel 138 angegebenen Vorschrift unter Verwendung von N-Methylpiperazin als Aminkomponente. Farblose Kirstalle, Schmp. 160 °C (Zers.).

Analog der in Beispiel 1a) angegebenen Vorschrift erhält man aus den entsprechend substituierten Ketonen der Formel II mit X in der Bedeutung von Chlor oder Brom und Z in der Bedeutung von $NR^6R^7$ die in den folgenden Beispielen aufgeführten Thiazoline der Formel I

Beispiel 288

4-(4-Fluor-3-sulfamoylphenyl)-3-methyl-2-phenylimino-4-thiazolin-hydrobromid, Schmp. 281 °C (Zers.).

Beispiel 289

3-Methyl-4-(4-methyl-3-sulfamoylphenyl)-2-phenylimino-4-thiazolin-hydrochlorid, Schmp. 268 °C (Zers.).

Beispiel 290

3-Methyl-4(3-dimethylsulfamoylphenyl)-2-phenylimino-4-thiazolin-hydrobromid, Schmp. 254 °C (Zers.).

Beispiel 291

2-(4-Methoxyphenyl-imino)-3-methyl-4-(3-dimethylsulfamoylphenyl)-4-thiazolin-hydrobromid, Schmp. 234 °C (Zers.).

Beispiel 292

4-(4-Chlor-3-dimethylsulfamoylphenyl)-2-(3-trifluormethylphenyl-imino)-3-methyl-4-thriazolin-hydrobro-

mid, Schmp. 242 °C.

Beispiel 293

4-(4-Chlor-3-methylsulfamoylphenyl)-3-methyl-2-phenylimino-4-thiazolin-hydrobromid, Schmp. 269 °C.

Beispiel 294

3-Allyl-4-(4-chlor-3-dimethylsulfamoylphenyl)-2-phenylimino-4-thiazolin-hydrobromid, Schmp. 207 °C (Zers.).

Beispiel 295

4-(4-Chlor-3-sulfamoylphenyl)-3-cyclopentyl-2-phenylimino-4-thiazolin-hydrobromid, Schmp. 236 °C.

Beispiel 296

4-(4-Chlor-3-sulfamoylphenyl)-3-cyclooctyl-2-phenylimino-4-thiazolin-hydrobromid, Schmp. 217 °C.

Beispiel 297

3-Methyl-4-(4-methyl-3-dimethylsulfamoylphenyl)-2-phenylimino-4-thiazolin-hydrobromid, Schmp. 226 °C.

Beispiel 298

2-(4-Methoxyphenyl-imino)-3-methyl-4-(4-methyl-3-dimethylsulfamoylphenyl)-4-thiazolin-hydrobromid, Schmp. 186 °C.

Beispiel 299

2-(2-Chlorphenyl-imino)-3-methyl-4-(4-methyl-3-dimethylsulfamoylphenyl)-4-thiazolin-hydrobromid, Schmp. 218 °C.

Beispiel 300

3-Äthyl-4-(4-methyl-3-dimethylsulfamoylphenyl)-2-phenylimino-4-thiazolin-hydrobromid, Schmp. 225 °C.

Aus den Säureadditionssalzen der Verbindungen der Formel I lassen sich durch Einwirkung einer Base analog der in den Beispielen 2a), 27 und 35 b) angegebenen Vorschriften die in den folgenden Beispielen aufgeführten basischen Verbindungen der Formel I erhalten :

Beispiel 301

3-Methyl-4-(3-dimethylsulfamoylphenyl)-2-phenylimino-4-thiazolin, Schmp. 254 °C.

Beispiel 302

2-(4-Methoxyphenylimino)-3-methyl-4-(3-dimethylsulfamoylphenyl)-4-thiazolin, Schmp. 234 °C.

Beispiel 303

4-(4-Chlor-3-dimethylsulfamoylphenyl)-3-methyl-2-(3-trifluormethylphenyl-imino)-4-thiazolin, Schmp. 226 °C.

Beispiel 304

4-(4-Chlor-3-methylsulfamoylphenyl)-3-methyl-2-phenylimino-4-thiazolin, Schmp. 274 °C.

Beispiel 305

2-(4-Bromphenylimino)-4-(4-chlor-3-dimethylsulfamoylphenyl)-3-methyl-4-thiazolin, Schmp. 185-188 °C.

Beispiel 306

2-(2-Bromphenylimino)-4-(4-chlor-3-dimethylsulfamoylphenyl)-3-methyl-4-thiazolin, Schmp. 155 °C.

Beispiel 307

3-Methyl-4-(4-methyl-3-dimethylsulfamoylphenyl)-2-phenylimino-4-thiazolin, Schmp. 175 °C.

Beispiel 308

2-(4-Methoxyphenyl-imino)-3-methyl-4-(4-methyl-3-dimethylsulfamoylphenyl)-4-thiazolin, Schmp. 180 °C.

Beispiel 309

2-(4-Chlorphenyl(-imino)-3-methyl-4-(4-methyl-3-dimethylsulfamoylphenyl)-4-thiazolin, Schmp. 172 °C.

Beispiel 310

3-Äthyl-4-(4-methyl-3-dimethylsulfamoylphenyl)-2-phenylimino-4-thiazolin, Schmp. 175 °C.

Beispiel 311

4-(4-Chlor-3-dimethylsulfamoylphenyl)-3-methyl-2-(2,6-dimethylphenyl-imino)-4-thiazolin, Schmp. 180 °C.

Analog der in Beispiel 1b) angegebenen Vorschrift erhält man aus den entsprechend substituierten Thiazolidin-4-ol-Derivaten IV die in den folgenden Beispielen aufgeführten Thiazolin-Derivate der Formel I

Beispiel 312

4-(4-Chlor-3-dimethylsulfamoylphenyl)-3-methyl-2-(2,6-dimethylphenylimino)-4-thiazolin-hydrobromid, Schmp. 249 °C.

Beispiel 313

2-(2-Bromphenylimino)-4-(4-chlor-3-dimethylsulfamoylphenyl)-3-methyl-4-thiazolin-hydrobromid, Schmp. 245 °C.

Beispiel 314

2-(4-Bromphenylimino)-4-(4-chlor-3-dimethylsulfamoylphenyl)-3-methyl-4-thiazolin-hydrobromid, Schmp. 269 °C.

Beispiel 315

3-Methyl-4-(2-methyl-5-dimethylsulfamoylphenyl)-2-phenylimino-4-thiazolin-hydrobromid, Schmp. 236 °C (Zers.).

Beispiel 316

3-Methyl-4-(3-methyl-5-dimethylsulfamoylphenyl)-2-phenylimino-4-thiazolin-hydrobromid, Schmp. 205 °C (Zers.).

Beispiel 317

2-(2-Chlorphenylimino)-3-methyl-4-(2-methyl-5-dimethylsulfamoylphenyl)-4-thiazolin-hydrobromid, Schmp. 234 °C (Zers.).

Beispiel 318

2-(4-Methoxyphenylimino)-3-methyl-4-(3-methyl-5-dimethylsulfamoylphenyl)-4-thiazolin-hydrobromid, Schmp. 247 °C (Zers.).

Beispiel 319

i-(4-Isopropylphenyl-imino)-3-methyl-4-(3-methyl-5-dimethylsulfamoylphenyl)-4-thiazolin-hydrobromid, Schmp. 224 °C (Zers.).

Beispiel 320

2-(4-Chlorphenyl-imino)-3-methyl-4-(3-methyl-5-dimethylsulfamoylphenyl)-4-thiazolin-hydrobromid, Schmp. 212 °C (Zers.).

Beispiel 321

2-(4-Fluorphenyl-imino)-3-methyl-4-(2-methyl-5-dimethylsulfamoylphenyl)-4-thiazolin-hydrobromid, Schmp. 225 °C (Zers.).

Beispiel 322

3-Methyl-2-(2-methylphenyl-imino)-4-(2-methyl-5-dimethylsulfamoylphenyl)-4-thiazolin-hydrobromid, Schmp. 263 °C (Zers.).

Beispiel 323

2-(4-Fluorphenyl-imino)-3-methyl-4-(3-methyl-5-dimethylsulfamoylphenyl)-4-thiazolin-hydrobromid, Schmp. 222 °C (Zers.).

Beispiel 324

3-Methyl-2-(2-methylphenyl-imino)-4-(3-methyl-5-dimethylsulfamoylphenyl)-4-thiazolin-hydrobromid, Schmp. 207 °C (Zers.).

Beispiel 325

2-(2-Chlorphenyl-imino)-4-(2-chlor-5-dimethylsulfamoylphenyl)-3-methyl-4-thiazolin-hydrobromid, Schmp. 227 °C (Zers.).

Beispiel 326

2-(2-Chlorphenylimino)-4-(3-chlor-5-dimethylsulfamoylphenyl)-3-methyl-4-thiazolin-hydrobromid, Schmp. 242 °C (Zers.).

Beispiel 327

4-(3-Chlor-5-dimethylsulfamoylphenyl)-2-(4-methoxyphenylimino)-3-methyl-4-thiazolin-hydrobromid, Schmp. 228 °C (Zers.).

Beispiel 328

3-Äthyl-4-(3-chlor-5-dimethylsulfamoylphenyl)-2-phenylimino-4-thiazolin-hydrobromid, Schmp. 230 °C (Zers.).

Beispiel 329

3-Methyl-4-(2-methyl-5-sulfamoylphenyl)-2-phenylimino-4-thiazolin-hydrobromid, Schmp. 220 °C (Zers.).

Beispiel 330

2-(2-Chlorphenyl-imino)-3-methyl-4-(2-methyl-5-sulfamoylphenyl)-4-thiazolin-hydrobromid, Schmp. 195 °C (Zers.).

Beispiel 331

3-Methyl-4-(3-methyl-5-sulfamoylphenyl)-2-phenylimino-4-thiazolin-hydrobromid, Schmp. 280 °C (Zers.).

Beispiel 332

2-(4-Chlorphenyl-imino)-3-methyl-4-(3-methyl-5-sulfamoyl)-phenyl)-4-thiazolin-hydrobromid, Schmp. 257 °C (Zers.).

Beispiel 333

2-(4-Isopropylphenyl-imino)-3-methyl-4-(3-methyl-5-sulfamoylphenyl)-4-thiazolin-hydrobromid, Schmp. 256 °C (Zers.).

Beispiel 334

2-(4-Methoxyphenyl-imino)-3-methyl-4-(2-methyl-5-sulfamoylphenyl)-4-thiazolin-hydrobromid, Schmp. 170 °C (Zers.).

Beispiel 335

4-(2-Chlor-5-dimethylsulfamoylphenyl)-2-(4-methoxyphenyl-imino)-3-methyl-4-thiazolin-hydrobromid, Schmp. 249 °C.

Beispiel 336

4-(2-Chlor-5-dimethylsulfamoylphenyl)-3-methyl-2-(4-methylphenyl-imino)-4-thiazolin-hydrobromid, Schmp. 205 °C.

Beispiel 337

4-(2-Chlor-5-sulfamoylphenyl)-3-methyl-2-phenylimino-4-thiazolin-hydrobromid, Schmp. 276 °C (Zers.).

Beispiel 338

2-(2-Chlorphenyl-imino)-4-(2-chlor-5-sulfamoylphenyl)-3-methyl-4-thiazolin-hydrobromid, Schmp. 254 °C (Zers.).

Beispiel 339

3-Äthyl-4-(2-chlor-5-sulfamoylphenyl)-2-phenylimino-4-thiazolin-hydrobromid, Schmp. 257 °C (Zers.).

Beispiel 340

4-(2-Chlor-5-sulfamoylphenyl)-2-(3-trifluormethylphenylimino)-3-methyl-4-thiazolin-hydrobromid, Schmp. 268 °C.

Beispiel 341

4-(2-Chlor-5-sulfamoylphenyl)-2-(4-methoxyphenyl(-imino)-3-methyl-4-thiazolin-hydrobromid, Schmp. 267 °C (Zers.).

Beispiel 342

4-(2-Chlor-5-methylsulfamoylphenyl)-3-methyl-2-phenylimino-4-thiazolin-hydrobromid, Schmp. 270 °C (Zers.).

Beispiel 343

2-(2-Chlorphenyl-imino)-4-(2-chlor-5-methylsulfamoylphenyl)-3-methyl-4-thiazolin-hydrobromid, Schmp. 257 °C (Zers.).

Beispiel 344

3-Äthyl-4-(2-chlor-5-methylsulfamoylphenyl)-2-phenylimino-4-thiazolin-hydrobromid, Schmp. 236 °C (Zers.).

Beispiel 345

4-(2-Chlor-5-methylsulfamoylphenyl)-2-(3-trifluormethylphenyl-imino)-3-methyl-4-thiazolin-hydrobromid, Schmp. 208 °C (Zers.).

Beispiel 346

4-(2-Chlor-S-methylsulfamoylphenyl)-2-(4-methoxyphenyl-imino)-3-methyl-4 thiazolin-hydrobromid Schmp. 257 °C (Zers.).

Beispiel 347

4-(2-Brom-5-dimethylsulfamoylphenyl)-3-methyl-2-phenylimino-4-thiazolin-hydrobromid, Schmp. 225-227 °C (Zers.).

Beispiel 348

4-(2-Brom-5-dimethylsulfamoylphenyl)-2-(2-chlorphenylimino)-3-methyl-4-thiazolin-hydrobromid, Schmp. 227 °C.

Beispiel 349

4-(2-Brom-5-dimethylsulfamoylphenyl)-3-methyl-2-(2,4-dimethylphenyl-imino)-4-thiazolin-hydrobromid, Schmp. 227-228 °C (Zers.).

Beispiel 350

3-Äthyl-4-(2-brom-5-dimethylsulfamoylphenyl)-2-(2-methylphenylimino)-4-thiazolin-hydrobromid, Schmp. 205-208 °C (Zers.).

Aus den entsprechenden Säureadditionssalzen der Verbindungen der Formel I lassen sich durch Einwirkung einer Base analog der in den Beispielen 2a, 27 und 35b angegebenen Vorschriften die in den folgenden Beispielen aufgeführten basischen Verbindungen der Formel I erhalten :

Beispiel 351

3-Methyl-4-(2-methyl-5-dimethylsulfamoylphenyl)-2-phenylimino-4-thiazolin, Schmp. 190 °C.

Beispiel 352

3-Methyl-4-(3-methyl-5-dimethylsulfamoylphenyl)-2-phenylimino-4-thiazolin, Schmp. 166 °C.

Beispiel 353

4-(2-Chlor-5-dimethylsulfamoylphenyl)-3-methyl-2-phenylimino-4-thiazolin, Schmp. 197 °C.

Beispiel 354

4-(3-Chlor-5-dimethylsulfamoylphenyl)-3-methyl-2-phenylimino-4-thiazolin, Schmp. 167 °C.

Beispiel 355

4-(2-Chlor-5-dimethylsulfamoylphenyl)-2-(2-chlorphenylimino)-3-methyl-4-thiazolin, Schmp. 227 °C.

Beispiel 356

3-Äthyl-4-(2-chlor-5-dimethylsulfamoylphenyl)-2-phenylimino-4-thiazolin, Schmp. 201 °C (Zers.).

Beispiel 357

4-(3-Chlor-5-dimethylsulfamoylphenyl)-2-(2-chlorphenylimino)-3-methyl-4-thiazolin, Schmp. 163 °C.

Beispiel 358

3-Methyl-4-(2-methyl-5-sulfamoylphenyl)-2-phenylimino-4-thiazolin, Schmp. 188-191 °C.

Beispiel 359

3-Methyl-4-(3-methyl-5-sulfamoylphenyl)-2-phenylimino-4-thiazolin, Schmp. 210-212 °C.

Beispiel 360

4-(2-Chlor-5-sulfamoylphenyl)-3-methyl-2-phenylimino-4-thiazolin, Schmp. 198-200 °C.

Beispiel 361

4-(2-Brom-5-dimethylsulfamoylphenyl)-3-methyl-2-phenylimino-4-thiazolin, Schmp. 204 °C.

Beispiel 362

4-(2-Brom-5-dimethylsulfamoylphenyl)-3-methyl-2-(2-chlorphenylimino)-4-thiazolin, Schmp. 242 °C (Zers.).

Beispiel 363

4-(2-Brom-5-dimethylsulfamoylphenyl)-3-methyl-2-(2,4-dimethylphenyl-imino)-4-thiazolin, Schmp. 260 °C (Zers.).

Beispiel 364

3-Äthyl-4-(2-brom-5-dimethylsulfamoylphenyl)-2-(2-methylphenyl-imino)-4-thiazolin, Schmp. 209-210 °C (Zers.).

Beispiel 365

2-(4-Methoxyphenyl-imino)-3-methyl-4-(2-methyl-5-dimethylsulfamoylphenyl)-4-thiazolin, Schmp. 186-189 °C.

Beispiel 366

3-Äthyl-4-(3-methyl-5-dimethylsulfamoylphenyl)-2-(2-methylphenylimino)-4-thiazolin, Schmp. 155 °C.

Beispiel 367

2-(4-Chlorphenyl-imino)-3-methyl-4-(3-methyl-5-sulfamoylphenyl)-4-thiazolin, Schmp. 195 °C.

Entsprechend der in Beispiel 1a) angegebenen Vorschrift erhält man aus den entsprechend substituierten Ketonen der Formel II mit X in der Bedeutung von Brom und Z in der Bedeutung von $NR^6R^7$ die in den folgenden Beispielen aufgeführten Thiazoline der Formel I

Beispiel 368

4-(2-Chlor-5-dimethylsulfamoylphenyl)-3-methyl-2-phenylimino-4-thiazolin-hydrobromid, Schmp. 247 °C (Zers.).

Beispiel 369

4-(3-Chlor-5-dimethylsulfamoylphenyl)-3-methyl-2-phenylimino-4-thiazolin-hydrobromid, Schmp. 234 °C (Zers.).

Beispiel 370

3-Äthyl-4-(2-chlor-5-dimethylsulfamoylphenyl)-2-phenylimino-4-thiazolin-hydrobromid, Schmp. 175 °C (Zers.).

Beispiel 371

4-(2-Chlor-5-dimethylsulfamoylphenyl)-2-(2-chlorphenylimino)-3-methyl-4-thiazolin-hydrobromid, Schmp. 227 °C (Zers.).

Beispiel 372

4-(2-Chlor-5-dimethylsulfamoylphenyl)-3-methyl-2-phenylimino-4-thiazolin vom Schmp. 195-197 °C erhält man analog der in Beispiel 284 b) angegebenen Vorschrift durch Umsetzung von 2'-Chlor-5'-dimethylsulfamoylacetophenon-2-thiol mit N-Methyl-N'-phenyl-chlorformamidin-hydrochlorid.

Das verwendete 2'-Chlor-5'-dimethylsulfamoylacetophenon-2-thiol erhält man als hellgelbes Kristallpulver durch alkalische Hydrolyse von 2-Acetylthio-2'-chlor-5'-dimethylsulfamoylacetophenon mit 5 %iger wäßriger Natronlauge bei Raumtemperatur unter Argonathmosphäre als Schutzgas.

Das 2-Acetylthio-2'-chlor-5'-dimethylsulfamoylacetophenon wird durch Umsetzung von 2-Brom-2'-chlor-5'-dimethylsulfamoylacetophenon mit Thioessigsäure, die mit KOH neutralisiert wurde, in Äthanol erhalten. Nach der Umsetzung gießt man das Reaktionsgemisch in Wasser, extrahiert mit Essigester, trocknet die organische Phase über Magnesiumsulfat und kristallisiert den durch Verfampfen des Lösungsmittels erhaltenen Rückstand aus Isopropanol (*Aktivkohle*) um. Schmp. 84-88 °C.

Analog Beispiel 155 werden weiterhin die in der folgenden Tabelle 4a) aufgeführten Verbindungen der Formel IV erhalten :

Tabelle 4a):

(Zeichenerklärung wie von Tabelle 4, die Stellung für den Substituenten Y ist jeweils angegeben, die Sulfamoylgruppe ist auf die 5-Position festgelegt)

| Beispiel Nr. | $R^1$ | $R^2$ | $R^3$ | $R^4$ | $R^5$ | $R^6$ | $R^7$ | Y | HA | Schmp.*1) (°C) |
|---|---|---|---|---|---|---|---|---|---|---|
| 373 | Me | H | H | H | H | Me | Me | 2-Me | HBr | 192 |
| 374 | Me | H | H | H | H | Me | Me | 3-Me | HBr | 205 |
| 375 | Me | 2-Cl | H | H | H | Me | Me | 2-Me | HBr | 228 |
| 376 | Me | 4-MeO | H | H | H | Me | Me | 3-Me | HBr | 157 |
| 377 | Me | 4-iProp | H | H | H | Me | Me | 3-Me | HBr | 219 |
| 378 | Me | 4-Cl | H | H | H | Me | Me | 3-Me | HBr | 212 |
| 379 | Me | 4-F | H | H | H | Me | Me | 2-Me | HBr | 220 |
| 380 | Me | 2-Me | H | H | H | Me | Me | 2-Me | HBr | 225 |
| 381 | Me | 4-F | H | H | H | Me | Me | 3-Me | HBr | 217 |
| 382 | Me | 2-Me | H | H | H | Me | Me | 3-Me | HBr | 208 |
| 383 | Me | 2-Cl | H | H | H | Me | Me | 2-Cl | HBr | 186 |
| 384 | Me | 2-Cl | H | H | H | Me | Me | 3-Cl | HBr | 237 |
| 385 | Me | 4-MeO | H | H | H | Me | Me | 3-Cl | HBr | 225 |
| 386 | Et | H | H | H | H | Me | Me | 3-Cl | HBr | 202 |
| 387 | Me | H | H | H | H | H | H | 2-Me | HBr | 208 |
| 388 | Me | 2-Cl | H | H | H | H | H | 2-Me | HBr | 180 |
| 389 | Me | H | H | H | H | H | H | 3-Me | HBr | 280 |
| 390 | Me | 4-Cl | H | H | H | H | H | 3-Me | HBr | 160 |
| 391 | Me | 4-iProp | H | H | H | H | H | 3-Me | HBr | 254 |
| 392 | Me | 4-OMe | H | H | H | H | H | 2-Me | HBr | 184 |
| 393 | Me | 4-OMe | H | H | H | Me | Me | 2-Cl | HBr | 177 |
| 394 | Me | 4-Me | H | H | H | Me | Me | 2-Cl | HBr | 197 |
| 395 | Me | H | H | H | H | H | H | 2-Cl | HBr | 271 |

Tabelle 4 (Fortsetzung)

| Beispiel Nr. | $R^1$ | $R^2$ | $R^3$ | $R^4$ | $R^5$ | $R^6$ | $R^7$ | Y | HA | Schmp. *1) |
|---|---|---|---|---|---|---|---|---|---|---|
| 396 | Me | 2-Cl | H | H | H | H | H | 2-Cl | HBr | 254 |
| 397 | Et | H | H | H | H | H | H | 2-Cl | HBr | 210 |
| 398 | Me | 3-$CF_3$ | H | H | H | H | H | 2-Cl | HBr | 268 |
| 399 | Me | 4-OMe | H | H | H | H | H | 2-Cl | HBr | 168 |
| 400 | Me | H | H | H | H | H | Me | 2-Cl | HBr | 268 |
| 401 | Me | 2-Cl | H | H | H | H | Me | 2-Cl | HBr | 190 |
| 402 | Et | H | H | H | H | H | Me | 2-Cl | HBr | 236 |
| 403 | Me | 3-$CF_3$ | H | H | H | H | Me | 2-Cl | HBr | 208 |
| 404 | Me | 4-OMe | H | H | H | H | Me | 2-Cl | HBr | 200 |
| 405 | Me | H | H | H | H | Me | Me | 2-Br | HBr | 239 |
| 406 | Me | 2-Cl | H | H | H | Me | Me | 2-Br | HBr | 193 |
| 407 | Me | 2-Me | 4-Me | H | H | Me | Me | 2-Br | HBr | 203 |
| 408 | Et | 2-Me | H | H | H | Me | Me | 2-Br | HBr | 188 |

Herstellung von Verbindungen der Formel (II)

Darstellung von 2-Brom-3'-methyl-5'-sulfamoylacetophenon

Zu einer Suspension von 5 g (0,0234 Mol) 3'-Methyl-5'-sulfamoylacetophenon in 70 ml Essigester tropft man ca. 5 ml einer Lösung von 3,7 g (0,0294 Mol) Brom in 30 ml Essigester und erwärmt auf ca. 40 °C bis zum plötzlichen Verschwinden der Bromfärbung. Nun tropft man bei Raumtemperatur unter Rührung rasch die restliche Bromlösung zu und destilliert sodann das Lösungsmittel ab. Kristalle, Schmp. 188-191 °C (aus Isopropanol).

In analoger Weise wurden die nachfolgenden Bromacetophenone dargestellt :
2,2'-Dibrom-5'-dimethylsulfamoyl-acetophenon, Schmp. 88 °C,
2-Brom-3'-chlor-5'-dimethylsulfamoyl-acetophenon, Schmp. 77-78 °C,
2-Brom-2'-chlor-5'-methylsulfamoyl-acetophenon, Schmp. 99-101 °C,
2-Brom-2'-chlor-5'-dimethylsulfamoyl-acetophenon, Schmp. 87-88 °C,
2-Brom-2'-chlor-5'sulfamoylacetophenon, Schmp. 152-154 °C,
2-Brom-3'-methyl-5'-dimethylsulfamoylacetophenon, Schmp. 71-75 °C,
2-Brom-2'-methyl-5'-dimethylsulfamoylacetophenon, Schmp. 69-71 °C,
2-Brom-2'-methyl-5'-sulfamoylacetophenon, Schmp. 112-115 °C.

Darstellung von 2'-Methyl-3'-sulfamoylacetophenon

Zu einer Suspension von 2,7 g Magnesiumspänen (0,11 Mol) in 2,5 g (0,043 Mol) wasserfreien Alkohol gibt man 0,25 ml Tetrachlorkohlenwasserstoff, wobei die Temperatur auf 40 °C ansteigt und tropft sodann langsam 75 ml Äthanol (absol.) zu. Man erwärmt zum Sieden und versetzt mit einem kleinen Teil einer Lösung aus 17,6 g (0,11 Mol) Malonsäurediäthylester, 10 ml (0,17 Mol) absol. Äthanol und 12,5 ml Diäthyläter. Nach dem Anspringen der Reaktion tropft man den Rest der Lösung so zu, daß das Gemisch ohne äußeres Erhitzen am Sieden bleibt. Sodann erhitzt man weitere 3 Stunden zum Rückfluß, wobei das Magnesium gelöst wird, tropft unter Beibehaltung des Siedens eine Lösung von 11,6 g (0,05 Mol) 2-Methyl-5-sulfamoylbenzoylchlorid in 100 ml Essigester zu und kocht weitere 2 Stunden am Rückflußkühler. Das Reaktionsgemisch wird nach dem Abkühlen auf Raumtemperatur in eine Mischung aus 15 g konz. Schwefelsäure, 200 ml Wasser und 300 ml Essigester gegossen, extrahiert, die organische Phase abgetrennt und die wäßrige Phase weitere 2 mal mit Essigester ausgeschüttelt. Man trocknet über Magnesiumsulfat, destilliert das Lösungsmittel ab und verarbeitet den öligen Rückstand (2'-Methyl-5'-sulfamoylbenzoyl-malonsäurediäthylester) ohne weitere Reinigungsoperation weiter.

Der als Öl anfallende 2'-Methyl-5'-sulfamoylbenzoylmalonester wird langsam auf 110 °C erwärmt, wobei man ab ca. 80 °C beginnt, 18 ml 85 %ige Phosphorsäure zuzutropfen. Man erwärmt bis zum Ende der $CO_2$-Entwicklung und heizt eine weitere halbe Stunde auf 110 °C. Nach dem Abkühlen versetzt man mit etwa 200 ml Wasser, extrahiert mehrfach mit Essigester, wäscht die vereinigten organischen Phasen mit Wasser, trocknet über $MgSO_4$, vertreibt das Lösungsmittel, versetzt den Rückstand mit Diisopropyläther und filtriert die Kristalle ab. kristalle, Schmp. 115-117 °C.

In analoger Weise werden die nachfolgenden Sulfamoylacetophenonderivate hergestellt :
2'-Methyl-5'-dimethylsulfamoyl-acetophenon, Schmp. 54-56 °C.
3'-Methyl-5'-sulfamoyl-acetophenon, Schmp. 165-168 °C.
3'-Methyl-5'-dimethylsulfamoyl-acetophenon, Schmp. 106-109 °C.
2'-Chlor-5'-sulfamoyl-acetophenon, Schmp. 114-116 °C.
2'-Chlor-5'-dimethylsulfamoyl-acetophenon, Schmp. 79 °C.
2'-Chlor-5'-methylsulfamoyl-acetophenon, Schmp. 74-75 °C.
3'-Chlor-5'-dimethylsulfamoyl-acetophenon, Schmp. 100-102 °C.
2'-Brom-5'-dimethylsulfamoyl-acetophenon, Schmp. 97-99 °C.

3-Sulfamoylbenzoylchloride

erhält man durch Rückfluß der entsprechenden Sulfamoylbenzoesäurederivate in etwa 15 bis 20-fach überschüssigen Thionylchlorid bis zum Ende der HCl-Entwicklung und nachfolgendem Abdestillieren des Thionylchlorids.

Auf diese Weise wurden die nachfolgenden Verbindungen hergestellt :
2-Methyl-5-sulfamoylbenzoylchlorid, Schmp. 160-161 °C.
2-Methyl-5-dimethylsulfamoylbenzoylchlorid, Schmp. 84-89 °C.
3-Methyl-5-sulfamoylbenzoylchlorid, Schmp. 152-155 °C.
3-Methyl-5-dimethylsulfamoylbenzoylchlorid, Schmp. 72 °C.
2-Chlor-5-sulfamoylbenzoylchlorid, Schmp. 114-116 °C.
2-Chlor-5-dimethylsulfamoylbenzoylchlorid, Schmp. 79 °C.
2-Chlor-5-methylsulfamoylbenzoylchlorid, Schmp. 74-75 °C.
3-Chlor-5-dimethylsulfamoylbenzoylchlorid, Schmp. 74-76 °C.
2-Brom-5-dimethylsulfamoylbenzoylchlorid.

Sulfamoylbenzoesäuren

erhält man durch Eintragen der entsprechenden Chlorsulfonylbenzoesäuren in eine äthanolische Lösung enthaltend mindestens 3 Mol des Amins.

$HNR^6R^7$ bei Raumtemperatur :
2-Brom-5-dimethylbenzoesäure, Schmp. 174-176 °C
3-Chlor-5-dimethylbenzoesäure, Schmp. 155-156 °C
3-Methyl-5-sulfamoylbenzoesäure, Schmp. 258-262 °C
3-Methyl-5-dimethylsulfamoylbenzoesäure, Schmp. 157-162 °C
2-Methyl-5-sulfamoylbenzoesäure, Schmp. 247-251 °C
2-Methyl-5-dimethylsulfamoylbenzoesäure, Schmp. 173-175 °C
2-Chlor-5-dimethylsulfamoylbenzoesäure, Schmp. 170 °C
2-Chlor-5-methylsulfamoylbenzoesäure, Schmp. 174 °C.

Die entsprechenden Chlorsulfonylbenzoesäuren erhält man in an sich bekannter Weise durch Erhitzen der Benzoesäuren mit Chlorsulfonsäuren auf 120 bis 165 °C und nachfolgendes Zersetzen des abgekühlten Reaktionsgemisches durch Eintropfen in Eis-Wassergemisch :
2-Methyl-5-chlorsulfonbenzoesäure, Schmp. 151-155 °C.
3-Methyl-5-chlorsulfonylbenzoesäure, Schmp. 176-180 °C.
3-Chlor-5-chlorsulfonylbenzoesäure,
2-Brom-5-chlorsulfonylbenzoesäure.

(Siehe die Tabelle 5, Seite 57)

Tabelle 5

zeigt einige hergestellten Thioharnstoffe III, die nach literaturbekannten Methoden gewonnen werden (vgl. z. B. Houben-Weyl, « Methoden der organischen » Chemie, Bd. *9*, S. 884, 4 Aufl., Georg-Thieme-Verlag, Stuttgart, 1955).

| $R^1$ | $R^2$ | $R^3$ | $R^4$ | Schmp. (°C) |
|---|---|---|---|---|
| Me | 2-Cl | 4-Cl | 5-Me | 132 |
| Hex | H | H | H | 77 |
| c-Prop | H | H | H | 124 |
| Me | $-O-CH_2-O-$ | | H | 133 |
| Me | $-O-(CH_2)_2-O-$ | | H | 173 |
| Me | $3-NMe_2$ | H | H | 133 |
| Me | $4-CF_3$ | H | H | 145 |
| Me | 3-MeO | 4-MeO | 5-MeO | 167 |
| Me | 2-MeO | 4-MeO | 5-Cl | 193 |
| Me | 2-EtO | 5-Me | H | 111 |
| Me | 2-MeO | 4-Me | 5-Me | 135 |
| Me | 2-Cl | H | H | 146 |
| Me | 4-F | H | H | 93 |
| Me | 2-Me | 4-Me | H | 153 |
| Me | 2-Me | 4-Cl | | 127 |
| Et | 2-Me | H | H | 66 |

**Ansprüche** (für die Vertragsstaaten : BE, CH, DE, FR, GB, IT, LI, NL, SE)

1. Thiazolinderivate der allgemeinen Formel (I)

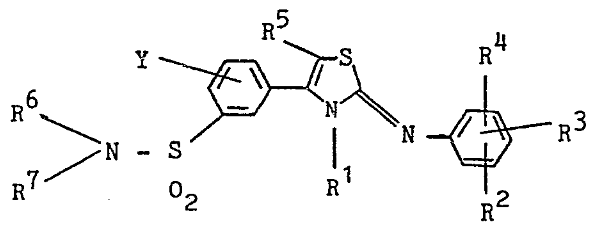

(I)

in welcher

$R^1$ $C_1$-$C_8$-Alkyl, Cycloalkyl mit 3 bis 8 C-Atomen oder Alkenyl mit 3 bis 4 C-Atomen,

$R^2$, $R^3$ und $R^4$ Wasserstoff, Halogen, Alkyl oder Alkoxy mit jeweils 1 bis 4 C-Atomen, Methylendioxy,

Äthylendioxy, Dimethyl- oder Diäthylamino, Trifluormethyl,

$R^5$ Wasserstoff oder Alkyl mit 1 bis 3 C-Atomen,

$R^6$ Wasserstoff oder Alkyl mit 1 bis 6 C-Atomen,

$R^7$ Wasserstoff, Alkyl mit 1 bis 12 C-Atomen, Cycloalkyl mit 3 bis 12 C-Atomen, Allyl, Phenyläthyl- oder einen Benzylrest

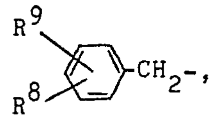

worin

$R^8$ und $R^9$ gleich oder verschieden sind und Wasserstoff, Methyl, Chlor oder Methoxy bedeuten, oder $R^6$ und $R^7$ sind über eine gegebenenfalls verzeigte Alkylenkette mit insgesamt 8 C-Atomen verbunden, worin eine Methylengruppe durch ein O-Atom oder eine $N\text{-}CH_3$-Gruppe ersetzt sein kann, und

Y Wasserstoff, Halogen oder Alkyl mit 1 bis 3 C-Atomen bedeutet, sowie deren Säureadditionssalze mit pharmakologisch verträglichen Säuren.

2. 4-(4-Chlor-3-dimethylsulfamoylphenyl)-3-methyl-2-phenylimino-4-thiazolin.
3. 4-(4-Chlor-3-diäthylsulfamoylphenyl)-3-methyl-2-phenylimino-4-thiazolin.
4. 4-(4-Chlor-3-dimethylsulfamoylphenyl)-2-(2-chlorphenylimino)-3-methyl-4-thiazolin.
5. 4-(4-Chlor-3-dimethylsulfamoylphenyl)-3-methyl-2-(2-methylphenylimino)-4-thiazolin.
6. 4-(4-Chlor-3-dimethylsulfamoylphenyl)-3-methyl-2-(2,4-dimethylphenyl-imino)-4-thiazolin.
7. 4-(2-Chlor-5-dimethylsulfamoylphenyl)-3-methyl-2-2-phenylimino-4-thiazolin.
8. 4-(3-Chlor-5-dimethylsulfamoylphenyl)-3-methyl-2-phenylimino-4-thiazolin.
9. 4-(4-Chlor-3-dimethylsulfamoylphenyl)-2-(4-methoxyphenylimino)-3-methyl-4-thiazolin.
10. 4-(4-Chlor-3-sulfamoylphenyl)-3-methyl-2-phenylimino-4-thiazolin.
11. Verfahren zur Herstellung der Verbindungen gemäss Anspruch 1, dadurch gekennzeichnet, dass man

a) Verbindungen der allgemeinen Formel (II)

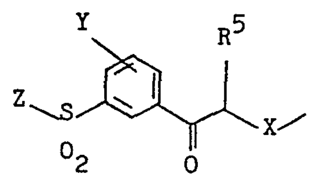

(II)

worin

$R^5$ und Y die angegebene Bedeutung besitzen,

Z für Halogen oder $R^6R^7N$- mit der für $R^6$ und $R^7$ angegebenen Bedeutung steht, und

X eine leaving group ist, unter kondensierenden Reaktionsbedingungen mit einem Thioharnstoff der allgemeinen Formel (III)

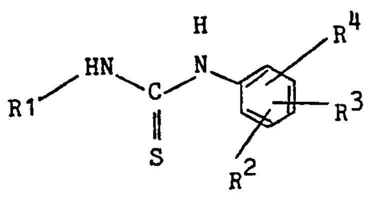

(III)

worin $R^1$ bis $R^4$ die angegebene Bedeutung besitzen, umsetzt, und im Falle dass Z für Halogen steht, eine erhaltene Verbindung der Formel (XI)

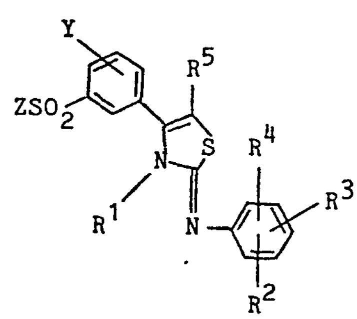

(XI)

worin $R^1$, $R^2$, $R^3$, $R^4$, $R^5$ und Y die zu Formel (I) angegebenen Bedeutungen besitzen und Z Halogen darstellt, nachfolgend mit einem Amin der allgemeinen Formel $HNR^6R^7$ mit der für $R^6$ und $R^7$ angegebenen Bedeutung zur Umsetzung bringt, oder

b) aus Verbindungen der allgemeinen Formel (IV)

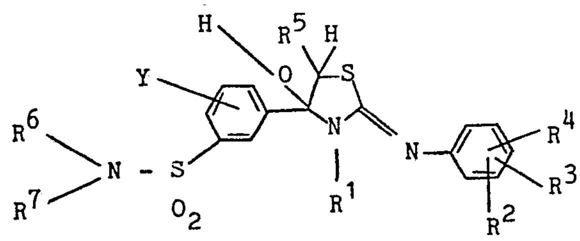

(IV)

worin $R^1$ bis $R^7$ und Y die angegebene Bedeutung besitzen, Wasser abspaltet, oder

c) Verbindungen der allgemeinen Formel (V)

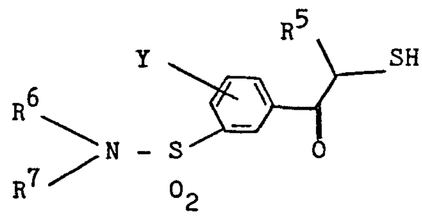

(V)

mit Verbindungen der allgemeinen Formel (VI)

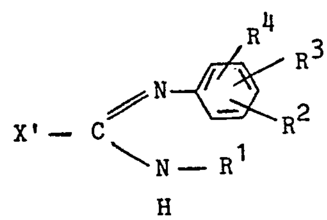

(VI)

zur Reaktion bringt, wobei $R^1$ bis $R^7$ die angegebene Bedeutung haben und X' eine leaving group ist, oder

d) Verbindungen der Formel (V) mit Carbodiimiden (VII)

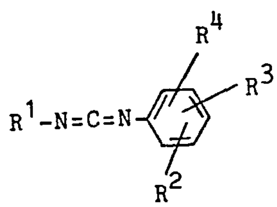

(VII)

umsetzt, wobei $R^1$ bis $R^4$ die angegebene Bedeutung haben, oder

e) Verbindungen der allgemeinen Formel (VIII)

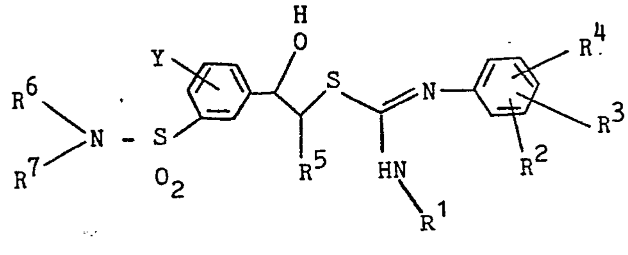

x HHal (VIII)

worin $R^1$ bis $R^7$ und Y die angegebene Bedeutung besitzen und Hal für Chlor oder Brom steht, mit einem Oxidationsmittel behandelt, oder

f) Verbindungen der allgemeinen Formel (IX)

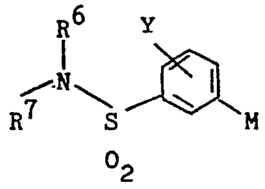

(IX)

worin $R^6$ und $R^7$ nicht für Wasserstoff und Y nicht für Brom und Jod steht, ansonsten aber die obige Bedeutung haben, und M für Lithium oder eine MgBr-Gruppe steht, mit Verbindungen der allgemeinen Formel (X)

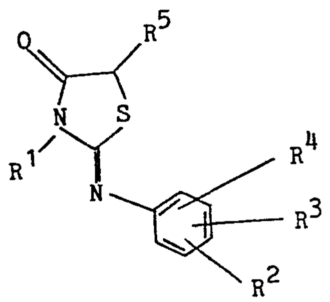

(X)

worin $R^1$ bis $R^5$ die angegebene Bedeutung besitzen, umsetzt und das erhaltene Reaktionsprodukt der Hydrolyse und der Dehydratation unterwirft, und gegebenenfalls die nach Weg a) bis f) erhaltenen Verbindungen der allgemeinen Formel (I), in denen $R^6$ und/oder $R^7$ Wasserstoff bedeutet, durch übliche Alkylierung in Verbindungen überführt, in denen $R^6$ und/oder $R^7$ eine der weiteren oben angegebenen Bedeutungen hat, und gegebenenfalls eine erhaltene Verbindung der Formel (I) mit organischen oder anorganischen Säuren der allgemeinen Formel H-A in ihre Säureadditionssalze oder erhaltene Salze der Verbindungen der allgemeinen Formel (I) mit Basen in die freien basischen Verbindungen der Formel (I) überführt.

12. Verbindungen der Formel (IV)

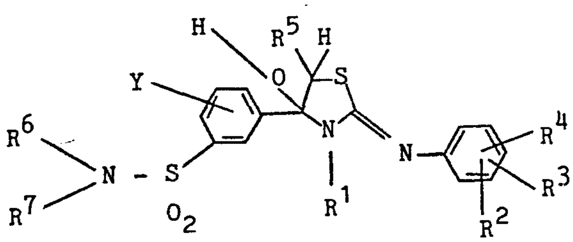

(IV)

worin $R^1$ bis $R^7$ und Y die zu Formel (I) genannten Bedeutungen besitzen, sowie deren Säureadditionssalze.

13. Verbindungen der allgemeinen Formel (XI)

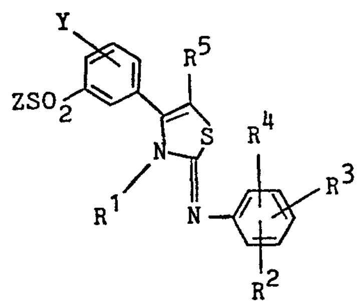

(XI)

worin $R^1$ bis $R^5$ und Y die zu Formel (I) angegebenen Bedeutungen haben und Z für Halogen steht sowie deren Säureadditionssalze.

14. Verbindungen der allgemeinen Formel (XVII)

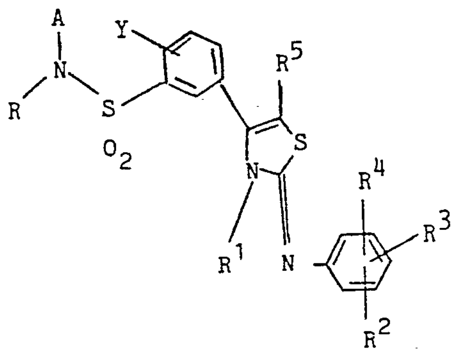

(XVII)

worin $R^1$ bis $R^5$ und Y zu Formel (I) genannten Bedeutungen haben, R die Bedeutung von $R^6$ oder $R^7$ besitzt, und A das Kation eines Alkali- oder Erdalkalimetalls bedeutet.

15. Pharmazeutische Präparate mit das Serumlipoproteinspektrum beeinflussender Wirkung, bestehend aus bzw. enthaltend eine Verbindung gemäss Anspruch 1.

16. Verfahren zur Herstellung pharmazeutischer Präparate mit einer das Serumlipoproteinspektrum beeinflussenden Wirkung, dadurch gekennzeichnet, dass man eine Verbindung gemäss Anspruch 1 gegebenenfalls mit pharmazeutischen Trägern und/oder Stabilisatoren in eine für therapeutische Zwecke geeignete Anwendungsform bringt.

**Ansprüche** (für den Vertragsstaat AT)

1. Verfahren zur Herstellung von Thiazolinderivaten der allgemeinen Formel (I)

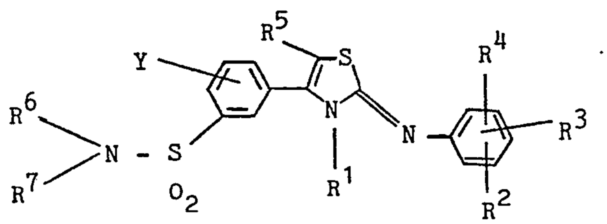

(I)

in welcher

$R^1$ $C_1$-$C_8$-Alkyl, Cycloalkyl mit 3 bis 8 C-Atomen oder Alkenyl mit 3 bis 4 C-Atomen,

$R^2$, $R^3$ und $R^4$ Wasserstoff, Halogen, Alkyl oder Alkoxy mit jeweils 1 bis 4 C-Atomen, Methylendioxy, Athylendioxy, Dimethyl- oder Diäthylamino, Trifluormethyl,

$R^5$ Wasserstoff oder Alkyl mit 1 bis 3 C-Atomen,

$R^6$ Wasserstoff oder Alkyl mit 1 bis 6 A-Atomen,

$R^7$ Wasserstoff, Alkyl mit 1 bis 12 C-Atomen, Cycloalkyl mit 3 bis 12 C-Atomen, Allyl, Phenyläthyl oder einen Benzylrest

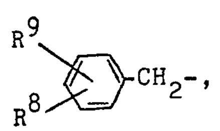

worin $R^8$ und $R^9$ gleich oder verschieden sind und Wasserstoff, Methyl, Chlor oder Methoxy bedeuten, oder $R^6$ und $R^7$ sind über eine gegebenenfalls verzweigte Alkylenkette mit insgesamt 8 C-Atomen verbunden, worin eine methylengruppe durch ein O-Atom oder eine N-$CH_3$-Gruppe ersetzt sein kann, und

Y Wasserstoff, Halogen oder Alkyl mit 1 bis 3 C-Atomen bedeutet, sowie von deren Säureadditionssalzen mit pharmakologisch verträglichen Säuren, dadurch gekennzeichnet, daß man

a) Verbindungen der allgemeinen Formel (II)

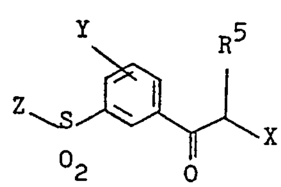

(II)

worin

$R^5$ und Y die angegebene Bedeutung besitzen,

Z für Halogen oder $R^6R^7N$- mit der für $R^6$ und $R^7$ angegebenen Bedeutung steht, und

X eine leaving group ist, unter kondensierenden Reaktionsbedingungen mit einem Thioharnstoff der allgemeinen Formel (III)

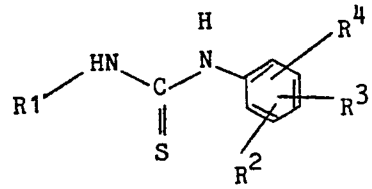

(III)

worin $R^1$ bis $R^4$ die angegebene Bedeutung besitzen, umsetzt, und im Falle dass Z für Halogen steht, eine erhaltene Verbindung der Formel (XI)

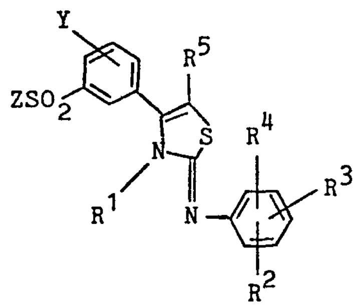

(XI)

worin $R^1$, $R^2$, $R^3$, $R^4$, $R^5$ und Y die zu Formel (I) angegebenen Bedeutungen besitzen und Z Halogen darstellt, nachfolgend mit einem Amin der allgemeinen Formel $HNR^6R^7$ mit der für $R^6$ und $R^7$ angegebenen Bedeutung zur Umsetzung bringt, oder

b) aus Verbindungen der allgemeinen Formel (IV)

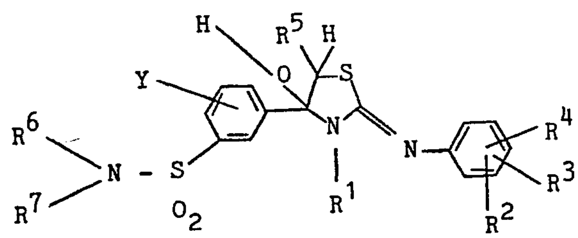

(IV)

worin $R^1$ bis $R^7$ und Y die angegebene Bedeutung besitzen, Wasser abspaltet, oder

c) Verbindungen der allgemeinen Formel (V)

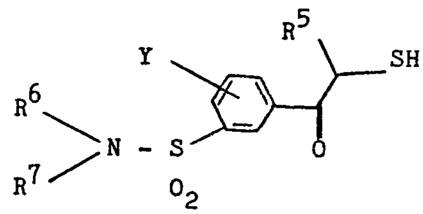

(V)

mit Verbindungen der allgemeinen Formel (VI)

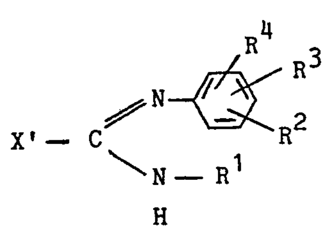

(VI)

zur Reaktion bringt, wobei $R^1$ bis $R^7$ die angegebene Bedeutung haben und X' eine leaving group ist, oder

d) Verbindungen der Formel (V) mit Carbodiimiden VII

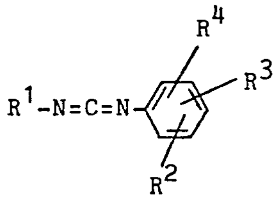

(VII)

umsetzt, wobei $R^1$ bis $R^4$ die angegebene Bedeutung haben, oder

e) Verbindungen der allgemeinen Formel (VIII)

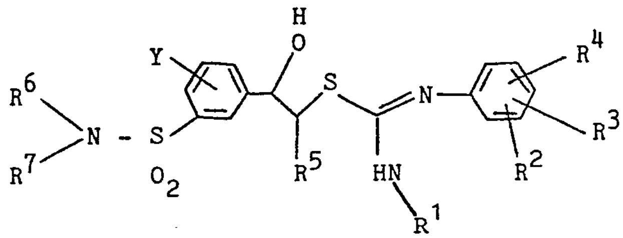

x HHal (VIII)

worin $R^1$ bis $R^7$ und Y die angegebene Bedeutung besitzen und Hal für Chlor oder Brom steht, mit einem Oxidationsmittel behandelt, oder

f) Verbindungen der allgemeinen Formel (IX)

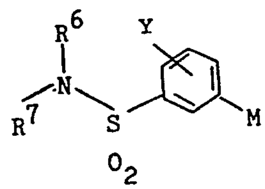

(IX)

worin $R^6$ und $R^7$ nicht für Wasserstoff und Y nicht für Brom und Jod steht, ansonsten aber die obige Bedeutung haben, und M für Lithium oder eine MgBr-Gruppe steht, mit Verbindungen der allgemeinen Formel (X)

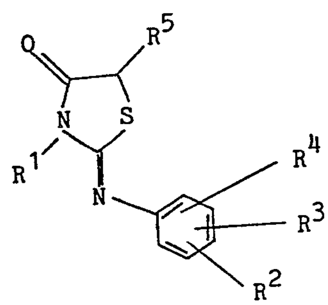

(X)

worin $R^1$ bis $R^5$ die angegebene Bedeutung besitzen, umsetzt und das erhaltene Reaktionsprodukt der Hydrolyse und der Dehydratation unterwirft, und gegebenenfalls die nach Weg a) bis f) erhaltenen Verbindungen der allgemeinen Formel (I), in denen $R^6$ und/oder $R^7$ Wasserstoff bedeutet, durch übliche Alkylierung in Verbindungen überführt, in denen $R^6$ und/oder $R^7$ eine der weiteren oben angegebenen Bedeutungen hat, und gegebenenfalls eine erhaltene Verbindung der Formel (I) mit organischen oder anorganischen Säuren der allgemeinen Formel H-A in ihre Säureadditionssalze oder erhaltene Salze der Verbindungen der allgemeinen Formel (I) mit Basen in die freien basischen Verbindungen der Formel (I) überführt.

2. Verfahren gemäß Anspruch 1, dadurch gekennzeichnet, daß man 4-(4-Chlor-3-dimethylsulfamoyl-phenyl)-3-methyl-2-phenylimino-4-thiazolin herstellt.

3. Verfahren gemäß Anspruch 1, dadurch gekennzeichnet, daß man 4-(4-Chlor-3-diäthylsulfamoyl-phenyl)-3-methyl-2-phenylimino-4-thiazolin herstellt.

4. Verfahren gemäß Anspruch 1, dadurch gekennzeichnet, daß man 4-(4-Chlor-3-dimethylsulfamoylphenyl)-2-(2-chlor-phenylimino)-3-methyl-4-thiazolin herstellt.

5. Verfahren gemäß Anspruch 1, dadurch gekennzeichnet, daß man 4-(4-Chlor-3-dimethylsulfamoylphenyl)-3-methyl-2-(2-methylphenylimino)-4-thiazolin herstellt.

6. Verfahren gemäß Anspruch 1, dadurch gekennzeichnet, daß man 4-(4-Chlor-3-dimethylsulfamoylphenyl)-3-methyl-2-(2,4-dimethylphenyl-imino)-4-thiazolin herstellt.

7. Verfahren gemäß Anspruch 1, dadurch gekennzeichnet, daß man 4-(2-Chlor-5-dimethylsulfamoylphenyl)-3-methyl-2-2-phenylimino-4-thiazolin herstellt.

8. Verfahren gemäß Anspruch 1, dadurch gekennzeichnet, daß man 4-(3-Chlor-5-dimethylsulfamoylphenyl)-3-methyl-2-phenylimino-4-thiazolin herstellt.

9. Verfahren gemäß Anspruch 1, dadurch gekennzeichnet, daß man 4-(4-Chlor-3-dimethylsulfamoylphenyl)-2-(4-methoxyphenylimino)-3-methyl-4-thiazolin herstellt.

10. Verfahren gemäß Anspruch 1, dadurch gekennzeichnet, daß man 4-(4-Chlor-3-sulfamoylphenyl)-3-methyl-2-phenylimino-4-thiazolin herstellt.

11. Verbindungen der Formel (IV)

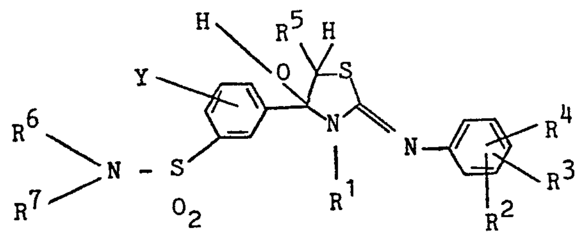

(IV)

worin $R^1$ bis $R^7$ und Y die zu Formel (I) genannten Bedeutungen besitzen, sowie deren Säureadditionssalze.

12. Verbindungen der allgemeinen Formel (XI)

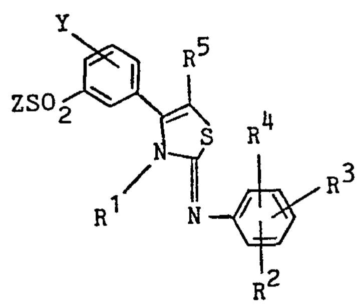

(XI)

worin $R^1$ bis $R^5$ und Y die zu Formel (I) angegebenen Bedeutungen haben und Z für Halogen steht sowie deren Säureadditionssalze.

13. Verbindungen der allgemeinen Formel (XVII)

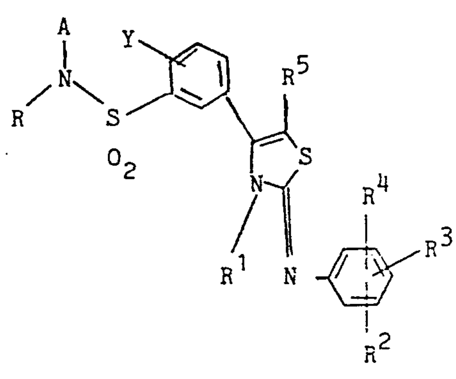

(XVII)

worin $R^1$ bis $R^5$ und Y die zu Formel (I) genannten Bedeutungen haben, R die Bedeutung von $R^6$ oder $R^7$ besitzt, und A das Kation eines Alkali- oder Erdalkalimetalls bedeutet.

**Claims** (for the Contracting States : BE, CH, DE, FR, GB, IT, LI, NE, SE)

1. Thioazoline derivatives of the general formula (I)

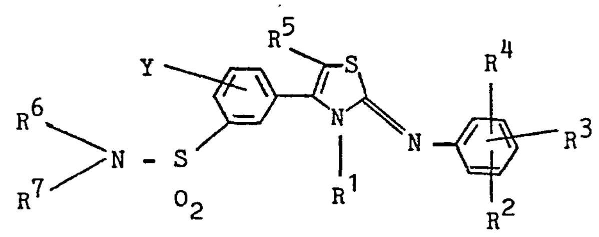

(I)

in which

$R^1$ denotes $C_1$-$C_8$-alkyl, cycloalkyl having 3 to 8 C atoms or alkenyl having 3 to 4 C atoms,

$R^2$, $R^3$ and $R^4$ denote hydrogen, halogen, alkyl or alkoxy each having 1 to 4 C atoms, methylenedioxy, ethylenedioxy, dimethyl- or diethyl-amino or trifluoromethyl,

$R^5$ denotes hydrogen or alkyl having 1 to 3 C atoms,

$R^6$ denotes hydrogen or alkyl having 1 to 6 C atoms and

$R^7$ denotes hydrogen, alkyl having 1 to 12 C atoms, cycloalkyl having 3 to 12 C atoms, allyl, phenylethyl or a benzyl radical

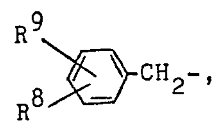

in which $R^8$ and $R^9$ are identical or different and denote hydrogen, methyl, chlorine or methoxy, or $R^6$ and $R^7$ are bonded via an alkylene chain, which can be branched and has a total of 8 C atoms and in which one methylene group can be replaced by a 0 atoms or a $N$-$CH_3$ group, and

Y denotes hydrogen, halogen or alkyl having 1 to 3 C atoms, and their acid addition salts with pharmacologically acceptable acids.

2. 4-(4)Chloro-3-dimethylsulfamoylphenyl)-3-methyl-2-phenyl-imino-4-thiazoline.
3. 4-(4-Chloro-3-diethylsulfamoylphenyl)-3-methyl-2-phenyl-imino-4-thiazoline.
4. 4-(4-Chloro-3-dimethylsulfamoylphenyl)-2-(2-chlorophenyl-imino)-3-methyl-4-thiazoline.
5. 4-(4-Chloro-3-dimethylsulfamoylphenyl)-3-methyl-2-(2-methylphenylimino-4-thiazoline.
6. 4-(4-Chloro-3-dimethylsulfamoylphenyl)-3-methyl-2-(2,4-dimethylphenyl-imino)-4-thiazoline.
7. 4-(2-Chloro-5-dimethylsulfamoylphenyl)-3-methyl-2-phenyl-imino-4-thiazoline.
8. 4-(3-Chloro-5-dimethylsulfamoylphenyl)-3-methyl-2-phenyl-imino-4-thiazoline.
9. 4-(4-Chloro-3-dimethylsulfamoylphenyl)-2-(4-methoxyphenyl-imino)-3-methyl-4-thiazoline.
10. 4-(4-Chloro-3-sulfamoylphenyl)-3-methyl-2-phenylimino-4-thiazoline.
11. Process for the preparation of the compounds as claimed in Claim 1, which comprises

a) reacting compounds of the general formula (II)

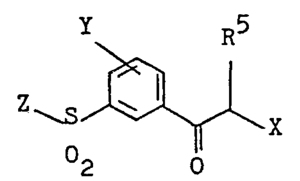

(II)

in which

$R^5$ and Y are as defined,

Z represents halogen or $R^6R^7N$-, in which $R^6$ and $R^7$ are as defined, and

X is a leaving group, under the reaction conditions for a condensation reaction, with a thiourea of the general formula (III)

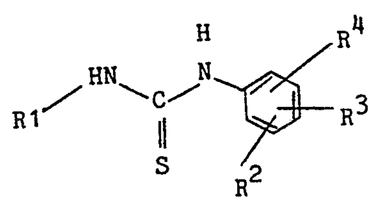

(III)

in which $R^1$ to $R^4$ are as defined, and, if Z represents halogen, subsequently reacting a resulting compound of the formula (XI)

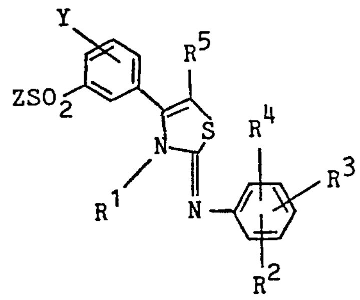

(XI)

in which $R^1$, $R^2$, $R^3$, $R^4$, $R^5$ and Y are as defined under formula (I) and Z represents halogen, with an amine of the general formula $HNR^6R^7$, in which $R^6$ and $R^7$ are as defined, or

b) splitting off water from compounds of the general formula (IV)

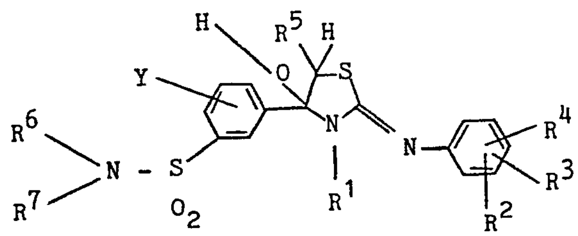

(IV)

in which $R^1$ to $R^7$ and Y are as defined, or

c) reacting compounds of the general formula (V)

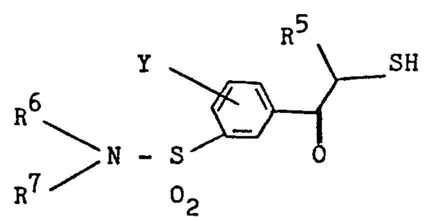

(V)

with compounds of the general formula (VI)

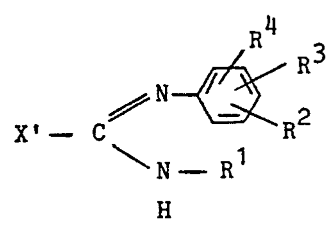

(VI)

in which formulae $R^1$ to $R^7$ are as defined and X' is a leaving group, or

d) reacting compounds of the formula (V) with carbodiimides VII

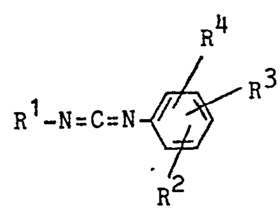

(VII)

in which $R^1$ to $R^4$ are as defined, or

e) treating compounds of the general formula (VIII)

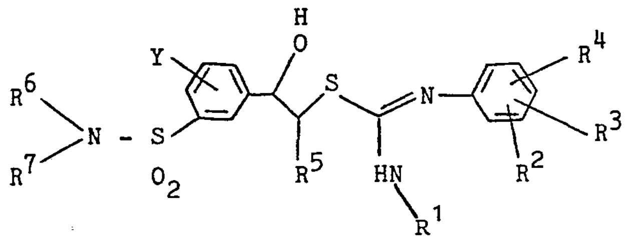

x HHal (VIII)

in which $R^1$ to $R^7$ and Y are as defined and Hal represents chlorine or bromine, with an oxidizing agent, or

f) reacting compounds of the general formula (IX)

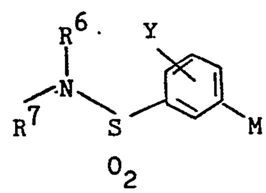

(IX)

in which $R^6$ and $R^7$ do not represent hydrogen and Y does not represent bromine or iodine, but these radicals are otherwise as defined above, and M represents lithium or a MgBr group, with compounds of the general formula (X)

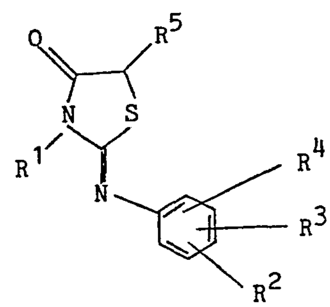

(X)

in which $R^1$ to $R^5$ are as defined, and subjecting the resulting reaction product to hydrolysis and dehydration, and, if desired, converting the compounds of the general formula (I) in which $R^6$ and/or $R^7$ denote hydrogen, which compounds are obtained by route a) to f), by conventional alkylation into compounds in which $R^6$ and/or $R^7$ have one of the other meanings defined above, and, if desired, converting a resulting compound of the formula (I) into its acid addition salts, using organic or inorganic acids of the general formula H-A, or converting resulting salts of the compounds of the general formula I into the free basic compounds of the formula I, using bases.

12. Compounds of the formula (IV)

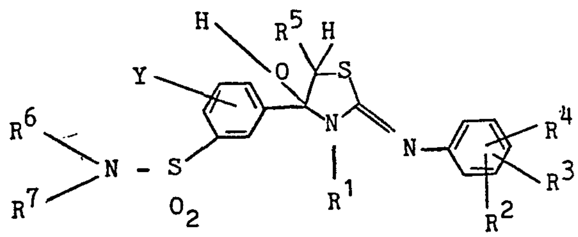

(IV)

in which $R^1$ to $R^7$ and Y are as defined under formula (I), and their acid addition salts.

13. Compounds of the general formula (XI)

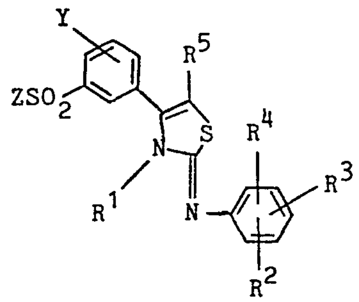

(XI)

in which $R^1$ to $R^5$ and Y are defined under formula (I) and Z represents halogen, and their acid addition salts.

14. Compounds of the general formula (XVII)

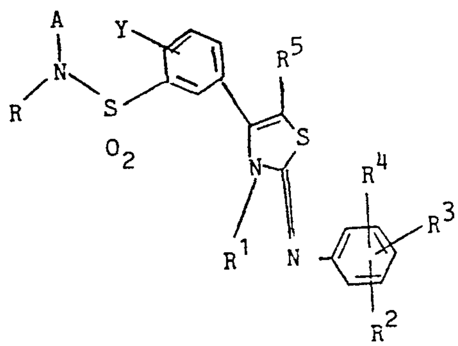

(XVII)

in which $R^1$ to $R^5$ and Y are defined under formula (I) R has the meaning defined for $R^6$ or $R^7$ and A denotes the cation of an alkali metal or alkaline earth metal.

15. Pharmaceutical preparations which have the effect of influencing the serum lipoprotein spectrum and which consist of or contain a compound according to Claim 1.

16. Process for the preparation of pharmaceutical preparations which have the effect of influencing the serum lipoprotein spectrum, which comprises bringing a compound as claimed in Claim 1, optionally with pharmaceutical excipients and/or stabilizers, into a use form suitable for therapeutic purposes.

**Claims** (for the Contracting State AT)

1. Process for the preparation of thiazoline derivatives of the general formula (I)

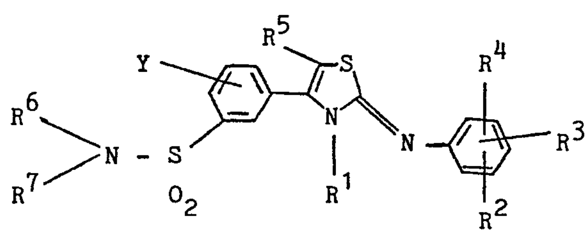

(I)

in which

$R^1$ denotes $C_1$-$C_8$-alkyl, cycloalkyl having 3 to 8 C atoms or alkenyl having 3 to 4 C atoms,

$R^2$, $R^3$ and $R^4$ denote hydrogen, halogen, alkyl or alkoxy each having 1 to 4 C atoms, methylenedioxy, ethylenedioxy, dimethyl- or diethyl-amino or trifluoromethyl,

$R^5$ denotes hydrogen or alkyl having 1 to 3 C atoms,

$R^6$ denotes hydrogen or alkyl having 1 to 6 C atoms and

$R^7$ denotes hydrogen, alkyl having 1 to 12 C atoms, cycloalkyl having 3 to 12 C atoms, allyl, phenylethyl or a benzyl radical

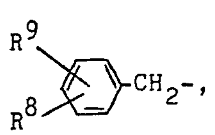

in which $R^8$ and $R^9$ are identical or different and denote hydrogen, methyl, chlorine or methoxy, or $R^6$ and $R^7$ are bonded via an alkylene chain, which can be branched and has a total of 8 C atoms and in which one methylene group can be replaced by a 0 atoms or a $N\text{-}CH_3$ group, and Y denotes hydrogen, halogen or alkyl having 1 to 3 C atoms, and of their acid addition salts with pharmacologically acceptable acids, which comprises

a) reacting compounds of the general formula (II)

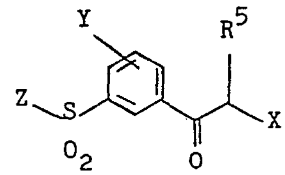

(II)

in which

$R^5$ and Y are as defined,

Z represents halogen or $R^6R^7N$-, in which $R^6$ and $R^7$ are as defined, and

X is a leaving group, under the reaction conditions for a condensation reaction, with a thiourea of the general formula (III)

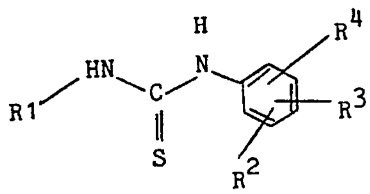

(III)

in which $R^1$ to $R^4$ are as defined, and, if Z represents halogen, subsequently reacting a resulting compound of the formula (XI)

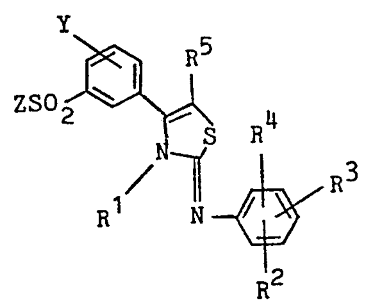

(XI)

in which $R^1$, $R^2$, $R^3$, $R^4$, $R^5$ and Y are defined under formula (I) and Z represents halogen, with an amine of the general formula $HNR^6R^7$, in which $R^6$ and $R^7$ are as defined, or

b) splitting off water from compounds of the general formula (IV)

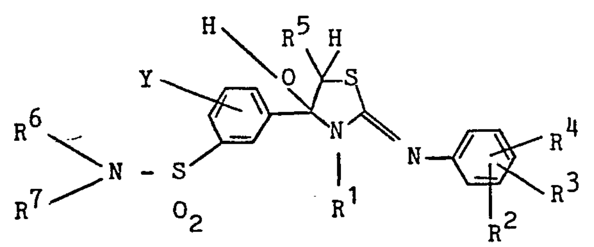

(IV)

in which $R^1$ to $R^7$ and Y are as defined, or

c) reacting compounds of the general formula (V)

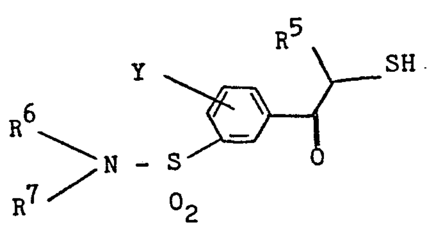

(V)

with compounds of the general formula (VI)

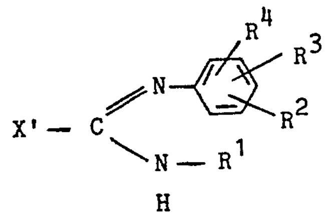

(VI)

in which formulae $R^1$ to $R^7$ are as defined and X' is a leaving group, or

d) reacting compounds of the formula (V) with carbodiimides VII

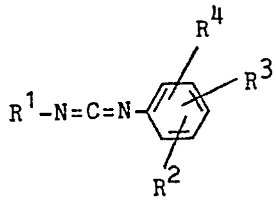

(VII)

in which $R^1$ to $R^4$ are as defined, or

e) treating compounds of the general formula (VIII)

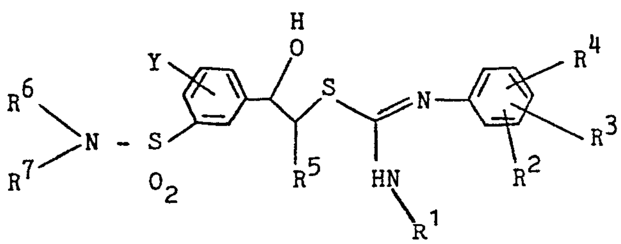

x HHal (VIII)

in which $R^1$ to $R^7$ and Y are defined and Hal represents chlorine or bromine, with an oxidizing agent, or

f) reacting compounds of the general formula (IX)

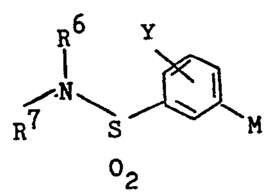

(IX)

in which $R^6$ and $R^7$ do not represent hydrogen and Y does not represent bromine or iodine, but these radicals are otherwise as defined above, and M represents lithium or a MgBr group, with compounds of the general formula (X)

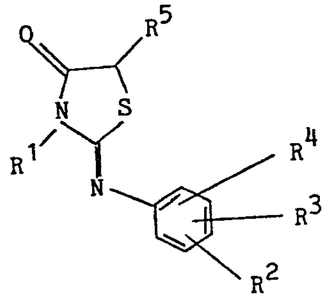

(X)

in which $R^1$ to $R^5$ are as defined, and subjecting the resulting reaction product to hydrolysis and dehydration, and, if desired, converting the compounds of the general formula (I) in which $R^6$ and/or $R^7$ denotes hydrogen, which compounds are obtained by route a) to f), by conventional alkylation into compounds in which $R^6$ and/or $R^7$ have one of the other meanings defined above, and, if desired,

converting a resulting compound of the formula (I) into its acid addition salts, using organic or inorganic acids of the general formula H-A, or converting resulting salts of the compounds of the general formula (I) into the free basic compounds of the formula (I), using bases.

2. A process as claimed in Claim 1 wherein 4-(4-chloro-3-dimethylsulfamoylphenyl)-3-methyl-2-phenylimino-4-thiazoline is prepared.

3. A process as claimed in Claim 1 wherein 4-(4-chloro-3-diethylsulfamoylphenyl)-3-methyl-2-phenylimino-4-thiazoline is prepared.

4. A process as claimed in Claim 1 wherein 4-(4-chloro-3-dimethylsulfamoylphenyl)-2-(2-chlorophenylimino)-3-methyl-4-thiazoline is prepared.

5. A process as claimed in Claim 1 wherein 4-(4-chloro-3-dimethylsulfamoylphenyl)-3-methyl-2-(2-methylphenylimino-4-thiazoline is prepared.

6. A process as claimed in Claim 1 wherein 4-(4-chloro-3-dimethylsulfamoylphenyl)-3-methyl-2-(2,4-dimethylphenylimino)-4-thiazoline is prepared.

7. A process as claimed in Claim 1 wherein 4-(2-Chloro-5-dimethylsulfamoylphenyl)-3-methyl-2-phenylimino-4-thiazoline is prepared.

8. A process as claimed in Claim 1 wherein 4-(3-chloro-5-dimethylsulfamoylphenyl)-3-methyl-2-phenylimino-4-thiazoline is prepared.

9. A process as claimed in Claim 1 wherein 4-(4-Chloro-3-dimethylsulfamoylphenyl)-2-(4-methoxyphenylimino)-3-methyl-4-thiazoline is prepared.

10. A process as claimed in Claim 1 wherein 4-(4-Chloro-3-sulfamoylphenyl)-3-methyl-2-phenylimino-4-thiazoline is prepared.

11. Compounds of the formula (IV)

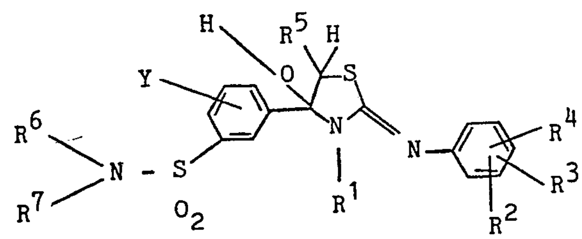

(IV)

in which $R^1$ to $R^7$ and Y are as defined under formula (I), and their acid addition salts,

12. Compounds of the general formula (XI)

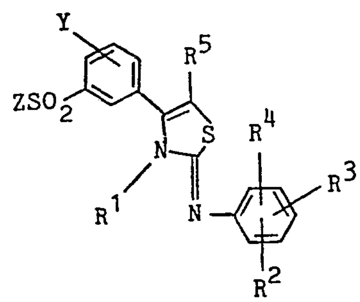

(XI)

in which $R^1$ to $R^5$ and Y are as defined under formula (I) and Z represents halogen, and their acid addition salts.

13. Compounds of the general formula (XVII)

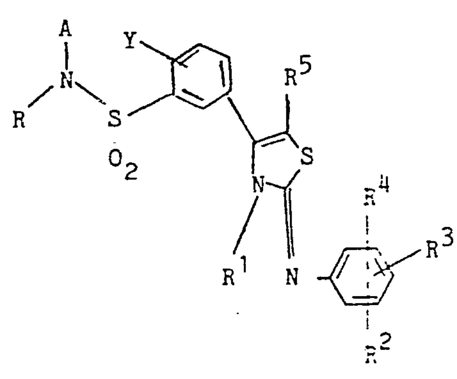

(XVII)

in which $R^1$ to $R^5$ and Y are as defined under formula (I) R has the meaning defined for $R^6$ or $R^7$ and A denotes the cation of an alkali metal or alkaline earth metal.

**Revendications** (pour les Etats contractants : BE, CH, DE, FR, GB, IT, LI, NL, SE)

1. Dérivés de thiazoline de formule générale (I) :

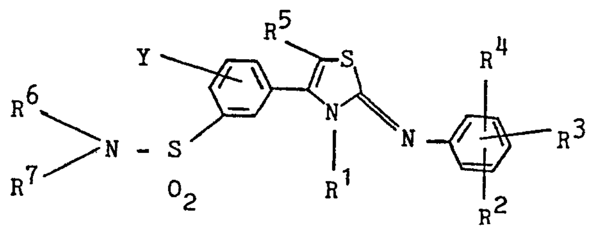

(I)

dans laquelle

$R^1$ représente un groupe alcoyle en $C_1$-$C_8$, cycloalcoyle ayant de 3 à 8 atomes de carbone, ou alcényle ayant de 3 à 4 atomes de carbone,

$R^2$, $R^3$ et $R^4$ représentent l'hydrogène, un halogène, un groupe alcoyle ou alcoxy ayant chacun de 1 à 4 atomes de carbone, méthylènedioxy, éthylènedioxy, diméthyl- ou diéthylamino, trifluorométhyle,

$R^5$ représente l'hydrogène ou un groupe alcoyle ayant de 1 à 3 atomes de carbone,

$R^6$ représente l'hydrogène ou un groupe alcoyle ayant de 1 à 6 atomes de carbone,

$R^7$ représente l'hydrogène, un groupe alcoyle ayant de 1 à 12 atomes de carbone, cycloalcoyle ayant de 3 à 12 atomes de carbone, allyle, phényléthyle ou un radical benzyle

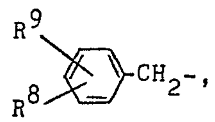

où $R^8$ et $R^9$ sont identiques ou différents et représentent l'hydrogène, un groupe méthyle, chloro ou méthoxy, ou $R^6$ et $R^7$ sont reliés par une chaîne alcoylène éventuellement ramifiée ayant au total 8 atomes de carbone, dans laquelle un groupe méthylène peut être remplacé par un atome d'oxygène ou un groupe $N$-$CH_3$ et

Y représente l'hydrogène, un halogène ou un groupe alcoyle ayant de 1 à 3 atomes de carbone, ainsi que leurs sels d'addition avec des acides pharmacologiquement acceptables.

2. 4-(4-chloro-3-diméthylsulfamoylphényl)-3-méthyl-2-phénylimino-4-thiazoline.
3. 4-(4-chloro-3-diéthylsulfamoylphényl)-3-méthyl-2-phénylimino-4-thiazoline.
4. 4-(4-chloro-3-diméthylsulfamoylphényl)-2-(2-chloro-phénylimino)-3-méthyl-4-thiazoline.
5. 4-(4-chloro-3-diméthylsulfamoylphényl)-3-méthyl-2-(2-méthylphénylimino)-4-thiazoline.
6. 4-(4-chloro-3-diméthylsulfamoylphényl)-3-méthyl-2-(2,4-diméthylphényl-imino)-4-thiazoline.
7. 4-(2-chloro-5-diméthylsulfamoylphényl)-3-méthyl-2-2-phénylimino-4-thiazoline.
8. 4-(3-chloro-5-diméthylsulfamoylphényl)-3-méthyl-2-phénylimino-4-thiazoline.
9. 4-(4-chloro-3-diméthylsulfamoylphényl)-2-(4-méthoxyphénylimino)-3-méthyl-4-thiazoline.
10. 4-(4-chloro-3-sulfamoylphényl)-3-méthyl-2-phénylimino-4-thiazoline.
11. Procédé pour la préparation des composés selon la revendication 1, caractérisé en ce qu'il consiste à :

a) faire réagir des composés de formule générale (II) :

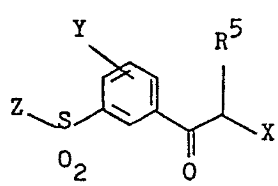

(II)

dans laquelle

$R^5$ et Y ont les significations indiquées,

Z représente un halogène ou $R^6R^7N$, où $R^6$ et $R^7$ ont les significations indiquées, et

X représente un groupe éliminable, dans des conditions de réaction de condensation, avec une thiourée de formule générale (III) :

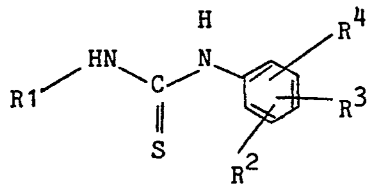

(III)

dans laquelle $R^1$ à $R^4$ ont les significations indiquées, et dans le cas où Z représente un halogène, à faire réagir ensuite un composé de formule (XI), obtenu :

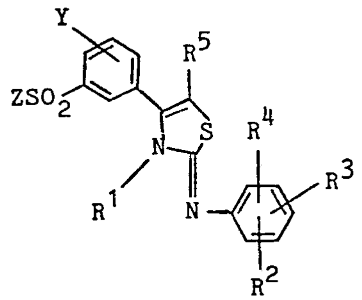

(XI)

dans laquelle $R^1$, $R^2$, $R^3$, $R^4$, $R^5$ et Y ont les significations indiquées pour la formule (I) et Z représente un halogène, avec une amine de formule générale $HNR^6R^7$ où $R^6$ et $R^7$ ont les singifications indiquées, ou

b) séparer l'eau de composés de formule générale (IV) :

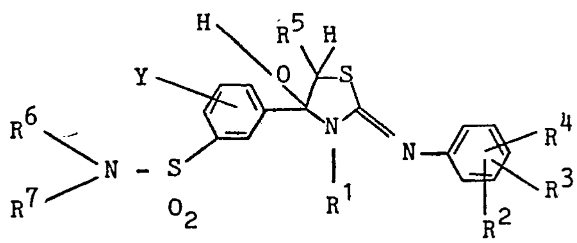

(IV)

dans laquelle $R^1$ à $R^7$ et Y ont les significations indiquées, ou

c) faire réagir des composés de formule générale (V) :

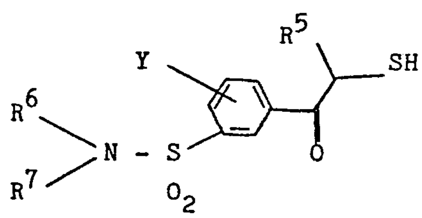

(V)

avec des composés de formule générale (VI) :

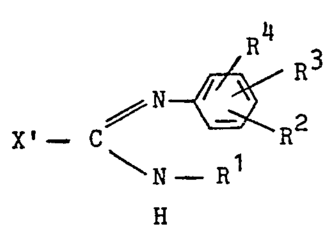

(VI)

où $R^1$ à $R^7$ ont les significations indiquées et X' est un groupe éliminable, ou

d) faire réagir des composés de formule (V) avec des carbodiimides VII :

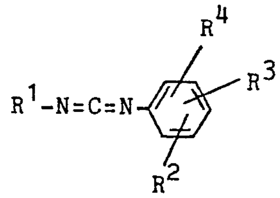

(VII)

où $R^1$ à $R^4$ ont les significations indiquées, ou

e) traiter des composés de formule générale (VIII) :

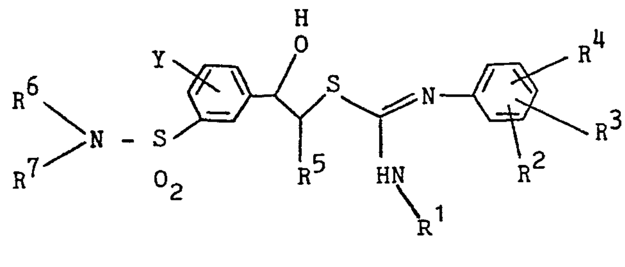

x HHal (VIII)

où $R^1$ à $R^7$ et Y ont les significations indiquées et Hal représente le chlore ou le brome, avec un agent oxydant, ou

f) faire réagir des composés de formule générale (IX) :

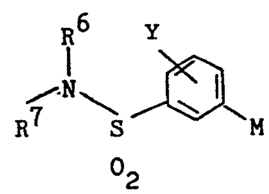

(IX)

où $R^6$ et $R^7$ ne représentent pas l'hydrogène et Y n'est pas le brome ou l'iode, mais ont par ailleurs les significations ci-dessus, et M représente le lithium ou un groupe MgBr, avec des composés de formule générale (X) :

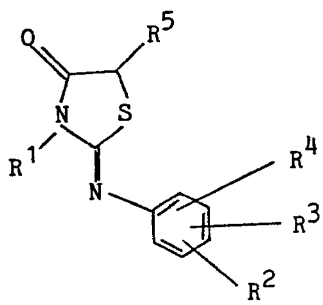

(X)

où $R^1$ à $R^5$ ont les significations indiquées, et soumettre le produit de réaction obtenu à une hydrolyse et une déshydratation, et éventuellement convertir par une alcoylation usuelle les composés de formule générale I, obtenus selon les procédés a) à f), dans lesquels $R^6$ et/ou $R^7$ représentent l'hydrogène, en des composés dans lesquels $R^6$ et/ou $R^7$ ont une des autres significations indiquées ci-dessus, et éventuellement convertir un composé de formule (I), obtenu, avec des acides organiques ou minéraux de formule générale H-A en ses sels d'addition avec des acides, ou convertir les sels des composés de formule générale (I), obtenus, avec des bases en les composés basiques libres de formule (I).

12. Composés de formule (IV) :

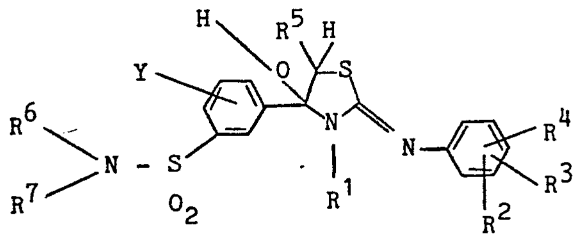

(IV)

où $R^1$ à $R^7$ et Y ont les significations indiquées pour la formule (I), et leurs sels d'addition avec des acides.

13. Composés de formule générale (XI) :

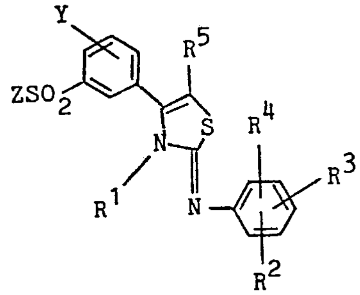

(XI)

où $R^1$ à $R^5$ et Y ont les significations indiquées pour la formule (I) et Z représente un halogène, ainsi que leurs sels d'addition avec des acides.

14. Composés de formule générale (XVII) :

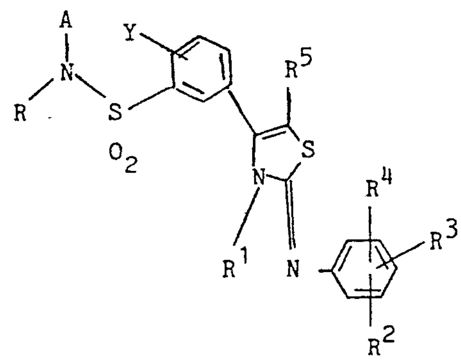

(XVII)

où $R^1$ à $R^5$ et Y ont les significations indiquées pour la formule (I), R a la signification de $R^6$ ou de $R^7$ et A représente le cation d'un métal alcalin ou alcalino-terreux.

15. Compositions pharmaceutiques possédant une activité influençant la gamme des lipoprotéines sériques, constituées de, ou contenant un composé selon la revendication 1.

16. Procédé pour la préparation de compositions pharmaceutiques possédant une activité influençant la gamme des lipoprotéines sériques, caractérisé en ce qu'on met un composé selon la revendication 1, éventuellement avec des véhicules et/ou des stabilisants pharmaceutiques, sous une forme d'administration appropriée pour un but thérapeutique.

**Revendications** (pour l'Etat contractant AT)

1. Procédé pour la préparation de dérivés de thiazoline de formule générale (I) :

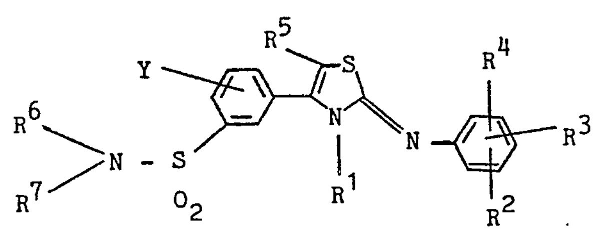

(I)

dans laquelle

$R^1$ représente un groupe alcoyle en $C_1$-$C_8$, cycloalcoyle ayant de 3 à 8 atomes de carbone ou alcényle ayant de 3 à 4 atomes de carbone,

$R^2$, $R^3$ et $R^4$ représentant l'hydrogène, un halogène, un groupe alcoyle ou alcoxy ayant chacun de 1 à 4 atomes de carbone, méthylènedioxy, éthylènedioxy, diméthyl- ou diéthylamino, trifluorométhyle,

$R^5$ représente l'hydrogène ou un groupe alcoyle ayant de 1 à 3 atomes de carbone,

$R^6$ représente l'hydrogène ou un groupe alcoyle ayant de 1 à 6 atomes de carbone,

$R^7$ représente l'hydrogène, un groupe alcoyle ayant de 1 à 12 atomes de carbone, cycloalcoyle ayant de 3 à 12 atomes de carbone, allyle, phényléthyle ou un radical benzyle

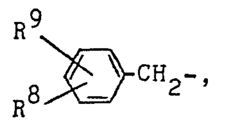

où $R^8$ et $R^9$ sont identiques ou différents et représentent l'hydrogène, un groupe méthyle, chloro ou méthoxy, ou $R^6$ et $R^7$ sont reliés par une chaîne alcoylène éventuellement ramifiée ayant au total 8 atomes de carbone, dans laquelle un groupe méthylène peut être remplacé par un atome d'oxygène ou un groupe $N\text{-}CH_3$, et

Y représente l'hydrogène, un halogène ou un groupe alcoyle ayant de 1 à 3 atomes de carbone, ainsi que leurs sels d'addition avec des acides pharmacologiquement acceptables, caractérisé en ce qu'il consiste à :

a) faire réagir des composés de formule générale (II) :

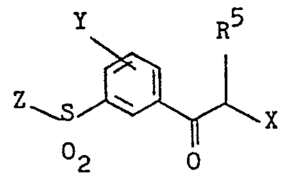

(II)

où

$R^5$ et Y ont les significations indiquées,

Z représente un halogène ou $R^6R^7N$-, $R^6$ et $R^7$ ayant les significations indiquées, et

X représente un groupe éliminable, dans des conditions de condensation, avec une thiourée de formule générale (III) :

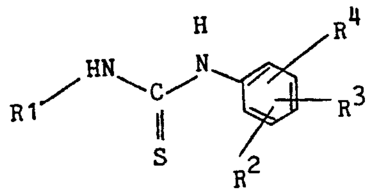

(III)

dans laquelle $R^1$ à $R^4$ ont les significations indiquées, et dans le cas où Z représente un halogène, à faire réagir ensuite un composé obtenu de formule (XI) :

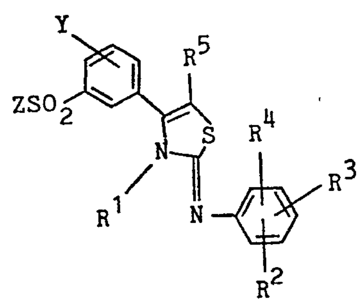

(XI)

dans laquelle $R^1$, $R^2$, $R^3$, $R^4$, $R^5$ et Y ont les significations indiquées pour la formule I et Z représente un halogène, avec une amine de formule générale $HNR^6R^7$ où $R^6$ et $R^7$ ont les significations indiquées, ou

b) séparer l'eau de composés de formule générale (IV) :

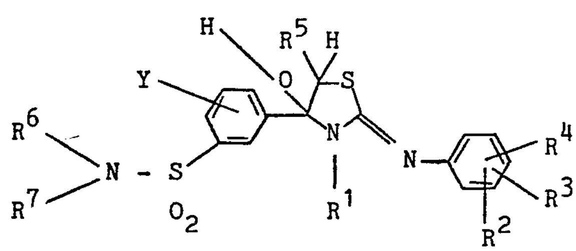

(IV)

dans laquelle $R^1$ à $R^7$ et Y ont les significations indiquées, ou

c) faire réagir des composés de formule générale (V) :

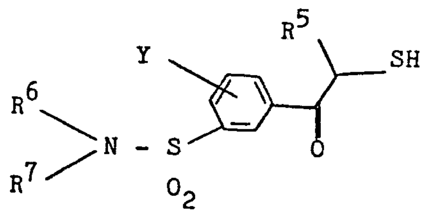

(V)

avec des composés de formule générale (VI) :

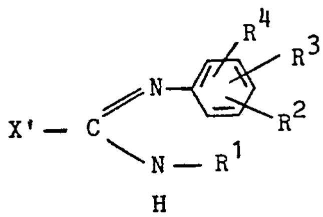

(VI)

où $R^1$ à $R^7$ ont les significations indiquées et X' est un groupe éliminable, ou

d) faire réagir des composés de formule (V) avec des carbodiimides VII :

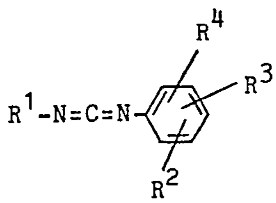

(VII)

où $R^1$ à $R^4$ ont les significations indiquées, ou

e) traiter des composés de formule générale (VIII) :

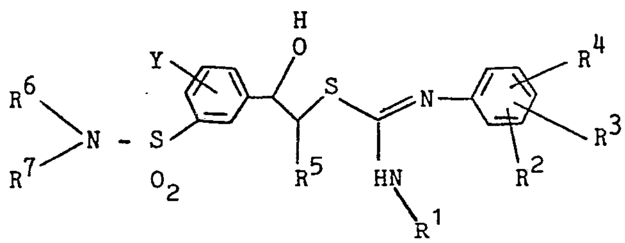

x HHal (VIII)

dans laquelle $R^1$ à $R^7$ et Y ont les significations indiquées et Hal représente le chlore ou le brome, avec un agent oxydant, ou

f) faire réagir des composés de formule générale (IX) :

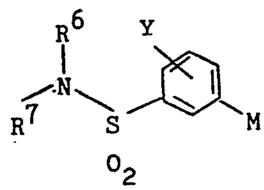

(IX)

dans laquelle $R^6$ et $R^7$ ne représentent pas l'hydrogène et Y ne représente pas le brome ou l'iode, mais ont les significations ci-dessus et M est le lithium ou un groupe MgBr, avec des composés de formule générale (X) :

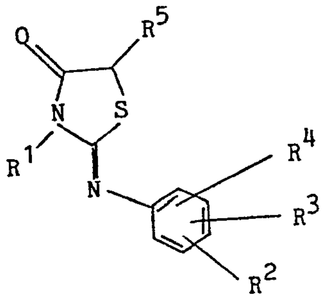

(X)

dans laquelle $R^1$ à $R^5$ ont les significations indiquées, et soumettre le produit de réaction obtenu à une hydrolyse et une déshydratation et éventuellement convertir par une alcoylation usuelle les composés de formule générale (I), dans laquelle $R^6$ et/ou $R^7$ représentent l'hydrogène, obtenus selon les procédés a) à f), en des composés dans lesquels $R^6$ et/ou $R^7$ a une des autres significations indiquées ci-dessus, et éventuellement convertir un composé de formule (I) obtenu, avec des acides organiques ou minéraux de formule générale H-A, en ses sels d'addition avec des acides ou convertir les sels des composés de formule générale (I), obtenus, avec des bases, en les composés basiques libres de formule (I).

2. Procédé selon la revendication 1, caractérisé en ce qu'on prépare la [4-(4-chloro-3-diméthylsulfamoylphényl)-3-méthyl-2-phénylimino-4-thiazoline].

3. Procédé selon la revendication 1, caractérisé en ce qu'on prépare la [4-(4-chloro-3-diéthylsulfamoylphényl)-3-méthyl-2-phénylimino-4-thiazoline].

4. Procédé selon la revendication 1, caractérisé en ce qu'on prépare la [4-(4-chloro-3-diméthylsulfamoylphényl)-2-(2-chloro-phénylimino)-3-méthyl-4-thiazoline].

5. Procédé selon la revendication 1, caractérisé en ce qu'on prépare la [4-(4-chloro-3-diméthylsulfamoylphényl)-3-méthyl-2-(2-méthylphénylimino)-4-thiazoline].

6. Procédé selon la revendication 1, caractérisé en ce qu'on prépare la [4-(4-chloro-3-diméthylsulfamoylphényl)-3-méthyl-2-(2-(2,4-diméthylphénylimino)-4-thiazoline].

7. Procédé selon la revendication 1, caractérisé en ce qu'on prépare la [4-(2-chloro-5-diméthylsulfamoylphényl)-3-méthyl-2-2-phénylimino-4-thiazoline].

8. Procédé selon la revendication 1, caractérisé en ce qu'on prépare la [4-(3-chloro-5-diméthylsulfamoylphényl)-3-méthyl-2-phénylimino-4-thiazoline].

9. Procédé selon la revendication 1, caractérisé en ce qu'on prépare la [4-(4-chloro-3-diméthylsulfamoylphényl)-2-(4-méthoxyphénylimino)-3-méthyl-4-thiazoline].

10. Procédé selon la revendication 1, caractérisé en ce qu'on prépare la [4-(4-chloro-3-sulfamoylphényl)-3-méthyl-2-phénylimino-4-thiazoline].

11. Composés de formule (IV) :

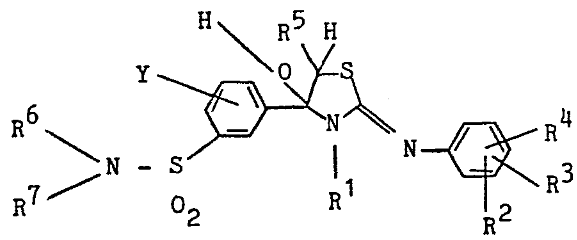

(IV)

où $R^1$ à $R^7$ et Y ont les significations indiquées pour la formule (I), et leurs sels d'addition avec des acides.

12. Composés de formule générale (XI) :

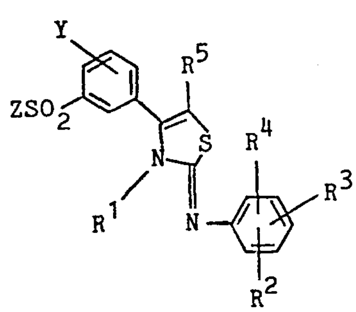

(XI)

où $R^1$ à $R^5$ et Y ont les significations indiquées pour la formule (I), et Z représente un halogène, ainsi que leurs sels d'addition avec des acides.

13. Composés de formule générale (XVII) :

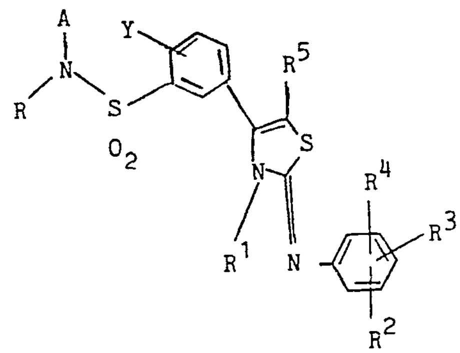

(XVII)

où $R^1$ à $R^5$ et Y ont les significations indiquées pour la formule (I), R a la signification de $R^6$ ou de $R^7$ et A est le cation d'un métal alcalin ou alcalino-terreux.